# EUROPEAN PATENT APPLICATION

(11) **EP 3 910 331 A1**
(43) Date of publication of application: **17.11.2021**
(21) Application number: 20175030.4
(22) Date of filing: 15.05.2020
(51) Int. Cl.: G01N 33/50, G01N 33/574

(54) **INTRACELLULAR KINASE ASSOCIATED WITH RESISTANCE AGAINST T-CELL MEDIATED CYTOTOXICITY, AND USES THEREOF**

(71) Applicant: iOmx Therapeutics AG, 82152 Martinsried (DE)
(72) Inventor: VOLPIN, Valentina, 93053 Regensburg (DE); BECKHOVE, Philipp, 93053 Regensburg (DE); SORRENTINO, Antonio, 93053 Regensburg (DE); BOUTROS, Michael, 69120 Heidelberg (DE); KHANDELWAL, Nisit, 82152 Martinsried 13 (DE); SENNHENN, Peter, 82152 Martinsried 13 (DE)
(74) Representative: Kuttenkeuler, David

(57) **Abstract**

The invention is based on the identification of the intracellular kinase calcium/calmodulin-dependent protein kinase 1D (CAMK1D) as a key checkpoint inhibitor in tumour cells mediating resistance against cytotoxic T lymphocytes (CTL). CAMKiD was identified in PD-L1 refractory tumours to impair CTL-induced death receptor signalling and apoptosis via caspase inhibition. The invention offers therapeutic approaches involving impairing CAMKiD immune checkpoint function by various CAMKiD inhibitors, especially nucleic acid or small molecule inhibitors of CAMKiD and/or treatments involving CAMKiD inhibitors with death receptor agonists. The medical approaches of the invention are useful for treating subjects suffering from various proliferative disorders; preferably such proliferative disorders that are characterized by a resistance to CTL mediated immune responses, or which are refractory or resistant to treatments with other immune checkpoint therapies, such as PD1-PDL1 antagonistic treatments. Provided are the medical applications and corresponding diagnostic approaches, kits, CAMKiD inhibitors and screening methods for the identification of new therapeutic agents for the treatment of proliferative disorders.

SEQUENCE LISTING

<110>  iOmx Therapeutics AG

<120>  INTRACELLULAR KINASE ASSOCIATED WITH RESISTANCE AGAINST T-CELL
       MEDIATED CYTOTOXICITY, AND USES THEREOF

<130>  I33723EP

<160>  13

<170>  PatentIn version 3.5

<210>  1
<211>  385
<212>  PRT
<213>  Homo sapiens

<400>  1

Met Ala Arg Glu Asn Gly Glu Ser Ser Ser Ser Trp Lys Lys Gln Ala
1               5                   10                  15

Glu Asp Ile Lys Lys Ile Phe Glu Phe Lys Glu Thr Leu Gly Thr Gly
            20                  25                  30

Ala Phe Ser Glu Val Val Leu Ala Glu Glu Lys Ala Thr Gly Lys Leu
        35                  40                  45

Phe Ala Val Lys Cys Ile Pro Lys Lys Ala Leu Lys Gly Lys Glu Ser
    50                  55                  60

Ser Ile Glu Asn Glu Ile Ala Val Leu Arg Lys Ile Lys His Glu Asn
65                  70                  75                  80

Ile Val Ala Leu Glu Asp Ile Tyr Glu Ser Pro Asn His Leu Tyr Leu
                85                  90                  95

Val Met Gln Leu Val Ser Gly Gly Glu Leu Phe Asp Arg Ile Val Glu
            100                 105                 110

Lys Gly Phe Tyr Thr Glu Lys Asp Ala Ser Thr Leu Ile Arg Gln Val
        115                 120                 125

Leu Asp Ala Val Tyr Tyr Leu His Arg Met Gly Ile Val His Arg Asp
    130                 135                 140

Leu Lys Pro Glu Asn Leu Leu Tyr Tyr Ser Gln Asp Glu Glu Ser Lys
145                 150                 155                 160

Ile Met Ile Ser Asp Phe Gly Leu Ser Lys Met Glu Gly Lys Gly Asp
                165                 170                 175

Val Met Ser Thr Ala Cys Gly Thr Pro Gly Tyr Val Ala Pro Glu Val
            180                 185                 190

Leu Ala Gln Lys Pro Tyr Ser Lys Ala Val Asp Cys Trp Ser Ile Gly
        195                 200                 205

Val Ile Ala Tyr Ile Leu Leu Cys Gly Tyr Pro Pro Phe Tyr Asp Glu
    210                 215                 220

Asn Asp Ser Lys Leu Phe Glu Gln Ile Leu Lys Ala Glu Tyr Glu Phe
225                 230                 235                 240

Asp Ser Pro Tyr Trp Asp Asp Ile Ser Asp Ser Ala Lys Asp Phe Ile
                245                 250                 255

Arg Asn Leu Met Glu Lys Asp Pro Asn Lys Arg Tyr Thr Cys Glu Gln
            260                 265                 270

Ala Ala Arg His Pro Trp Ile Ala Gly Asp Thr Ala Leu Asn Lys Asn
        275                 280                 285

Ile His Glu Ser Val Ser Ala Gln Ile Arg Lys Asn Phe Ala Lys Ser
    290                 295                 300

Lys Trp Arg Gln Ala Phe Asn Ala Thr Ala Val Val Arg His Met Arg
305                 310                 315                 320

Lys Leu His Leu Gly Ser Ser Leu Asp Ser Ser Asn Ala Ser Val Ser
                325                 330                 335

Ser Ser Leu Ser Leu Ala Ser Gln Lys Asp Cys Leu Ala Pro Ser Thr
            340                 345                 350

Leu Cys Ser Phe Ile Ser Ser Ser Ser Gly Val Ser Gly Val Gly Ala
        355                 360                 365

Glu Arg Arg Pro Arg Pro Thr Thr Val Thr Ala Val His Ser Gly Ser
    370                 375                 380

Lys
385

<210> 2
<211> 357
<212> PRT
<213> Homo sapiens

<400> 2
   Met Ala Arg Glu Asn Gly Glu Ser Ser Ser Ser Trp Lys Lys Gln Ala
   1               5                   10                  15
   Glu Asp Ile Lys Lys Ile Phe Glu Phe Lys Glu Thr Leu Gly Thr Gly
               20                  25                  30
   Ala Phe Ser Glu Val Val Leu Ala Glu Glu Lys Ala Thr Gly Lys Leu
           35                  40                  45
   Phe Ala Val Lys Cys Ile Pro Lys Lys Ala Leu Lys Gly Lys Glu Ser
       50                  55                  60
   Ser Ile Glu Asn Glu Ile Ala Val Leu Arg Lys Ile Lys His Glu Asn
   65                  70                  75                  80
   Ile Val Ala Leu Glu Asp Ile Tyr Glu Ser Pro Asn His Leu Tyr Leu
                   85                  90                  95
   Val Met Gln Leu Val Ser Gly Gly Glu Leu Phe Asp Arg Ile Val Glu
               100                 105                 110
   Lys Gly Phe Tyr Thr Glu Lys Asp Ala Ser Thr Leu Ile Arg Gln Val
           115                 120                 125
   Leu Asp Ala Val Tyr Tyr Leu His Arg Met Gly Ile Val His Arg Asp
       130                 135                 140
   Leu Lys Pro Glu Asn Leu Leu Tyr Tyr Ser Gln Asp Glu Glu Ser Lys
   145                 150                 155                 160
   Ile Met Ile Ser Asp Phe Gly Leu Ser Lys Met Glu Gly Lys Gly Asp
                   165                 170                 175
   Val Met Ser Thr Ala Cys Gly Thr Pro Gly Tyr Val Ala Pro Glu Val
               180                 185                 190
   Leu Ala Gln Lys Pro Tyr Ser Lys Ala Val Asp Cys Trp Ser Ile Gly
           195                 200                 205
   Val Ile Ala Tyr Ile Leu Leu Cys Gly Tyr Pro Pro Phe Tyr Asp Glu
       210                 215                 220
   Asn Asp Ser Lys Leu Phe Glu Gln Ile Leu Lys Ala Glu Tyr Glu Phe
   225                 230                 235                 240
   Asp Ser Pro Tyr Trp Asp Asp Ile Ser Asp Ser Ala Lys Asp Phe Ile
                   245                 250                 255
   Arg Asn Leu Met Glu Lys Asp Pro Asn Lys Arg Tyr Thr Cys Glu Gln
               260                 265                 270
   Ala Ala Arg His Pro Trp Ile Ala Gly Asp Thr Ala Leu Asn Lys Asn
           275                 280                 285
   Ile His Glu Ser Val Ser Ala Gln Ile Arg Lys Asn Phe Ala Lys Ser
       290                 295                 300
   Lys Trp Arg Gln Ala Phe Asn Ala Thr Ala Val Val Arg His Met Arg
   305                 310                 315                 320
   Lys Leu His Leu Gly Ser Ser Leu Asp Ser Ser Asn Ala Ser Val Ser
                   325                 330                 335
   Ser Ser Leu Ser Leu Ala Ser Gln Lys Asp Cys Ala Tyr Val Ala Lys
               340                 345                 350
   Pro Glu Ser Leu Ser
           355

<210>  3
<211>  19
<212>  DNA
<213>  artificial

<220>
<223>  siRNA sequence targeting CAMK1D

<400>  3
ugaaguguau cccuaagaa                                                    19

<210>  4
<211>  19
<212>  RNA
<213>  artificial

<220>
<223>  siRNA sequence targeting CAMK1D

<400>  4
caaaucaccu guacuuggu                                                    19

<210>  5
<211>  19
<212>  RNA
<213>  artificial

<220>
<223>  siRNA sequence targeting CAMK1D

<400>  5

ccgaaaaucu cuuguacua                                                    19


<210>  6
<211>  19
<212>  RNA
<213>  artificial

<220>
<223>  siRNA sequence targeting CAMK1D

<400>  6
gagaaggacc cgaauaaaa                                                    19


<210>  7
<211>  20
<212>  DNA
<213>  artificial

<220>
<223>  gRNA sequence for targeting mouse CAMK1D by gene editing

<400>  7
tcgatcggat agtggagaag                                                   20


<210>  8
<211>  20
<212>  DNA
<213>  artificial

<220>
<223>  gRNA sequence for targeting mouse CAMK1D by gene editing

<400>  8
ggagatagta tacggcatcc                                                   20


<210>  9
<211>  20
<212>  DNA
<213>  artificial

<220>
<223>  gRNA sequence for targeting CAMK1D by gene editing

<400>  9
tagccgagga gaaagctact                                                   20


<210>  10
<211>  20
<212>  DNA
<213>  Mus musculus

<400>  10
ggagatagta tacggcatcc                                                   20


<210>  11
<211>  149
<212>  PRT

<213>  Homo sapiens

<400>  11


<210>  12
<211>  505
<212>  PRT
<213>  Homo sapiens

<400>  12

Met Glu Gly Gly Pro Ala Val Cys Cys Gln Asp Pro Arg Ala Glu Leu
1               5                   10                  15

Val Glu Arg Val Ala Ala Ile Asp Val Thr His Leu Glu Glu Ala Asp
            20                  25                  30

Gly Gly Pro Glu Pro Thr Arg Asn Gly Val Asp Pro Pro Pro Arg Ala
        35                  40                  45

Arg Ala Ala Ser Val Ile Pro Gly Ser Thr Ser Arg Leu Leu Pro Ala
    50                  55                  60

Arg Pro Ser Leu Ser Ala Arg Lys Leu Ser Leu Gln Glu Arg Pro Ala
65                  70                  75                  80

Gly Ser Tyr Leu Glu Ala Gln Ala Gly Pro Tyr Ala Thr Gly Pro Ala
                85                  90                  95

Ser His Ile Ser Pro Arg Ala Trp Arg Arg Pro Thr Ile Glu Ser His
            100                 105                 110

His Val Ala Ile Ser Asp Ala Glu Asp Cys Val Gln Leu Asn Gln Tyr
        115                 120                 125

Lys Leu Gln Ser Glu Ile Gly Lys Gly Ala Tyr Gly Val Val Arg Leu
    130                 135                 140

Ala Tyr Asn Glu Ser Glu Asp Arg His Tyr Ala Met Lys Val Leu Ser
145                 150                 155                 160

Lys Lys Lys Leu Leu Lys Gln Tyr Gly Phe Pro Arg Arg Pro Pro Pro
                165                 170                 175

Arg Gly Ser Gln Ala Ala Gln Gly Gly Pro Ala Lys Gln Leu Leu Pro
            180                 185                 190

Leu Glu Arg Val Tyr Gln Glu Ile Ala Ile Leu Lys Lys Leu Asp His
        195                 200                 205

Val Asn Val Val Lys Leu Ile Glu Val Leu Asp Asp Pro Ala Glu Asp
    210                 215                 220

Asn Leu Tyr Leu Val Phe Asp Leu Leu Arg Lys Gly Pro Val Met Glu
225                 230                 235                 240

Val Pro Cys Asp Lys Pro Phe Ser Glu Glu Gln Ala Arg Leu Tyr Leu
                245                 250                 255

Arg Asp Val Ile Leu Gly Leu Glu Tyr Leu His Cys Gln Lys Ile Val
            260                 265                 270

His Arg Asp Ile Lys Pro Ser Asn Leu Leu Leu Gly Asp Asp Gly His
        275                 280                 285

Val Lys Ile Ala Asp Phe Gly Val Ser Asn Gln Phe Glu Gly Asn Asp
    290                 295                 300

Ala Gln Leu Ser Ser Thr Ala Gly Thr Pro Ala Phe Met Ala Pro Glu
305                 310                 315                 320

Ala Ile Ser Asp Ser Gly Gln Ser Phe Ser Gly Lys Ala Leu Asp Val
                325                 330                 335

Trp Ala Thr Gly Val Thr Leu Tyr Cys Phe Val Tyr Gly Lys Cys Pro
            340                 345                 350

Phe Ile Asp Asp Phe Ile Leu Ala Leu His Arg Lys Ile Lys Asn Glu
        355                 360                 365

Pro Val Val Phe Pro Glu Glu Pro Glu Ile Ser Glu Glu Leu Lys Asp
    370                 375                 380

Leu Ile Leu Lys Met Leu Asp Lys Asn Pro Glu Thr Arg Ile Gly Val
385                 390                 395                 400

Pro Asp Ile Lys Leu His Pro Trp Val Thr Lys Asn Gly Glu Glu Pro
                405                 410                 415

Leu Pro Ser Glu Glu Glu His Cys Ser Val Val Glu Val Thr Glu Glu
            420                 425                 430

Glu Val Lys Asn Ser Val Arg Leu Ile Pro Ser Trp Thr Thr Val Ile
        435                 440                 445

Leu Val Lys Ser Met Leu Arg Lys Arg Ser Phe Gly Asn Pro Phe Glu
    450                 455                 460

Pro Gln Ala Arg Arg Glu Glu Arg Ser Met Ser Ala Pro Gly Asn Leu
465                 470                 475                 480

Leu Val Lys Glu Gly Phe Gly Glu Gly Gly Lys Ser Pro Glu Leu Pro
                485                 490                 495

Gly Val Gln Glu Asp Glu Ala Ala Ser
            500                 505

<210>  13
<211>  520
<212>  PRT
<213>  Homo sapiens

<400>  13

Met Glu Gly Gly Pro Ala Val Cys Cys Gln Asp Pro Arg Ala Glu Leu
1               5                   10                  15

Val Glu Arg Val Ala Ala Ile Asp Val Thr His Leu Glu Glu Ala Asp
            20                  25                  30

Gly Gly Pro Glu Pro Thr Arg Asn Gly Val Asp Pro Pro Pro Arg Ala
        35                  40                  45

Arg Ala Ala Ser Val Ile Pro Gly Ser Thr Ser Arg Leu Leu Pro Ala
    50                  55                  60

Arg Pro Ser Leu Ser Ala Arg Lys Leu Ser Leu Gln Glu Arg Pro Ala
65                  70                  75                  80

Gly Ser Tyr Leu Glu Ala Gln Ala Gly Pro Tyr Ala Thr Gly Pro Ala
                85                  90                  95

Ser His Ile Ser Pro Arg Ala Trp Arg Arg Pro Thr Ile Glu Ser His
            100                 105                 110

His Val Ala Ile Ser Asp Ala Glu Asp Cys Val Gln Leu Asn Gln Tyr
        115                 120                 125

Lys Leu Gln Ser Glu Ile Gly Lys Gly Ala Tyr Gly Val Val Arg Leu
    130                 135                 140

Ala Tyr Asn Glu Ser Glu Asp Arg His Tyr Ala Met Lys Val Leu Ser
145                 150                 155                 160

Lys Lys Lys Leu Leu Lys Gln Tyr Gly Phe Pro Arg Arg Pro Pro Pro
                165                 170                 175

Arg Gly Ser Gln Ala Ala Gln Gly Gly Pro Ala Lys Gln Leu Leu Pro
            180                 185                 190

Leu Glu Arg Val Tyr Gln Glu Ile Ala Ile Leu Lys Lys Leu Asp His
        195                 200                 205

Val Asn Val Val Lys Leu Ile Glu Val Leu Asp Asp Pro Ala Glu Asp
    210                 215                 220

Asn Leu Tyr Leu Ala Leu Gln Asn Gln Ala Gln Asn Ile Gln Leu Asp
225                 230                 235                 240

Ser Thr Asn Ile Ala Lys Pro His Ser Leu Leu Pro Ser Glu Gln Gln
                245                 250                 255

Asp Ser Gly Ser Thr Trp Ala Ala Arg Ser Val Phe Asp Leu Leu Arg
            260                 265                 270

Lys Gly Pro Val Met Glu Val Pro Cys Asp Lys Pro Phe Ser Glu Glu
        275                 280                 285

Gln Ala Arg Leu Tyr Leu Arg Asp Val Ile Leu Gly Leu Glu Tyr Leu
    290                 295                 300

His Cys Gln Lys Ile Val His Arg Asp Ile Lys Pro Ser Asn Leu Leu
305                 310                 315                 320

Leu Gly Asp Asp Gly His Val Lys Ile Ala Asp Phe Gly Val Ser Asn
                325                 330                 335

Gln Phe Glu Gly Asn Asp Ala Gln Leu Ser Ser Thr Ala Gly Thr Pro
            340                 345                 350

Ala Phe Met Ala Pro Glu Ala Ile Ser Asp Ser Gly Gln Ser Phe Ser
        355                 360                 365

Gly Lys Ala Leu Asp Val Trp Ala Thr Gly Val Thr Leu Tyr Cys Phe
    370                 375                 380

Val Tyr Gly Lys Cys Pro Phe Ile Asp Asp Phe Ile Leu Ala Leu His
385                 390                 395                 400

Arg Lys Ile Lys Asn Glu Pro Val Val Phe Pro Glu Glu Pro Glu Ile
                405                 410                 415

Ser Glu Glu Leu Lys Asp Leu Ile Leu Lys Met Leu Asp Lys Asn Pro
            420                 425                 430

Glu Thr Arg Ile Gly Val Pro Asp Ile Lys Leu His Pro Trp Val Thr
        435                 440                 445

Lys Asn Gly Glu Glu Pro Leu Pro Ser Glu Glu Glu His Cys Ser Val
    450                 455                 460

Val Glu Val Thr Glu Glu Glu Val Lys Asn Ser Val Arg Leu Ile Pro
465                 470                 475                 480

Ser Trp Thr Thr Val Ile Leu Val Lys Ser Met Leu Arg Lys Arg Ser
                485                 490                 495

Phe Gly Asn Pro Phe Glu Pro Gln Ala Arg Arg Glu Glu Arg Ser Met
            500                 505                 510

Ser Ala Pro Gly Asn Leu Leu Val

515                                                    520

## Description

### FIELD OF THE INVENTION

The invention is based on the identification of the intracellular kinase calcium/calmodulin-dependent protein kinase 1D (CAMK1D) as a key checkpoint inhibitor in tumour cells mediating resistance against cytotoxic T lymphocytes (CTL). CAMK1D was identified in PD-L1 refractory tumours to impair CTL-induced death receptor signalling and apoptosis via caspase inhibition. The invention offers therapeutic approaches involving impairing CAMK1D immune checkpoint function by various CAMK1D inhibitors, especially nucleic acid or small molecule inhibitors of CAMK1D and/or treatments involving CAMK1D inhibitors with death receptor agonists. The medical approaches of the invention are useful for treating subjects suffering from various proliferative disorders; preferably such proliferative disorders that are characterized by a resistance to CTL mediated immune responses, or which are refractory or resistant to treatments with other immune checkpoint therapies, such as PD1-PDL1 antagonistic treatments. Provided are the medical applications and corresponding diagnostic approaches, kits, CAMK1D inhibitors and screening methods for the identification of new therapeutic agents for the treatment of proliferative disorders.

### DESCRIPTION

Endogenous T cell responses against tumour antigens occur frequently in a broad variety of cancer types (1-4). Although these can correlate with an overall improvement of patient prognosis (2, 5, 6), they often do not rescue patients from tumour progression. A major reason for the inability of tumour-reactive cytotoxic T cells to eradicate established tumours lies in the capacity of tumour cells to regulate T cell activity through expression of the immune-inhibitory ligand PD-L1, which stimulates the inhibitory receptor PD-1 expressed on effector T cells and reduces T cell receptor signalling. PD-L1 expression in healthy and tumour tissues is itself induced by effector T cell activity through the secretion of inflammatory cytokines such as IFN-gamma (7-9) and serves as an important mechanism to prevent autoimmune diseases. Consequently, blockade of PD-L1/PD-1 interactions by therapeutic antibodies has resulted in stunning immune rejection of established, even metastasized tumours in many patients with PD-L1 expressing tumours (10-15).

However, despite noteworthy improvements, a significant proportion of cancer patients lack response to anti-PD-Li/PD-i antibody therapies (16-19). Several mechanisms of PD-L1/PD-1 treatment resistance have been recently introduced including impaired tumour intrinsic IFN-gamma responsiveness resulting in reduced PD-L1 expression, severe and irreversible T cell exhaustion or block of T cell differentiation induced by chronic PD-1 over-stimulation (20). However, since functional capacity of tumour reactive T cells is found in many patients with PD-L1/PD-1 refractory tumours (3, 6), these mechanisms may only explain immune response resistance in a minor fraction of cases. Therefore, additional immune regulatory interactions may impose dominant levels of protection against immune destruction. Indeed, several immune inhibitory receptors such as TIM3 or VISTA, which can be triggered by ligands expressed in tumours, have been characterized in the past (21, 22). Nevertheless, it is well conceivable that immune resistance is not only conveyed by immune regulatory ligands controlling T cell activity, but may also be achieved by tumour intrinsic mechanisms of resistance against the cytotoxic T cell attack.

Multiple myeloma (MM) is still a rarely curable B-cell malignancy characterized by the accumulation of malignant plasma cell clones in the bone marrow. In MM spontaneous cytotoxic T cell responses against myeloma-associated antigens occur (1). Several studies showed that immune-checkpoint molecules are expressed by myeloma cells and induce tumour-related immune suppression (23-25). In line, PD-L1 is commonly expressed on malignant plasma cells (9) and high expression of PD-L1 is associated with disease progression and has been found further upregulated at relapse or in the refractory stage (26). Still, preliminary results of a phase I trial with PD-1 blocking antibodies were disappointing, reporting no objective responses amongst the 27 treated multiple myeloma patients (27). Therefore, there is a rationale to assume that other immune-checkpoint molecules may play a crucial role in tumour escape mechanisms in this tumour entity. This is of special relevance as immunotherapeutic treatment options emerge in multiple myeloma, including monoclonal antibodies against CD38 (e.g. daratumumab, isatuximab), SLAMF7 (elotuzumab), BCMA-CAR-T-based treatments or BCMA-T-cell bispecific antibodies (28-32).

The immune elimination of tumour cells is mediated by apoptosis that can be induced either by death receptor signalling (extrinsic pathway) or by the release of cytotoxic granules (intrinsic or mitochondrial pathway) [74]. Ligand-induced apoptosis (extrinsic apoptotic pathway), is mediated by death receptors, expressed on tumour cells such as Fas, DR3/DR4 and TNFR1, which belong to the tumour necrosis factor superfamily. The interaction with their respective ligands (FasL, TRAIL and TNF) induces apoptosis in the tumour cells via the death inducing signalling complex (DISC) [71, 73]. More specifically, the interaction between the receptors and the ligands induces oligomerization of the receptor, which recruits and binds through its cytoplasmatic death domain (DD) the adaptor protein FADD (Fas associated death domain) in case of Fas and DR3/4 or TRADD (Tumour necrosis factor receptor type 1-associated death domain) in case of TNFR1. FADD carries a death effector domain (DED) and by homologous interaction it recruits the DED containing initiator pro-caspase-8 or pro-caspase-10 protein. This complex is referred to as DISC. After binding to DISC, pro-caspase-8 homodimers undergo a conformational change and autocatalytic processing, resulting in the generation of active caspase-8 and caspase-10. Subsequently, these initiator caspases can directly cleave and activate executioner caspases such as caspase-3, caspase-6 and caspase-7, which in turn cleave substrates within the cells, thus triggering the apoptotic signal [76].

Recombinant human tumour necrosis factor (TNF)-related apoptosis inducing ligand (rhTRAIL) and its agonistic antibodies have been under intense focus as crucial, molecularly targeted, antitumour biologies. Unlike conventional anticancer agents and even other TNF family members, rhTRAIL selectively transduces apoptotic signals by binding to death receptors (DRs) that are widely expressed in most cancers, TRAIL-R1/DR4 and TRAIL-R2/DR5, while sparing normal cells. This high tumour specificity along with broad applicability across multiple cancer types and proven safety in humans make TRAIL an ideal candidate for cancer therapy. However, recent clinical trials of rhTRAIL, e.g. dulanermin, or humanized DR agonistic monoclonal antibodies, tested as either a monotherapy or combined with anticancer agents have failed to demonstrate benefits in cancer patients compared with historical controls. The disappointing results raise concerns for the therapeutic implications of rhTRAIL. The primary challenge in TRAIL-based therapy is natural resistance. The majority of primary cancer cells are TRAIL-resistant. Mechanisms of TRAIL resistance are distinct among cancer cell types; however, they commonly comprise of: reduced cell surface DR expression, inhibited caspase-8 activation - the initiator caspase, up-regulated anti-apoptotic molecules such as Bcl-2 and the inhibitors of apoptosis (IAP) family proteins, and reduced expression of pro-apoptotic markers like Bax/Bak. The role of diverse molecules like anticancer agents and natural compounds in sensitizing TRAIL-resistant cancer cells has been investigated and introduced as an addition to TRAIL monotherapy. TRAIL-based combinations were well validated in vitro and in a few in vivo cancer models; however, they fail to demonstrate a similar synergy in cancer patients. The critical reasons for ineffectiveness of rhTRAIL combination in humans are not clearly explained in the literature. This implies a need for alternative approaches to realize rhTRAIL combination therapy in the clinic.

CD95L (also known as FasL, Apo-1) belongs to the TNF (Tumour Necrosis Factor) family and is the ligand of the "death receptor" CD95 (also known as Fas or TNR6). In contrast to its ubiquitously expressed receptor, CD95L has been reported to exhibit a restricted expression pattern and is observed primarily at the surface of activated T lymphocytes and natural killer (NK) cells, where it plays a pivotal role in the elimination of infected and transformed cells. CD95L is also expressed on the surface of epithelial cells, macrophages and dendritic cells under inflammatory conditions (Tauzin et al, 2012). CD95L is a transmembrane "cytokine" whose extracellular domain can be cleaved by metalloproteases such as MMP3 (Matsuno et al, 2001), MMP7 (Vargo-Gogola et al, 2002), MMP9 (Kiaei et al, 2007) or ADAM- 10 (A Disintegrin And Metalloproteinase 10) (Kirkin et al, 2007; Schulte et al, 2007) to produce a soluble ligand. The CD95/CD95L system has been also endowed with pro-oncogenic functions by promoting proliferation of ovarian and liver cancers (Chen et al, 2010), invasion of glioblastomas (Kleber et al, 2008) and chemotherapy resistance of lung cancers (Bivona et al, 2011) through molecular mechanisms that remain to be elucidated. From a molecular standpoint, the binding of membrane-bound CD95L (m-CD95L) to CD95 leads to recruitment of the adaptor protein Fas-associated death domain protein (FADD) at the CD95 intracellular region called the death domain (DD). In turn, FADD binds and aggregates caspases 8 and 10. This CD95/FADD/caspase complex, called the Death- Inducing Signalling Complex (DISC) (76), plays a pivotal role in the initiation of the apoptotic signal. CD95-CD95L based therapies had only limited success to date because systemic activation of the pathway is highly toxic and in clinical trials led to severe liver injury and hepatitis. However, alternatively restricted application by targeted viral delivery offered are more constraint and tissue specific option for treatment and proofed successful in models for adenovirus based gene therapy, although none of the approaches was tested in clinical studies. Another option for targeting the CD95 axis in therapy offered fusion proteins of CD95L and anti-CD20/CD7 antibodies to limit CD95L to the tumour target. Finally, much effort was made to develop antibodies targeting CD95/CD95L. For example, the hamster antimouse CD95 antibody RK8 efficiently kills thymocytes, but does not affect hepatocytes. In contrast to this, Jo2, which is also a hamster antimouse CD95antibody, kills thymocytes with about the same efficiency as reported for RK8, but is additionally toxic for hepatotcytes and confers systemic toxicity [Nishimura Y, et al. In vivo analysis of Fas antigen-mediated apoptosis: effects of agonistic anti-mouse Fas mAb on thymus, spleen and liver. Int Immunol. 1997;9:307-16.]. Another antibody showing tissue-specific activity is HFE7A which has been derived by the immunization of CD95 deficient mice with human CD95 and has already been tested in many species, among them Macaca fascicularis and marmosets, without showing hepatotoxicity [Nishimura Y, et al. In vivo analysis of Fas antigen-mediated apoptosis: effects of agonistic anti-mouse Fas mAb on thymus, spleen and liver. Int Immunol. 1997;9:307-16.]. The most recent anti-CD95 antibody is R-125224, a humanized anti-human CD95 monoclonal antibody that was shown to selectively induce apoptosis in type I activated lymphocytes but not in type II cells, e.g. Jurkat cells or hepatocytes. In a SCID mouse model, R-125224 reduced the number of activated CD3+ cells in vivo. Taking into consideration that hepatotoxicity is one of the biggest problems of CD95 agonist treatment and that hepatocytes are classified as type II cells, which are not affected by R-125224 [Nakayama J, et al. A humanized anti-human Fas antibody, R-125224, induces apoptosis in type I activated lymphocytes but not in type II cells. Int Immunol. 2006;18:113-24].

Calmodulin (CaM) is a calcium-binding messenger protein produced in all eukaryotic cells. CaM is a multifunctional intermediate messenger protein that transduces calcium signals by binding calcium ions and then modifying its interactions with various target proteins. Calmodulin is a small, highly conserved protein approximately 148 amino acids long (16706 Daltons). It contains four EF-hand motifs, each of which binds a Ca2+ ion. The protein has two approximately symmetrical globular domains (the N- and C-domain), separated by a flexible linker region. Calcium participates in an intracellular signalling system by acting as a diffusible second messenger to the initial stimuli.

Therefore, there is a need, from one or more of the above perspectives, for novel approaches to render cells involved with a proliferative disorder (such as a tumour) more susceptible to the immune system, and in particular to circumvent tumour immune escape mechanisms. The present invention seeks to provide, in particular, novel therapeutic approaches and methods involving existing or novel compounds; for example compounds that sensitise such cells towards a cytotoxic response of the immune system or components thereof. Furthermore, the invention seeks to provide novel strategies to diagnose, prognose and/or monitor cell resistance to such an immune response or components, as wells as screening approaches for the identification of compounds that are useful in the treatment of proliferative disorders. Accordingly, it is an object of the present invention to provide alternative, improved, simpler, cheaper and/or integrated means or methods that address one or more of these or other problems. Such an object underlying the present invention is solved by the subject matter as disclosed or defined anywhere herein, for example by the subject matter of the attached claims.

### BRIEF DESCRIPTION OF THE INVENTION

Generally, and by way of brief description, the main aspects of the present invention can be described as follows:
In a **first aspect,** the invention pertains to a Calcium/calmodulin-dependent protein kinase type 1D (CAMK1D) inhibitor for use in a treatment of a proliferative disorder in a subject, wherein the treatment involves inhibiting an activity, function, expression and/or stability of CAMK1D, preferably thereby sensitising cells involved with the proliferative disorder to a cell-dependent or cell-independent cytotoxic stimulus; wherein the treatment comprises administering the CAMK1D inhibitor to the subject.

In a **second aspect,** the invention pertains a CAMK1D inhibitor for use in a treatment of a proliferative disorder in a subject, the treatment comprising exposing cells involved with the proliferative disorder in the subject to: (i) cell-dependent or cell-independent cytotoxic stimulus; and (ii) the CAMK1D inhibitor.

In a **third aspect,** the invention pertains to a CAMK1D inhibitor for use in a treatment of a proliferative disorder in a subject, wherein the treatment is for sensitizing cells involved with the proliferative disorder to a cell-dependent or cell-independent cytotoxic stimulus, the treatment comprising administering the CAMK1D inhibitor to the subject.

In a **fourth aspect,** the invention pertains to a CAMK1D inhibitor for use in a treatment for increasing the therapeutic index of treatment with a cell-dependent or cell-independent cytotoxic stimulus in a subject being treated therewith for a proliferative disorder, the method comprising administering the inhibitor of CAMK1D to the subject.

In a **fifth aspect,** the invention pertains to a CAMK1D inhibitor for use in a treatment for the sensitisation of a subject suffering from a proliferative disorder to a therapy involving the administration of a cell-dependent or cell-independent cytotoxic stimulus to the subject, the method comprising administering the CAMK1D inhibitor to the subject

In a **sixth aspect,** the invention pertains to a CAMK1D inhibitor for use in the treatment of a proliferative disorder in a subject in need of such treatment, the treatment comprising a step of administering to the subject the CAMK1D inhibitor recited in any one of the preceding claims, in an effective amount to sensitise cells involved with the proliferative disorder in the subject to a death receptor stimulating agent

In a **seventh aspect,** the invention pertains to a method for determining whether a subject has, or is at risk of, developing a proliferative disorder, such as a tumour, that is associated with cellular resistance against a cell-dependent or cell-independent cytotoxic stimulus, such as of a cell-mediated immune response, the method comprising the steps of:
- detecting an applicable biomarker in a biological sample from said subject;
wherein the detection of the applicable biomarker in the sample indicates the proliferative disorder, or a risk of developing the proliferative disorder, in the subject; and
wherein the applicable biomarker is one or more selected from the group consisting of:
- CAMK1D, in particular the presence (or an amount) of or expression and/or activity of CAMK1D, preferably of phosphorylated CAMK1D;
- A death receptor, in particular the presence (or an amount) of or expression and/or activity of a death receptor;
- A death receptor ligand or a cell expressing a death receptor ligand, such as a FAS ligand, in particular the presence (or an amount) of or expression and/or activity of a death receptor ligand:
   Preferably, wherein the applicable biomarker is detected in cells involved with the proliferative disorder contained in the biological sample.

In **an eighth aspect,** the invention pertains to a method for determining whether a subject has, or has a risk of developing, a disease, disorder or condition that is associated with resistance against pro apoptotic stimuli, such as pro apoptotic stimuli elicited by cell-mediated immune responses, and wherein the proliferative disorder is associated with expression or activity of CAMK1D, the method comprising the steps of:
- contacting cells of the subject suspected to be involved with the disease, disorder or condition with a CAMK1D inhibitor in the presence of a pro apoptotic signal: (i) immune cells capable of eliciting or eliciting pro apoptotic stimuli towards cells involved with the proliferative disease, disorder or condition, such as lymphocytes, NK cells, T-cells, CTLs and TILs; or (ii) a pro apoptotic stimulus such as a soluble or substrate bound death receptor agonist or ligand; and
- determining initiation of apoptosis in the cells involved with the proliferative disorder of the subject,
wherein an enhancement of the initiation of apoptosis in the cells of the subject indicates that the subject has or has a risk of developing such disease, disorder or condition.

In a **ninth aspect,** the invention pertains to a method for stratifying a subject that suffers from a proliferative disorder into a patient group that is distinguished by having a poor prognosis or into a patient group that does not have a poor prognosis, the method comprising the steps of:
- detecting an applicable biomarker in a biological sample from said subject, in particular wherein the biological sample comprises cells involved with the proliferative disorder;
wherein the detection of the applicable biomarker in the sample indicates that the subject is stratified into the group of patients having a poor prognosis, and wherein no detection of the applicable biomarker in the sample indicates that the subject is stratified into the group of patients not having a poor prognosis; and
wherein the applicable biomarker is one, preferably both, selected from the group consisting of:
- CAMK1D, in particular the presence (or an amount) of or expression and/or activity of CAMK1D, preferably of phosphorylated CAMK1D; or
- a death receptor, in particular the presence (or an amount) of or expression and/or activity of a death receptor;
Preferably, wherein the applicable biomarker is detected in cells involved with the proliferative disorder contained in the biological sample.

In **a tenth aspect,** the invention pertains to a use of an antigen binding protein (ABP) capable of binding specifically to CAMK1D or phosphorylated CAMK1D in an *in-vitro* diagnosis of a proliferative disease, disorder or condition in a subject; wherein the proliferative disease, disorder or condition is associated with resistance against a pro apoptotic signal, such as pro apoptotic stimuli elicited by cell-mediated immune responses, and wherein the proliferative disease, disorder or condition is associated with expression or activity of CAMK1D.

In **an eleventh aspect,** the invention pertains to a use of a kit in an *in-vitro* diagnosis of a proliferative disease, disorder or condition in a subject, wherein the proliferative disease, disorder or condition is associated with resistance against a pro apoptotic stimuli, such as pro apoptotic stimuli elicited by cell-mediated immune responses, for example which are mediated by a TNR6 ligand positive immune cell, and wherein the proliferative disease, disorder or condition is associated with expression or activity of CAMK1D, wherein the kit comprises: (a) either (x) a nucleic acid capable of binding specifically to CAMK1D, or (y) an ABP binding specifically to CAMK1D; and (b) optionally (i) instructions describing how to use the ABP or a nucleic acid or kit for detecting CAMK1D activity in a sample obtained from a subject; and/or (ii) one or more other item, component, reagent or other means useful for the use of the kit or the detection of CAMK1D activity in the sample.

In the eleventh aspect the invention also pertains to a kit for use in a diagnostic method for determining whether a subject has, or has a risk of developing, a disease, disorder or condition that is associated with resistance against a pro apoptotic stimulus, such as a cell-mediated response mediated by a TNR6 ligand positive immune cell, and that is associated with expression or activity of CAMK1D; wherein:
- the diagnostic method comprises a step of surgically obtaining a sample from the subject; and
- the kit comprises: (a) either (x) a nucleic acid capable of binding specifically to CAMK1D, or (y) an ABP binding specifically to CAMK1D; and (b) optionally (i) instructions describing how to use the ABP or a nucleic acid or kit for detecting CAMK1D activity in the sample; and/or (ii) one or more other item, component, reagent or other means useful for the use of the kit or the detection of CAMK1D activity in the sample.

In **a twelfth aspect,** the invention pertains to a method for identifying and/or characterising a compound suitable for a treatment of a disease, disorder or condition that is characterised by resistance against death receptor signalling, in particular resistant against a cell-mediated immune response, and that is characterised by expression or activity of CAMK1D, the method comprising the steps of:
(a) bringing into contact a first cell or cell-free system which comprises and/or expresses CAMK1D mRNA or protein and (i) a candidate compound, or (ii) a candidate compound and a cell-dependent or cell-independent cytotoxic stimulus; and
(b) determining (i) the expression, activity, function and/or stability of the (eg protein or mRNA of) CAMK1D, in the first cell or cell-free system; and/or (ii) the cytotoxicity of the cell-dependent or cell-independent cytotoxic stimulus against the first cell or cell-free system;
wherein: (i) a reduced expression, activity function and/or stability of the (eg protein or mRNA of) CAMK1D, in said first cell or cell-free system contacted with the candidate compound compared to said first cell or cell-free system not contacted with said candidate compound; and/or (ii) an enhanced cytotoxicity of the cell-dependent or cell-independent cytotoxic stimulus against the first cell or cell-free system contacted with the candidate compound compared to the cytotoxicity of the cell-dependent or cell-independent cytotoxic stimulus against the first cell or cell-free system not contacted with the candidate compound; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.

In **a thirteenth aspect,** the invention pertains to a method for identifying and/or characterising a compound suitable for a treatment of a disease, disorder or condition that is characterised by resistance against death receptor signalling, in particular resistant against a cell-mediated immune response, and that is characterised by expression or activity of CAMK1D, the method comprising the steps of:
(a) bringing into contact a first cell or cell free system which comprises and/or expresses CAMK1D mRNA or protein and one or more effector caspase(s) - in particular caspase-3, -6 and/or -7 - mRNA or protein, and (i) a candidate compound, or (ii) a candidate compound, and a cell-dependent or cell-independent cytotoxic stimulus; and
(b) determining (i) the expression, activity, function and/or stability of the (eg protein or mRNA of) of the one or more effector caspase(s) - in particular caspase-3, -6 and/or -7 - in the first cell or cell-free system; and/or (ii) the cytotoxicity of the cell-dependent or cell-independent cytotoxic stimulus against the first cell or cell-free system;
wherein: (i) an increased expression, activity function and/or stability of the (eg protein or mRNA of) one or more effector caspase(s) - in particular caspase-3, -6 and/or -7 - in the first cell or cell-free system, in said first cell or cell-free system contacted with the candidate compound compared to said first cell or cell-free system not contacted with said candidate compound; and/or (ii) an enhanced cytotoxicity of the cell-dependent or cell-independent cytotoxic stimulus against the first cell or cell-free system contacted with the candidate compound compared to the cytotoxicity of the cell-dependent or cell-independent cytotoxic stimulus against the first cell or cell-free system not contacted with the candidate compound; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.

In **a fourteenth aspect,** the invention pertains to a method for identifying and/or characterising a compound suitable for a treatment of a disease, disorder or condition that is characterised by resistance against death receptor signalling, in particular resistant against a cell-mediated immune response, and that is characterised by expression or activity of CAMK1D, the method comprising the steps of:
(a) bringing into contact a first cell or cell free system which comprises and/or expresses CAMK1D mRNA or protein and calmodulin mRNA or protein, and (i) a candidate compound, or (ii) a candidate compound, and a cell-dependent or cell-independent cytotoxic stimulus; and
(b) determining or detecting (i) the expression, activity, function and/or stability of the (eg protein or mRNA of) of calmodulin in the first cell or cell-free system; and/or (ii) the specific binding of a Ca2+/calmodulin protein complex to CAMK1D protein;
wherein: (i) an increased expression, activity function and/or stability of the (eg protein or mRNA of) calmodulin in the first cell or cell-free system, in said first cell or cell-free system contacted with the candidate compound compared to said first cell or cell-free system not contacted with said candidate compound; and/or (ii) a reduced specific binding of a Ca2+/calmodulin protein complex to CAMK1D protein in the presence of the candidate compound compared to the absence of the candidate; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.

In **a fifteenth aspect,** the invention pertains to a method for identifying and/or characterising a compound suitable for a treatment of a disease, disorder or condition that is characterised by resistance against death receptor signalling, in particular resistant against a cell-mediated immune response, and that is characterised by expression or activity of CAMK1D, the method comprising the steps of:
(a) bringing into contact a first cell or cell free system which comprises and/or expresses CAMK1D mRNA or protein and CAMK kinase (CAMKK) mRNA or protein, and (i) a candidate compound, or (ii) a candidate compound, and a cell-dependent or cell-independent cytotoxic stimulus; and
(b) determining or detecting (i) the expression, activity, function and/or stability of the (eg protein or mRNA of) of CAMKK in the first cell or cell-free system; and/or (ii) the specific binding of a CAMKK protein to CAMK1D protein;
wherein: (i) an increased expression, activity function and/or stability of the (eg protein or mRNA of) CAMKK in the first cell or cell-free system, in said first cell or cell-free system contacted with the candidate compound compared to said first cell or cell-free system not contacted with said candidate compound; and/or (ii) a reduced specific binding of a CAMKK protein to CAMK1D protein in the presence of the candidate compound compared to the absence of the candidate; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.

In **a sixteenth aspect,** the invention pertains to a method for identifying and/or characterising a compound suitable for a treatment of a disease, disorder or condition that is characterised by resistance against death receptor signalling and that is characterised by expression or activity of CAMK1D, the method comprising the steps of:
(a) bringing into contact a first cell which comprises and/or expresses CAMK1D mRNA or protein and (i) a candidate compound, or (ii) a candidate compound and a death receptor stimulating agent; and
(b) determining (i) the expression, activity, function and/or stability of the (eg protein or mRNA of) CAMK1D, in the first cell; and/or (ii) the cytotoxicity of the agonist of death receptor signalling against the first cell,
wherein: (i) a reduced expression, activity function and/or stability of the (eg protein or mRNA of) CAMK1D, in said first cell contacted with the candidate compound compared to said first cell not contacted with said candidate compound; and/or (ii) an enhanced cytotoxicity of agonist of death receptor signalling against the first cell contacted with the candidate compound compared to the cytotoxicity of the death receptor stimulating agent against the first cell not contacted with the candidate compound; indicates that the candidate compound is a compound suitable for the treatment of the disease, disorder or condition that is characterised by cellular resistance against death receptor signalling and that is characterised by expression or activity of CAMK1D.

### DETAILED DESCRIPTION OF THE INVENTION

In the following, the elements of the invention will be described. These elements are listed with specific embodiments and aspects, however, it should be understood that they may be combined in any manner and in any number to create additional embodiments and aspects. The variously described examples and preferred embodiments should not be construed to limit the present invention to only the explicitly described embodiments and aspects. This description should be understood to support and encompass embodiments and aspects which combine two or more of the explicitly described embodiments and aspects or which combine the one or more of the explicitly described embodiments and aspects with any number of the disclosed and/or preferred elements. Furthermore, any permutations and combinations of all described elements in this application should be considered disclosed by the description of the present application unless the context indicates otherwise.

In **a first aspect,** the invention pertains to a Calcium/calmodulin-dependent protein kinase type 1D (CAMK1D) inhibitor for use in a treatment of a proliferative disorder in a subject, wherein the treatment comprises administering the CAMK1D inhibitor to the subject.

In this first aspect, the invention pertains to a CAMK1D inhibitor for use in a treatment of a proliferative disorder in a subject, wherein the treatment involves inhibiting an activity, function, expression and/or stability of CAMK1D, preferably thereby sensitising cells involved with the proliferative disorder to a cell-dependent or cell-independent cytotoxic stimulus; wherein the treatment comprises administering the CAMK1D inhibitor to the subject.

In one alternative first aspect, and as may be further described, defined, claimed or otherwise disclosed herein, the invention relates to a method for the treatment of a proliferative disorder in a subject by sensitising cells involved with the proliferative disorder to a cell-dependent or cell-independent cytotoxic stimulus, preferably which is a cell-mediated immune response, the method comprising administering a CAMK1D inhibitor to the subject.

In another alternative first aspect, and as may be further described, defined, claimed or otherwise disclosed herein, the invention relates to a method for the treatment of a proliferative disorder in a subject, by inhibiting CAMK1D and (for example, thereby) sensitising cells involved with the proliferative disorder to a cell-dependent or cell-independent cytotoxic stimulus, preferably a cell-mediated immune response, the method comprising administering a CAMK1D inhibitor to the subject.

In one related first aspect, and as may be further described, defined, claimed or otherwise disclosed herein, the invention relates to an inhibitor of CAMK1D for use in the treatment of a proliferative disorder in a subject, wherein the treatment involves (eg is mediated by): (i) sensitising cells involved with the proliferative disorder to a cell-dependent or cell-independent cytotoxic stimulus, preferably which is a cell-mediated immune response; and/or (ii) inhibiting CAMK1D. In another related first aspect, and as may be further described, defined, claimed or otherwise disclosed herein, the invention pertains to a use of a CAMK1D inhibitor for the manufacture of a medicament for the treatment of a proliferative disease in a subject, wherein the treatment involves (eg is mediated by): (i) sensitising cells involved with the proliferative disorder to a cell-dependent or cell-independent cytotoxic stimulus, preferably which is a cell-mediated immune response; and/or (ii) inhibiting CAMK1D. Preferably the manufacture of the medicament includes a step of preparing, formulating, or otherwise providing, the CAMK1D inhibitor in a form suitable for specific delivery of the CAMK1D inhibitor to cells involved with the proliferative disorder. In certain embodiments of these related embodiments, in such treatment the inhibitor: (i) sensitises the cells involved with the proliferative disorder to the cell-dependent or cell-independent cytotoxic stimulus, preferably which is a cell-mediated immune response; and/or (i) inhibits CAMK1D. In certain of such embodiments, the CAMK1D inhibitor is one capable of: (i) sensitising cells involved with the proliferative disorder to a cell-dependent or cell-independent cytotoxic stimulus, preferably which is a cell-mediated immune response; and/or (ii) inhibiting CAMK1D.

In a further aspect, and as may be further described, defined, claimed or otherwise disclosed herein, the invention relates to a method for the sensitisation of cells involved with a proliferative disorder to a cell-dependent or cell-independent cytotoxic stimulus, preferably which is a cell-mediated immune response, in the treatment of the proliferative disorder in a subject, the method comprising administering a CAMK1D inhibitor to the subject; and in another further aspect, and as may be further described, defined, claimed or otherwise disclosed herein, the invention relates to a method for the inhibition of CAMK1D in the treatment of a proliferative disorder in a subject, the method comprising administering a CAMK1D inhibitor to the subject.

"Calcium/calmodulin-dependent protein kinase type 1D" or "CAMK1D" is a member of the calcium/calmodulin-dependent protein kinase 1 family, a subfamily of the serine/threonine kinases. The encoded protein is a component of the calcium-regulated calmodulin-dependent protein kinase cascade and is an intracellular located protein. It has been associated with multiple processes including regulation of granulocyte function, activation of CREB-dependent gene transcription, aldosterone synthesis, differentiation and activation of neutrophil cells. Alternatively spliced transcript variants encoding at least two different protein isoforms of this gene have been described. Pertinent information on the human CAMK1D protein is accessible on UniProt: Q8IU85 (Entry version 13-May-2020), and more preferably comprises an amino acid sequence shown in any of SEQ ID NOs: 1 or 2. A Calcium/calmodulin-dependent protein kinase operates in the calcium-triggered CaMKK-CaMKi signalling cascade and, upon calcium influx, gets bound and activated by a Ca2+/calmodulin complex and in turn activates CREB-dependent gene transcription. In neutrophil cells, CAMK1D is required for cytokine-induced proliferative responses and activation of the respiratory burst. The human CAMK1D gene is located on chromosome 10: 12,349,482-12,835,545 forward strand. The term CAMK1D in some embodiments of the invention may also pertain to variants of the human CAMK1D protein having an amino acid sequence that is substantially identical to, or of at least 80%, preferably 85%, more preferably 90, 95, 96, 97, 98, 99, or 100% sequence identity to, the amino acid sequence shown in any of SEQ ID NO: 1 or 2, as determined using, e.g., the "Blast 2 sequences" algorithm described by Tatusova & Madden 1999 (FEMS Microbiol Lett 174: 247-250), and which (preferably) retain biological activity identical or substantially identical to the respective reference CAMK1D (eg to bind to and phosphorylate one or more caspases such as caspase-3, -6 and/or -7). Preferred variants of CAMK1D protein comprise sequence variants thereof due to sequence polymorphism between and within populations of the respective species, as well as mutations compared to the wild-type sequence of CAMK1D.

An "inhibitor of CAMKiD" (or "CAMK1D inhibitor") is any moiety that inhibits CAMK1D, which can mean inhibition of the expression (eg the amount), function, activity and/or stability of CAMK1D, especially of mRNA and/or protein of CAMK1D, and in particular of activated, eg phosphorylated CAMK1D. A CAMK1D inhibitor may impair, suppress, reduce and/or lower the expression of CAMK1D (eg CAMK1D mRNA or protein) in a cell. The term "expression" means in this context the cellular process of transcribing a gene into an mRNA and the following translation of the mRNA into a protein (and in certain embodiment, the subsequent transport and localisation of such protein). "Gene expression" therefore may thus refer only to the generation of mRNA, irrespectively from the fate of the so produced mRNA, or alternatively/additionally to the translation of the expressed mRNA into a protein (or transport and localisation of such protein). The term "protein expression" on the other hand may refer to the complete cellular process of synthesis of proteins and/or transport/localisation thereof into certain cellular compartments. A CAMK1D inhibitor may impair (eg, induces a decrease or reduction in) the efficiency, effectiveness, amount or rate of one or more activities of CAMK1D (for example, by impairing the expression of CAMK1D protein and/or amount of phosphorylated CAMK1D protein), such as one or more of those activities described herein, for example, the activity of CAMK1D to phosphorylate one or more caspases, in particular effector caspases such as caspase-3, -6 and/or -7, and/or to sensitise a cell involved with a proliferative disorder to a cytotoxic stimulus, such as a cell-mediated immune response. A CAMK1D inhibitor may have a negative effect towards the stability of CAMK1D (eg CAMK1D mRNA or protein), which shall be understood in its broadest sense, and shall include inhibitors which, for example, interfere with and reduce the CAMK1D protein half-life or interfere with and disturb CAMK1D protein folding, protein presentation or transport/localisation within the cell.

Such a CAMK1D inhibiting moiety can act directly, for example, by binding to CAMK1D and decreasing the amount or rate of one or more of the properties of CAMK1D such as its expression, function and/or stability, in particular its ability to act as a kinase (eg to phosphorylate effector caspases), for example by reducing the amount or activity of phosphorylated CAMK1D in the cell. A CAMK1D inhibitor may also decrease the amount or rate of CAMK1D function or activity by impairing its expression, stability, for example, by binding to CAMK1D protein or mRNA and modifying it, such as by removal or addition of a moiety, or altering its three-dimensional conformation; and by binding to CAMK1D protein or mRNA and reducing its stability or conformational integrity. A CAMK1D inhibitor may, alternatively, act indirectly, for example, by binding to a regulatory molecule or gene region to modulate such regulatory protein or gene region function and hence consequentially affect a decrease in the amount or rate of CAMK1D expression (eg amount), function/activity and/or stability, in particular by impairing one or more activity of CAMK1D protein or mRNA (such as by changing the amount or rate of expression and/or stability of CAMK1D protein or mRNA). Thus, a CAMK1D inhibitor can act by any mechanisms that impair, such as result in a decrease in, the amount or rate of CAMK1D expression (eg amount), function/activity and/or stability. Non-limiting examples of CAMK1D inhibitors that act directly on CAMK1D include: (i) siRNA or shRNA molecules that bind to and reduce expression of CAMK1D mRNA; (ii) small molecule moieties that bind to the catalytic domain of CAMK1D and reduce the kinase activity of CAMK1D, and (iii) gene editing components such as guide nucleic acids (gRNA or gDNA).

In some preferred embodiments of the invention a CAMK1D inhibitor moiety is an allosteric inhibitor of CAMK1D. The term "allosteric CAMK1D inhibitor" refers to an agent, and preferably a small molecular compound as described herein, inhibiting a protein or a protein-protein interaction (e.g. the binding of CAMK1D to a substrate caspase) by binding at a site other than the protein's active site or protein-protein binding site, in one embodiment of the invention, an allosteric inhibitor is an agent, preferably a small molecule, that binds to CAMK1D or to any one of caspase-3, -6, and/or -7, at a site other than the binding site. In addition, other than its binding to effector caspases, the allosteric inhibitor of CAMK1D can interfere with a protein-protein interaction between CAMK1D and Calmodulin (CaM) or CAMK kinase (CAMKK).

In another embodiment of the invention, which may be preferred, the CAMK1D inhibitor is a competitive inhibitor of CAMK1D. The term "competitive CAMK1D inhibitor" refers to an agent, and preferably a small molecular compound as described herein, inhibiting a protein or a protein-protein interaction (e.g. the binding of CAMK1D to a substrate caspase) by binding at a site close to or similar to the protein's active site or to a protein-protein binding site. A competitive inhibitor may in addition refer to a CAMK1D inhibitor that binds to CAMK1D kinase active site, or to a direct interaction (binding) site of CAMK1D and one of its substrate effector caspases, such as caspase-3, -6, and/or -7. In addition, other than its binding to effector caspases, the competitive inhibitor of CAMK1D can interfere with a protein-protein interaction between CAMK1D and Calmodulin (CaM) or CAMK kinase (CAMKK).

General and specific examples of CAMK1D inhibitors are described elsewhere herein, including those as may be characterised by the applicable functional and/or structural features set out herein.

In a related further aspect, and as may be further described, defined, claimed or otherwise disclosed herein, the invention relates to an inhibitor of CAMK1D (eg, a CAMK1D inhibitor) for use as a medicament for: (i) sensitising cells involved with a proliferative disorder to a cell-mediated immune response; and/or (ii) inhibiting CAMK1D.

In yet a related further aspect, and as may be further described, defined, claimed or otherwise disclosed herein, the invention relates to a CAMK1D inhibitor for use as a medicament (eg an immuno-oncology medicament) sensitising cells involved with a proliferative disorder (such as a tumour or cancer) to a cell-mediated immune response, for example sensitising cells involved with a proliferative disorder to killing (cell-death) that may be induced by the cell-mediated immune response. An "immune-oncology" medicament is one that would be recognised by the person of ordinary skill, and includes a medicament that is intended to (eg, specifically designed to) enhance one or more components of the immune system of an organism (such as a human) towards cancerous or tumourous cells present in such organism. An immune-oncology medicament may be one (eg an antibody) than binds to an extrinsic immune (inhibitory) checkpoint molecule (such as one described elsewhere herein) and that (eg directly) suppresses T cell function against the cancerous or tumourous cells, or an immune-oncology medicament may be one that inhibits an immune regulator (such as CAMK1D, as in the present invention) that is intrinsic to the cancerous or tumourous cells where such intrinsic immune regulator does not actively (eg directly) suppress T cells but rather protects the tumour or cancer cells from an immune response via a resistance mechanism.

In particular embodiments of such aspects, the cells involved with a proliferative disorder may be sensitised to killing (preferably cell-death by apoptosis) by (such as induced by) the a cell-dependent or cell-independent cytotoxic stimulus, such as preferably elicited by a cell-mediated immune response. A "proliferative disorder" refers to a disorder characterised by abnormal proliferation of cells. A proliferative disorder does not imply any limitation with respect to the rate of cell growth, but merely indicates loss of normal controls that affect growth and cell division. Thus, in some embodiments, cells of a proliferative disorder can have the same cell division rates as normal cells but do not respond to signals that limit such growth. Within the ambit of "proliferative disorder" is neoplasm or tumour, which is an abnormal growth of tissue or cells. Cancer is art understood, and includes any of various malignant neoplasms characterised by the proliferation of cells that have the capability to invade surrounding tissue and/or metastasise to new colonisation sites. Proliferative disorders include cancer, atherosclerosis, rheumatoid arthritis, idiopathic pulmonary fibrosis and cirrhosis of the liver. Non-cancerous proliferative disorders also include hyperproliferation of cells in the skin such as psoriasis and its varied clinical forms, Reiter's syndrome, pityriasis rubra pilaris, and hyperproliferative variants of disorders of keratinisation (e.g., actinic keratosis, senile keratosis), scleroderma, and the like.

In more particular embodiments, the proliferative disorder is a cancer or tumour, in particular a solid tumour (including a condition or symptom associated with such cancer or tumour). Such proliferative disorders including but not limited to head and neck cancer, squamous cell carcinoma, multiple myeloma, solitary plasmacytoma, renal cell cancer, retinoblastoma, germ cell tumours, hepatoblastoma, hepatocellular carcinoma, melanoma, rhabdoid tumour of the kidney, Ewing Sarcoma, chondrosarcoma, any haemotological malignancy (e.g., chronic lymphoblastic leukemia, chronic myelomonocytic leukemia, acute lymphoblastic leukemia, acute lymphocytic leukemia, acute myelogenous leukemia, acute myeloblasts leukemia, chronic myeloblastic leukemia, Hodgekin's disease, non-Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, marginal zone lymphoma, Burkitt Lymphoma, mycosis fungoides, seary syndrome, cutaneous T-cell lymphoma, peripheral T cell lymphoma, chronic myeloproliferative disorders, myelofibrosis, myeloid metaplasia, systemic mastocytosis), and central nervous system tumours (eg, brain cancer, glioblastoma, non- glioblastoma brain cancer, meningioma, pituitary adenoma, vestibular schwannoma, a primitive neuroectodermal tumour, medulloblastoma, astrocytoma, anaplastic astrocytoma, oligodendroglioma, ependymoma and choroid plexus papilloma), myeloproliferative disorders (eg, polycythemia vera, thrombocythemia, idiopathic myelfibrosis), soft tissue sarcoma, thyroid cancer, endometrial cancer, carcinoid cancer, or liver cancer.

The cell that is sensitised to the cell-dependent or cell-independent cytotoxic stimulus, such as preferably elicited by a cell-mediated immune response, is one involved with the proliferative disorder (eg, a cell associated with the proliferative disorder), which in certain embodiments such cell is one involved in the proliferative disorder (eg, a cell that is abnormally proliferating, such as one that is over-proliferating). For example, such cell may be a cell characterised by loss of normal controls that affect its growth and cell division, such as a cell of a neoplasm or tumour. In particular embodiments, such cell may be a cancerous cell or one that is derived form or is a cell of a cancer or tumour. In other embodiments, such cell may be skin cell, such as one showing hyperproliferation such as one involved in psoriasis, Reiter's syndrome, pityriasis rubra pilaris or scleroderma.

A cell may be "involved with a proliferative disorder" if, for example, it is associated therewith, such as it being a causative factor in such proliferative disorder or if it is affected by such proliferative disorder. In particular a cell is "involved with a proliferative disorder" if the cell is characterised by an abnormal proliferation such as abnormal cell growth or cell division, and if the abnormal cell growth or cell division is part of the pathology of, or causative for, the proliferative disease. A cell "involved with a proliferative disorder", in those embodiments wherein the proliferative disorder is a tumour or cancer, can as a non-limiting example, be a tumour (or cancer) cell, or a cell of derived from (tissue) of such tumour or cancer; in particular of a solid tumour.

In particular embodiments, the CAMK1D inhibitor is administered in an amount (such as a therapeutically effective amount) that is effective to reduce activity of CAMK1D, preferably of CAMK1D in (of) the cells involved with the proliferative disorder. In such embodiments, a "therapeutically effective amount" of the CAMK1D inhibitor can be an amount that is capable to reduce the activity of the CAMK1D to an applicable level, but that does not lead to significant (eg intolerable) side effects or over-dosage in respect of other activities of the CAMK1D inhibitor.

Preferably, the activity of CAMK1D is effectively inhibited (reduced) in the cells involved with a proliferative disorder. For example, an "effective" inhibition (or reduction) may include one where the activity of intracellular CAMK1D is lowered by a degree (or to a level) that has a physiological effect (eg to a therapeutically effective level), such as a reduction by about 10%, 20%, 50%, or more than 50% such as 70% or 90% of activity of the respective kinase.

The term "immune cell" is art recognised to describe any cell of an organism involved in the immune system of such organism, in particular of a mammal such as a human. Leukocytes (white blood cells) are immune cells that are involved in the innate immune system, and the cells of the adaptive immune system are special types of leukocytes, known as lymphocytes. B cells and T cells are the major types of lymphocytes and are derived from hematopoietic stem cells in the bone marrow. B cells are involved in the humoral immune response, whereas T cells are involved in cell-mediated immune response. In preferred embodiments of the invention, the immune cell can be a myeloid cell eg a T cell, and in particular (such as when an increase in cell-mediated immune response is required, such as to treat a cancer) the T cell can be a cytotoxic T cell (also known as TC, cytotoxic T lymphocyte, CTL, T-killer cell, cytolytic T cell, CD8+ T-cell or killer T cell). A CTL is a T-cell that is involved in the killing of cancer cells, cells that are infected (particularly with viruses), or cells that are damaged in other ways. Other preferred immune cells for such embodiments can include Tumour-Infiltrating Lymphocytes (TILs). TILs are white blood cells that have left the bloodstream and migrated into a tumour. Typically, TILs are a mix of different types of cells (i.e., T cells, B cells, NK cells, macrophages) in variable proportions, T cells being the most abundant cells. TILs can often be found in the stroma and within the tumour itself, and are implicated in killing tumour cells. The presence of lymphocytes in tumours is often associated with better clinical outcomes. In some embodiments an "immune cell" in context of the invention can be a "natural killer" or "NK" cell. NK cells are known to express Fas ligand and thereby induce apoptotic self-killing in tumour cells and thus are similar to CTL a source for a cell-dependent cytotoxic stimulus in context of the invention.

The term "cell dependent cytotoxic stimulus" shall refer to a cytotoxic stimulus or signal, such as through a cytokine or other protein, being or elicited by any cell, or cellular component, be it either secreted by the cell or be it membrane bound, such as a membrane bound ligand or receptor, which has an activity and/or function to induce cell death, in particular apoptosis, in a cell contacted with the cell, or cellular component. On the other hand the term "cell-independent cytotoxic stimulus" shall refer to a substance or composition that has an activity and/or function to induce cell death, in particular apoptosis in a cell contacted with said substance or composition, but wherein the substance or composition is active or present independently of the presence of a cell, such as an immune cell, secreting or presenting such substance or composition. A "cell-independent cytotoxic stimulus" may be for example a soluble substance administered to a subject to induce cytotoxic, in particular apoptotic, responses in a target cell. A cell dependent cytotoxic stimulus of the invention is preferably a cytotoxic stimulus that is elicited in context of a cell-mediated immune response, such as a response by a cytotoxic T lymphocyte (CTL) or natural killer (NK) cell. In certain preferred embodiments of the invention the cell-dependent or cell-independent cytotoxic stimulus is capable of inducing or increasing apoptosis in the cells involved with the proliferative disorder, and/or preferably wherein the cell-dependent cytotoxic stimulus is cell-mediated immune response, such as a cytotoxic T-lymphocyte (CTL) response, or natural killer (NK) cell response, and wherein preferably the cell-mediated immune response involves the expression and/or secretion, such as a cell-surface expression or secretion, of at least one immune cell effector molecule (e.g. TNR6 (Fas) ligand or soluble or membrane bound TRAIL). In certain such preferred embodiments the cell-dependent or cell-independent cytotoxic stimulus is capable of inducing death receptor signalling dependent apoptosis in cells involved with the proliferative disorder, preferably wherein the death receptor is of the Tumour Necrosis Factor Receptor (TNFR) superfamily. A cell-dependent or cell-independent cytotoxic stimulus in context of the invention may be, in preferred embodiments, selected from a substance or composition capable of binding to, and activating or increasing an activity of, a death receptor signalling pathway in the cells involved with the proliferative disorder, for example selected from (i) an agonist of TNR6 signalling (such as an agonistic anti-TNR6 antibody, a membrane bound or soluble TNR6 ligand (FAS ligand), or (ii) an agonist of TRAIL receptor signalling, such as a TRAIL or an agonistic anti-TRAIL receptor (DR4 or DR5) antibody.

The term "cell-mediated immune response", as used herein, may include, but is not limited to, a response in a host organism involving, utilising, and/or promoting any one or combinations of T cell maturation, proliferation, activation, migration, infiltration and/or differentiation, and/or the activation/modulation/migration/infiltration of a macrophage, a natural killer cell, a T lymphocyte (or T cell), a helper T lymphocyte, a memory T lymphocyte, a suppressor T lymphocyte, a regulator T lymphocyte, and/or a cytotoxic T lymphocyte (CTL), and/or the production, release, and/or effect of one or more cell-secretable or cell-secreted factor (soluble and membrane bound) such as a cytokine or autocoid (in particular a pro apoptotic protein or small molecular compound, such as a TNR6 ligand or TRAIL), and/or one or more components of any of such processes (in particular a pro apoptotic protein or small molecular compound such as a TNR6 ligand or TRAIL). The term "cell-mediated immune response," as used herein, may include a cellular response involving a genetically engineered, in-vitro cultured, autologous, heterologous, modified, and/or transferred T lymphocyte, or it may include a cell-secretable or cell-secreted factor (in particular a pro apoptotic protein or small molecular compound such as a TNR6 ligand or TRAIL) produced by genetic engineering. A cell-mediated immune response is preferably not a humoral immune response, such as an immune response involving the release of antibodies. In certain embodiments, in particular when the proliferative disorder is a cancer or tumour, the cell-mediated immune response is an anti-tumour cell-mediated immune response. For example, one that leads to a reduction in tumour (cell) growth, such as a cytotoxic cell-mediated immune response (in particular involving a pro apoptotic protein or small molecular compound such as a TNR6 ligand or TRAIL) that kills cells of the cancer or tumour.

The terms "sensitising", "sensitisation" and "to sensitise" (and the like), as used herein in the context of cell(s) being sensitised to a cell-dependent or cell-independent cytotoxic stimulus, such as a cell-mediated immune response, will be understood by the person of ordinary skill, and include the meaning that such cells can exhibit an increased susceptibility to one or more effect (eg a treatment effect) that the cell-mediated immune response may have on such cells. In particular, cells that are so sensitised may, when in the presence of (eg exposed to) a cytotoxic stimulus, be killed more easily (such as more rapidly, a greater proportion of cells dying or being killed and/or upon a lower amount or exposure of the cell-mediated immune response) than analogous cells that have not been so "sensitised". For example, cell(s) so sensitised may be induced into cell-death (eg apoptosis) upon exposure to a lower number of T cells or to a lower concentration of a death receptor agonist (such as about 10%, 20%, 30% 40%, 50% or more than 50% fewer T cells or lower concentration of a death receptor agonist). Methods to determine whether such cells have been sensitised (and by which degree) to cell-mediated immune responses are described herein, such as in the examples. Accordingly, in certain embodiments of the present invention, cells involved with the proliferative disorder may be sensitised to cell-death/killing (eg by entry into apoptosis) by a cell-mediated immune response (such as CTL or a death receptor agonist, such as a TNR6 ligand or TRAIL).

The term "death receptor" refers to a cell- surface receptor that induces cellular apoptosis once bound by a ligand or a functional equivalent of a ligand (such as an agonistic antibody). Death receptors preferably include tumour necrosis factor (TNF) receptor superfamily members having death domains (e.g., receptors known as TNFR1, TNR6 (Fas), DR3, DR4, DR5, DR6, and LTpR).

The term "death receptor agonist" refers to a substance that is capable of binding a death receptor on a cell and initiating apoptosis. For example, a "death receptor agonist small molecule" is a compound that is capable of interacting with the death receptor to initiate apoptosis. Suitable death receptor agonists include any substance (molecule, drug, protein, etc.) that is capable of binding a death receptor on a cell and initiating apoptosis in the cell expressing the death receptor. The death receptor agonist can be a natural ligand of a death receptor, including fragments or variants or soluble versions of the natural ligand. The death receptor agonist can be an antibody that binds and activates a death receptor. The death receptor agonist can be a compound, such as a small molecule identified from a compound library. For example, the death receptor agonist can be a death receptor ligand that initiates apoptosis when it binds a death receptor on a cell. For example, death receptor ligand can be a member of the TNF superfamily. In preferred embodiments, the death receptor ligand is a TNR6 ligand (also known as FAS ligand) or is TNF-related apoptosis-inducing ligand (TRAIL).

TRAIL has a strong apoptosis-inducing activity against cancer cells. Unlike other death-inducing ligands of the TNF superfamily such as TNFα and TNR6 ligand, TRAIL preferentially induces apoptosis of tumour cells, having little or no effect on normal cells. At least five receptors for TRAIL have been identified, two of which, DR4 (TRAIL-Ri) and DR5 (TRAIL-R2), are capable of transducing the apoptosis signal whereas the other three (TRAIL-R3, TRAIL-R4 and OPG) serve as decoy receptors to block TRAIL-mediated apoptosis. The intracellular segments of both DR4 and DR5 contain a death domain and transduce an apoptosis signal through a FADD- and caspase 8-dependent pathway. Administration of the recombinant soluble form of TRAIL induces significant tumour regression without systemic toxicity in animal models. In humans, however, TRAIL has been shown to elicit side effects such as liver toxicity. Therefore, alternative agonists of TRAIL receptors have been developed. The death receptor agonist in context of the invention can be an apoptosis-inducing antibody that binds the death receptor. For example, the death receptor agonist can be an antibody specific for a death receptor, such that the antibody activates the death receptor. The agonist can be an antibody specific for TNR6, DR4 or DR5.

"Death receptor antibody" is used herein to refer generally to antibody or antibodies directed to a receptor in the tumour necrosis factor receptor superfamily and containing a death domain capable of signalling apoptosis, and such antibodies include a Fas, DR5 antibody and DR4 antibody. "DR5 receptor antibody", "DR5 antibody", or "anti-DRs antibody" is used in a broad sense to refer to antibodies that bind to at least one form of a DR5 receptor. Optionally the DR5 antibody is fused or linked to a heterologous sequence or molecule. Preferably the heterologous sequence allows or assists the antibody to form higher order or oligomeric complexes. Optionally, the DR5 antibody binds to DR5 receptor but does not bind or cross-react with any additional Apo-2L receptor (e.g. DR4, DcR1, or DcR2). Optionally the antibody is an agonist of DR5 signalling activity. Optionally, the DR5 antibody of the invention binds to a DR5 receptor at a concentration range of about 0.1 nM to about 20 mM as measured in a BIAcore binding assay. Optionally, the DR5 antibodies of the invention exhibit an Ic 50 value of about 0.6 nM to about 18 mM as measured in a BIAcore binding assay. A "DR4 receptor antibody", "DR4 antibody", or "anti-DR4 antibody" is used in a broad sense to refer to antibodies that bind to at least one form of a DR4 receptor or extracellular domain thereof. Optionally the DR4 antibody is fused or linked to a heterologous sequence or molecule. Preferably the heterologous sequence allows or assists the antibody to form higher order or oligomeric complexes. Optionally, the DR4 antibody binds to DR4 receptor but does not bind or cross-react with any additional Apo-2L receptor (e.g. DR5, DcR1 or DcR2). Optionally the antibody is an agonist of DR4 signalling activity. Optionally, the DR4 antibody of the invention binds to a DR4 receptor at a concentration range of about 0.1 nM to about 20 mM as measured in a BIAcore binding assay. Optionally, the DR4 antibodies of the invention exhibit an Ic 50 value of about 0.6 nM to about 18 mM as measured in a BIAcore binding assay. For example, the agonist can be a DR5 antibody having the same epitope specificity, or secreted by, a mouse-mouse hybridoma having ATCC Accession Number PTA-1428 (e.g., the TRA-8 antibody), ATCC Accession Number PTA-1741 (e.g., the TRA-1 antibody), ATCC Accession Number PTA-1742 (e.g., the TRA-10 antibody), or ATCC Accession Number PTA- 3798 (e.g., the 2E12 antibody). The death receptor agonist can be death receptor LTpR mAb (e.g., Biolegend Inc. clone 31 G4D8).

A "Fas antibody", "CD95 antibody", "TNR6-antbody" or "anti-Fas antibody" is used in a broad sense to refer to antibodies that bind to at least one form of a Fas receptor or extracellular domain thereof. Optionally the Fas antibody is fused or linked to a heterologous sequence or molecule. Preferably the heterologous sequence allows or assists the antibody to form higher order or oligomeric complexes. Optionally, the Fas antibody binds to Fas receptor but does not bind or cross-react with any additional death domain containing receptor (e.g. DR5, DR4 DcR1 or DcR2). Optionally the antibody is an agonist of Fas signalling activity. Optionally, the Fas antibody of the invention binds to a Fas receptor at a concentration range of about 0.1 nM to about 20 mM as measured in a BIAcore binding assay. Optionally, the Fas antibodies of the invention exhibit an Ic 50 value of about 0.6 nM to about 18 mM as measured in a BIAcore binding assay. In preferred embodiments of the invention the Fas antibody is an agonistic antibody which is to be understood in the sense that the Fas antibody binds and activates or increases activation of death receptor signalling by Fas.

In certain embodiments, the death receptor agonist is an immune effector molecule and is preferably a cell effector molecule selected from Fas ligand (TNFSF6/FASLG, FasL or CD95L) and TNF-related apoptosis-inducing ligand (TRAIL, CD253 or TNFSF10).

The term "Fas receptor", "FasR", "Tumour necrosis factor receptor superfamily member 6", "CD95 Antigen", "CD95" or "TNR6" are used synonymously herein and all refer to a death domain containing receptor for which pertinent information can be retrieved from UniProt under the identifier P25445 in the version of May-13 2020. The protein is a member of the TNF-receptor superfamily and contains a death domain. It has been shown to play a central role in the physiological regulation of programmed cell death, and has been implicated in the pathogenesis of various malignancies and diseases of the immune system. The interaction of this receptor with its ligand (FAS ligand) allows the formation of a death-inducing signalling complex that includes Fas-associated death domain protein (FADD), caspase 8, and caspase 10. The autoproteolytic processing of the caspases in the complex triggers a downstream caspase cascade, and leads to apoptosis. This receptor has been also shown to activate NF-kappaB, MAPK3/ERK1, and MAPK8/JNK, and is found to be involved in transducing the proliferating signals in normal diploid fibroblast and T cells.

The term "FAS ligand" or "Tumour Necrosis Factor Ligand Superfamily Member 6", "TNFSF6" "TNR6 ligand", "CD95L" or "CD95 ligand" are used synonymously herein and all refer to a protein which is a member of the tumour necrosis factor superfamily that binds to TNFRSF6/FAS, a receptor that transduces the apoptotic signal into cells. Pertinent information can be derived from the UniProt database under the accession number P48023 in the version of May 13, 2020. The primary function of the encoded transmembrane protein is the induction of apoptosis triggered by binding to FAS receptor. The FAS/FASLG signalling pathway is essential for immune system regulation, including activation-induced cell death (AICD) of T cells and cytotoxic T lymphocyte induced cell death. It has also been implicated in the progression of several cancers. Defects in this gene may be related to some cases of systemic lupus erythematosus (SLE). Alternatively spliced transcript variants have been described.

As used herein, the term "TRAIL" refers to the tumour necrosis factor (TNF)- related apoptosis-inducing ligand, also known as Apo-2 ligand (Apo2L), which is a member of the cytokine superfamily. By cross-linking TRAIL- Receptor 1 (TRAIL-Ri) or TRAIL-Receptor 2 (TRAIL-R2), also known as death receptors 4 and 5 (DR4 and DR5, respectively, or also known as Tumour necrosis factor receptor superfamily member 10A (TNFRSF10A) or Tumour necrosis factor receptor superfamily member 10B (TNFRSF10B)), TRAIL has the capability to induce apoptosis in a wide variety of tumour cells while sparing vital normal cells. The discovery of this unique property led to the development of TRAIL-based anticancer agents. The terms "TRAIL-Ri", "TNFRSF10A", "DR4" and "DR4 receptor" are used herein interchangeably. They refer to the full length TRAIL receptor sequence and soluble, extracellular domain forms of the receptor described in Pan et al, Science, 1997, 276: 111-113; WO98/32856; U.S. Pat. No. 6,342,363; and WO99/37684. The terms "TRAIL-R2", "TNFRSF10B", "DR5" and "DR5 receptor" are used herein interchangeably. They refer to the full length TRAIL receptor sequence and soluble, extracellular domain forms of the receptor described in Sheridan et al, Science, 1997, 277: 818-821; Pan et al, Science, 1997, 277: 815-818; U.S. Pat. No. 6,072,047; U.S. Pat. No. 6,342,369; WO98/51793; WO98/41629; Screaton et al, Curr. Biol., 1997, 7: 693-696; Walczak et al, EMBO J., 1997, 16: 5386-5387; Wu et al, Nature Genetics, 1997, 17: 141-143; WO98/35986; WO98/46643; WO99/02653; WO99/09165; and WO99/11791.

The term "calmodulin" or "CaM" is well known to the person skilled in the art and as used herein is intended to mean a polypeptide comprising at least one EF-hand motif that is capable of binding calcium. Calmodulin plays a major role in transmitting intracellular Ca2+ signals to a wide variety of effector systems (Rhyner et al., (1994) Euro J Biochem 225: 71-81). In mammals, 3 genes (CALM1, CALM2 and CALM3) encode the single, highly conserved, calmodulin (CaM) protein (Berchtold et al., (1993) Genomics 16: 461-465). In particular embodiments of the invention the term calmodulin is a polypeptide of SEQ ID NO: 11 or encodes an amino acid sequence having 70%, 75%, 80%, 85%, 90%, 95%, 96%, 97%, 98% or 99% sequence identity to SEQ ID NO:11.

The terms "CAMK kinase" or "CAMKK" or "CAMKK1" are use synonymously and refer to a kinase polypeptide with an activity to phosphorylate CAMK1D. Human CAMKK1 belongs to the Serine/Threonine protein kinase family, and to the Ca(2+)/calmodulin-dependent protein kinase subfamily. This protein plays a role in the calcium/calmodulin-dependent (CaM) kinase cascade. Three transcript variants encoding two distinct isoforms have been identified for the CAMKK1 gene. A CAMKK In particular embodiments of the invention the term CAMKK is a polypeptide of SEQ ID NO: 12 or 13 or encodes an amino acid sequence having 70%, 75%, 80%, 85%, 90%, 95%, 96%, 97%, 98% or 99% sequence identity to SEQ ID NO:12 or 13.

As used herein, a "subject" includes all mammals, including without limitation humans, but also non-human primates such as cynomolgus monkeys. It also includes dogs, cats, horses, sheep, goats, cows, rabbits, pigs and rodents (such as mice and rats). It will be appreciated that a particularly preferred subject according to the invention is a human subject, such as a human suffering from (or at risk of suffering from) a disorder, disease or condition, for example a human patient.

As used herein, "therapy" is synonymous with treating a disease, disorder or condition, which includes reducing symptoms of the disease, disorder or condition, inhibiting progression of the disease, disorder or condition, causing regression of the disease, disorder or condition and/or curing the disease, disorder or condition.

The term "treatment" in the present invention is meant to include therapy, e.g. therapeutic treatment, as well as prophylactic or suppressive measures for a disease (or disorder or condition). Thus, for example, successful administration of a CAMK1D inhibitor prior to onset of the disease results in treatment of the disease. "Treatment" also encompasses administration of a CAMK1D inhibitor after the appearance of the disease in order to ameliorate or eradicate the disease (or symptoms thereof). Administration of a CAMK1D inhibitor after onset and after clinical symptoms, with possible abatement of clinical symptoms and perhaps amelioration of the disease, also comprises treatment of the disease. Those "in need of treatment" include subjects (such as a human subject) already having the disease, disorder or condition, as well as those prone to or suspected of having the disease, disorder or condition, including those in which the disease, disorder or condition is to be prevented. Preferably, in accordance with the herein disclosed and preferred treatments, the administration of the CAMK1D inhibitor is associated with a reduced inhibition of, in particular by a reduced phosphorylation of, effector caspases in the cells involved with the proliferative disorder, such as caspase-3, caspase-6 and/or caspase-7.

The compounds of the present invention, or pharmaceutically acceptable salts thereof, may also be administered simultaneously with, prior to, or after administration of one or more of the therapeutic agents described herein. Thus the invention includes combinatorial treatments where two or more CAMK1D inhibitors that are different are administered in combination to the subject in order to increase treatment efficiency. Such combination therapy may include administration of a single pharmaceutical dosage formulation which contains a compound of the present invention and one or more additional agents given below, as well as administration of the compound of the present invention and each of additional agent in its own separate pharmaceutical dosage formulation. For example, a compound of the present invention and a chemotherapeutic agent, where the agent is an inhibitor of a direct activator of CAMK1D such as an inhibitor of CaM or CAMKK. Furthermore, in some particular embodiments, combinatorial treatments of the invention may include the use of other anti-cancer agents such as taxol (paclitaxel), taxotere, etoposide, cisplatin, vincristine, vinblastine, and the like, can be administered to the patient either together in a single oral dosage composition such as a tablet or capsule, or each agent administered in separate oral dosage formulations or via intravenous injection. Where separate dosage formulations are used, the compounds of the present invention and one or more additional agents can be administered at essentially the same time, i.e., concurrently, or at separately staggered times, i.e., sequentially; combination therapy is understood to include all these regimens. In addition, these compounds may stimulate the death receptor apoptotic pathway and thereby synergize with the CAMK1D inhibitor of the invention through a direct or indirect manner, as for example, the compounds of the present invention may be used in combination with soluble FasL or TRAIL any agent or procedures that can cause an increase in circulating level of TRAIL, such as interferon-alpha or radiation.

In **a second aspect,** the invention pertains a CAMK1D inhibitor for use in a treatment of a proliferative disorder in a subject, the treatment comprising exposing cells involved with the proliferative disorder in the subject to: (i) cell-dependent or cell-independent cytotoxic stimulus; and (ii) the CAMK1D inhibitor. In such aspect, in preferred embodiments, the cell-dependent or cell-independent cytotoxic stimulus is capable of inducing or increasing apoptosis in the cells involved with the proliferative disorder, in particular death receptor mediated apoptosis.

In this aspect, preferably, the cell-dependent or cell-independent cytotoxic stimulus is an agent that when exposed to the cells involved with the proliferative disorder induces or increases apoptosis in the cells involved with the proliferative disorder by activating, or by increasing the activation of, death receptor signalling, such as signalling by a receptor of the Tumour necrosis factor receptor superfamily.

Preferably, the dependent or cell-independent cytotoxic stimulus is an agonist of death receptor signalling, preferably such agonists of death receptor signalling as described herein elsewhere. Such agonists include in preferred embodiments a FasL or TRAIL, or any functional variant of such proteins.

In certain preferred embodiments of this aspect, in (i) the cells involved with the proliferative disorder are exposed to the cell-dependent or cell-independent cytotoxic stimulus by
- a cell-mediated immune response, such as CTL response, wherein the immune cells express and/or secrete a cell-dependent or cell-independent cytotoxic stimulus (for example a TNR6 ligand or TRAIL), in particular wherein the cells involved with the proliferative disorder are exposed to the immune cells such as CTL;
- an administration of immune cells, such as CTL, which express and/or secrete a cell-dependent or cell-independent cytotoxic stimulus (for example a TNR6 ligand or TRAIL); and/or
- an administration of a substance or composition eliciting the cell-dependent or cell-independent cytotoxic stimulus to the subject.

In specific embodiments of the second aspect, the invention also pertains to the exposure of cells involved with the proliferative disorder with (i) cell-dependent or cell-independent cytotoxic stimulus in context of a T cell, or CAR T cell, therapy. A "T-cell therapy" or "adoptive T-cell therapy" refers to the ex vivo expansion and selection of T-lymphocytes that will be subsequently administered to a subject for therapeutic purposes. This therapy has been widely used in allogeneic hematopoietic stem cells transplantation (HSCT), wherein the infusion of the donor's lymphocytes gives rise to immune rejection. The term "T-cell therapy", as used in the present invention, also includes therapy with T-lymphocytes that have been genetically manipulated to express chimeric antigen receptors (known as "CAR T cell therapy'). In this context it is preferred that the cell transplant is enriched for a cell-dependent or cell-independent cytotoxic stimulus, in particular of such embodiments, where the cell transplant is enriched for death receptor signalling agonists as disclosed herein elsewhere. Preferably the death receptor signalling agonists are FasL, TRAIL, or any functional variant thereof, or is in particular a Chimeric Antigen Receptor introduced into and expressed in the cell transplant.

The term "Chimeric Antigen Receptor" or "CAR" has its general meaning in the art and refers to an artificially constructed hybrid protein or polypeptide containing the antigen binding domains of an antibody (e.g., scFv) linked to T-cell signalling domains. Characteristics of CARs include their ability to redirect T-cell specificity and reactivity toward a selected target in a non-MHC-restricted manner, exploiting the antigen-binding properties of monoclonal antibodies. Hence, in particular preferred embodiments CARs of the invention include antigen binding portions of antibodies which have an agonistic activity towards death receptor signalling, such as agonistic anti-Fas or anti-DR4/DR5 antibodies. The non-MHC-restricted antigen recognition gives T cells expressing CARs the ability to recognize antigen independently of antigen processing, thus bypassing a major mechanism of tumour escape. Moreover, when expressed in T-cells, CARs advantageously do not dimerize with endogenous T cell receptor (TCR) alpha and beta chains. Strategies to design and produce such CARs are well known in the art, references can be found for example in Bonini and Mondino, Eur. J. Immunol. 2015 (19), Srivastava and Riddell, Trends Immunol. 2015 (20), Jensen and Riddell, Curr. Opin. Immunol. 2015 (21), Gill and June, Immunol. Rev. 2015 (22). In preferred embodiments of such second aspect, therefore, the invention comprises the administration of immune cells, such as CTL, which express and/or secrete a cell-dependent or cell-independent cytotoxic stimulus which is a CAR, for example a CAR comprising antigen binding domains of one or more agonistic anti-Fas or anti-DR4/DR5 antibodies.

In **a third aspect,** the invention pertains to a CAMK1D inhibitor for use in a treatment of a proliferative disorder in a subject, wherein the treatment is for sensitizing cell involved with the proliferative disorder to a cell-dependent or cell-independent cytotoxic stimulus, the treatment comprising administering the CAMK1D inhibitor to the subject.

In **a fourth aspect,** the invention pertains to a CAMK1D inhibitor for use in a treatment for increasing the therapeutic index of treatment with a cell-dependent or cell-independent cytotoxic stimulus in a subject being treated therewith for a proliferative disorder, the method comprising administering the inhibitor of CAMK1D to the subject.

In **a fifth aspect,** the invention pertains to a CAMK1D inhibitor for use in a treatment for the sensitisation of a subject suffering from a proliferative disorder to a therapy involving the administration of a cell-dependent or cell-independent cytotoxic stimulus to the subject, the method comprising administering the CAMK1D inhibitor to the subject

In **a sixth aspect,** the invention pertains to a CAMK1D inhibitor for use in the treatment of a proliferative disorder in a subject in need of such treatment, the treatment comprising a step of administering to the subject the CAMK1D inhibitor recited in any one of the preceding claims, in an effective amount to sensitise cells involved with the proliferative disorder in the subject to a death receptor stimulating agent.

### Nucleic acid CAMK1D inhibitors:

In one particular set of embodiments, the CAMK1D inhibitor is a nucleic acid.

The terms "nucleic acid", "polynucleotide" and "oligonucleotide" are used interchangeably throughout and include DNA molecules (e.g., cDNA or genomic DNA), RNA molecules (e.g., mRNA), analogues of the DNA or RNA generated using nucleotide analogues (e.g., peptide nucleic acids and non-naturally occurring nucleotide analogues), and hybrids thereof. The nucleic acid molecule can be single-stranded or double-stranded.

In the case of CAMK1D inhibitors being CRISPR/Cas9 constructs and/or guide RNA/DNAs (gRNA/gDNA) and/or tracrRNAs, the basic rules for the design of CRISPR/Cas9 mediated gene editing approaches are known to the skilled artisan and for example reviewed in Wiles MV et al (Mamm Genome 2015, 26:501) or in Savić N and Schwank G (Transl Res 2016, 168:15).

In particular embodiments, the CAMK1D inhibitor may be an inhibitory nucleic acid molecule, such as antisense nucleotide molecule including a siRNA or shRNA molecule, for example as described in detail herein below.

In more particular of such embodiments, the inhibitory nucleic acid (such as siRNA or shRNA) can bind to, such as specifically bind to, a nucleic acid (such as mRNA) that encodes or regulates the expression, amount, function, activity or stability of: (i) CAMK1D; or (ii) a gene (such as CaM or CAMKK) that controls the expression, amount, function and/or stability of CAMK1D and, for example, thereby modulates the expression, amount function, activity and/or stability of CAMK1D.

An inhibitor of CAMK1D that is a nucleic acid can be, for example, an anti-sense nucleotide molecule, an RNA, DNA or PNA molecule, or an aptamer molecule. An anti-sense nucleotide molecule can, by virtue of it comprising an anti-sense nucleotide sequence, bind to a target nucleic acid molecule (eg based on sequence complementarity) within a cell and modulate the level of expression (transcription and/or translation) of CAMK1D, or it may modulate expression of another gene that controls the expression, function and/or stability of CAMK1D. Similarly, an RNA molecule, such as a catalytic ribozyme, can bind to and alter the expression of the CAMK1D gene, or it can bind to and alter the expression of other genes that control the expression, function and/or stability of CAMK1D, such as a kinase molecule, interacting protein, a transcription factor for or repressor protein of CAMK1D. An aptamer is a nucleic acid molecule that has a sequence that confers it an ability to form a three-dimensional structure capable of binding to a molecular target.

An inhibitor of CAMK1D that is a nucleic acid can be, for example, can further be a double-stranded RNA molecule for use in RNA interference. RNA interference (RNAi) is a process of sequence-specific gene silencing by post-transcriptional RNA degradation or silencing (prevention of translation). RNAi is initiated by use of double-stranded RNA (dsRNA) that is homologous in sequence to the target gene to be silenced. A suitable double-stranded RNA (dsRNA) for RNAi contains sense and antisense strands of about 21 contiguous nucleotides corresponding to the gene to be targeted that form 19 RNA base pairs, leaving overhangs of two nucleotides at each 3'end (Elbashir et al., Nature 411:494-498 (2001); Bass, Nature 411:428-429 (2001); Zamore, Nat. Struct. Biol. 8:746-750 (2001)). dsRNAs of about 25-30 nucleotides have also been used successfully for RNAi (Karabinos et al., Proc. Natl. Acad. Sci. USA 98:7863-7868 (2001). dsRNA can be synthesised in vitro and introduced into a cell by methods known in the art.

A particularly preferred example of an antisense molecule of the invention is a small interfering RNA (siRNA) or endoribonuclease- prepared siRNA (esiRNA). An esiRNA is a mixture of siRNA oligos resulting from cleavage of a long double-stranded RNA (dsRNA) with an endoribonuclease such as Escherichia coli RNase III or dicer. esiRNAs are an alternative concept to the usage of chemically synthesised siRNA for RNA Interference (RNAi). An esiRNAs is the enzymatic digestion of a long double stranded RNA in vitro.

As described above, a modulator of the invention that is an RNAi molecule (such as an siRNA) may bind to and directly inhibit or antagonise the expression of mRNA of CAMK1D. However, a modulator of the invention that is an RNAi molecule (such as an siRNA) may bind to and inhibit or antagonise the expression of mRNA of another gene that itself controls the expression (or function or stability) of CAMK1D. Such other genes may include activator or other binding proteins such as CaM or CAMKK.

The sequence identity of the antisense molecule according to the invention in order to target a CAMK1D mRNA (or to target mRNA of a gene controlling expression, function and/or stability of CAMK1D), is with increasing preference at least 75%, at least 80%, at least 85%, at least 90%, at least 95%, at least 98%, at least 99% and 100% identity to a region of a sequence encoding the CAMK1D protein, as disclosed herein (or of such other controlling gene, of which a preferred example is the CaM or CAMKK gene). Preferably, the region of sequence identity between the target gene and the modulating antisense molecule is the region of the target gene corresponding to the location and length of the modulating antisense molecule. For example, such a sequence identity over a region of about 19 to 21bp of length corresponding to the modulating siRNA or shRNA molecule). Means and methods for determining sequence identity are known in the art. Preferably, the BLAST (Basic Local Alignment Search Tool) program is used for determining the sequence identity with regard to one or more CAMK1D RNAs as known in the art. On the other hand, preferred antisense molecules such as siRNAs and shRNAs of the present invention are preferably chemically synthesised using appropriately protected ribonucleoside phosphoramidites and a conventional RNA synthesiser. Suppliers of RNA synthesis reagents include Proligo (Hamburg, Germany), Dharmacon Research (Lafayette, CO, USA), Pierce Chemical (part of Perbio Science, Rockford, IL (USA), Glen Research (Sterling, VA, USA), ChemGenes (Ashland, MA, USA), and Cruachem (Glasgow, UK).

The ability of antisense molecules, siRNA, and shRNA to potently, but reversibly, silence genes *in vivo* make these molecules particularly well suited for use in the pharmaceutical composition of the invention which will be also described herein below. Ways of administering siRNA to humans are described in De Fougerolles et al., Current Opinion in Pharmacology, 2008, 8:280-285. Such ways are also suitable for administering other small RNA molecules like shRNA. Accordingly, such pharmaceutical compositions may be administered directly formulated as a saline, via liposome based and polymer-based nanoparticle approaches, as conjugated or complexation pharmaceutical compositions, or via viral delivery systems. Direct administration comprises injection into tissue, intranasal and intratracheal administration. Liposome based and polymer- based nanoparticle approaches comprise the cationic lipid Genzyme Lipid (GL) 67, cationic liposomes, chitosan nanoparticles and cationic cell penetrating peptides (CPPs). Conjugated or complexation pharmaceutical compositions comprise PEI-complexed antisense molecules, siRNA, shRNA or miRNA. Further, viral delivery systems comprise influenza virus envelopes and virosomes.

The antisense molecules, siRNAs, shRNAs may comprise modified nucleotides such as locked nucleic acids (LNAs). The ribose moiety of an LNA nucleotide is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired. Such oligomers are synthesised chemically and are commercially available. The locked ribose conformation enhances base stacking and backbone pre-organisation. This significantly increases the hybridisation properties (melting temperature) of oligonucleotides. Particularly preferred example of siRNAs is GapmeR (LNA™ GapmeRs (Exiqon)). GapmeRs are potent antisense oligonucleotides used for highly efficient inhibition of CAMK1D mRNA (or of mRNA of a gene controlling expression, function and/or stability of CAMK1D). GapmeRs contain a central stretch of DNA monomers flanked by blocks of LNAs. The GapmeRs are preferably 14-16 nucleotides in length and are optionally fully phosphorothioated. The DNA gap activates the RNAse H-mediated degradation of targeted RNAs and is also suitable to target transcripts directly in the nucleus.

Preferred antisense molecules for targeting CAMK1D are antisense molecules or constructs having a sequence complementary to a region (such as one described above) of a nucleic acid sequence of an CAMK1D mRNA, preferably a sequence complementary to a region of a sequence encoding the amino acid sequence shown in SEQ ID NO: 1 or 2, in particular of SEQ ID NO: 1, more preferably, a sequence complementary to a region of between about 15 to 25 bp (such as between about 19 and 21 bp) of a sequence encoding the amino acid sequence shown in SEQ ID NO: 1 or 2, in particular of SEQ ID NO: 1.

In particular embodiments, an antisense molecule for targeting CAMK1D may be an siRNA selected from the CAMK1D siRNA molecules identified as "s1", "s2", "s3, or "s4" herein (eg in Table A1; SEQ ID NOs: 7, 8, 9 and 10, respectively).

**Table A1: exemplary siRNA sequences used ("h" indicates human and "m" indicates mouse homolog of the target gene)**

| Gene | siRNA ID | siRNA sequence | Order number (Dharmacon/GE Lifesciences) | SEQ ID NO. |
|---|---|---|---|---|
| hCAMKiD | s1 | UGAAGUGUAUCCCUAAGAA | D-004946-01 | 3 |
| hCAMKiD | s2 | CAAAUCACCUGUACUUGGU | D-004946-02 | 4 |
| hCAMKiD | s3 | CCGAAAAUCUCUUGUACUA | D-004946-03 | 5 |
| hCAMKiD | s4 | GAGAAGGACCCGAAUAAAA | D-004946-04 | 6 |
| hCAMKiD | Pool | As above: s1, s2, s3 and s4 | As above | N/A |
| mCAMKiD | Pool | N/A | MQ-063690-01-0002 | N/A |

In one embodiment the antisense molecules of the invention may be isolated. In another embodiment, the antisense molecules of the invention may be recombinant, synthetic and/or modified, or in any other way non-natural or not a product of nature. For example, a nucleic acid of the invention may contain at least one nucleic acid substitution (or deletion) modification such as between 1 and about 5 such modifications, preferably no more than 1, 2 or 3 such modifications) relative to a product of nature, such as a human nucleic acid. As described above, the antisense molecules of the invention may be modified by use of non-natural nucleotides, or may be conjugated to another chemical moiety. For example, such chemical moieties may be a heterologous nucleic acid conferring increased stability or cell/nucleus penetration or targeting, or may be a non-nucleic acid chemical moiety conferring such properties, of may be a label.

Certain preferred embodiments pertain to a genetic construct for gene editing that is used as an inhibitor of expression, function and/or stability of CAMK1D in the context of the herein described invention. By using genome editing constructs it is possible to modulate the expression, stability and/or activity of CAMK1D. Genome editing approaches are well known in the art and may be easily applied when the respective target genomic sequences are known. Preferably, such approaches may be used in gene therapy using e.g. viral vectors, which specifically target tumour cells in accordance with the above descriptions.

In case of genome editing, DNA is inserted, replaced, or removed, from a genome using artificially engineered nucleases, or so called "molecular scissors". The nucleases create specific double-stranded break (DSBs) at desired locations in the genome, and harness the cell's endogenous mechanisms to repair the induced break by natural processes of homologous re-combination (HR) and non-homologous end-joining (NHEJ). For doing so, engineered nucleases such as zinc finger nucleases (ZFNs), Transcription Activator-Like Effector Nucleases (TALENs), the CRISPR/Cas system, and engineered meganuclease re-engineered homing endonucleases are routinely used for genome editing. According to another preferred embodiment, for genome editing approaches for modulating/inhibiting CAMK1D, the rare-cutting endonuclease is Cas9, Cpfl, TALEN, ZFN, or a homing endonuclease may be used. Also, it may be convenient to engineer using DNA-guided Argonaute interference systems (DAIS). Basically, said Argonaute (Ago) protein is heterologously expressed from a polynucleotide introduced into said cell in the presence of at least one exogenous oligonucleotide (DNA guide) providing specificity of cleavage to said Ago protein to a preselected locus. The TALEN and Cas9 systems are respectively described in WO 2013/176915 and WO 2014/191128. The Zinc-finger nucleases (ZFNs) are initially described in Kim, YG; Cha, J.; Chandrasegaran, S. ("Hybrid restriction enzymes: zinc finger fusions to Fok I cleavage domain" (1996). Proc Natl Acad Sci USA 93 (3): 1156-60). Cpfl is class 2 CRISPR Cas System described by Zhang et al. (Cpfl is a single RNA-guided Endonuclease of a Class 2 CRIPR-Cas System (2015) Cell; 163:759-771). The argonaute (AGO) gene family was initially described in Guo S, Kemphues KJ. ("par-1, a gene required for establishing polarity in C. elegans embryos, encodes a putative Ser/Thr kinase that is asymmetrically distributed" (1995) Cell;81(4):611-20).

The use of the CRISPR/Cas9, CRISPR/Cpfl or the Argonaute genome-editing systems is particularly adapted to be used in combination with the transfection of guide RNA or guide DNA sequences. In this context the guide-RNAs and a nucleic acid sequence coding for Cas9 nickase (or similar enzymes), is transfected into a target cell (preferably a tumour cell) so that they form a complex able to induce a nick event in double-stranded nucleic acid targets in order to cleave the genetic sequence between said nucleic acid targets.

In certain embodiments, it may be useful to deliver the guide RNA-nanoparticle formulations separately from the Cas9. In such an instance a dual-delivery system is provided such that the Cas9 may be delivered via a vector and the guide RNA is provided in a nanoparticle formulation, where vectors are considered in the broadest sense simply as any means of delivery, rather than specifically viral vectors. Separate delivery of the guide RNA-nanoparticle formulation and the Cas9 may be sequential, for example, first Cas9 vector is delivered via a vector system followed by delivery of sgRNA-nanoparticle formulation) or the sgRNA-nanoparticle formulation and Cas9 may be delivered substantially contemporaneously (i.e., co-delivery). Sequential delivery may be done at separate points in time, separated by days, weeks or even months. In certain embodiments, multiple guide RNAs formulated in one or more delivery vehicles (e.g., where some guide RNAs are provided in a vector and others are formulated in nanoparticles) may be provided with a Cas9 delivery system. In certain embodiments, the Cas9 is also delivered in a nanoparticle formulation. In such an instance the guide RNA-nanoparticle formulation and the Cas9 nanoparticle formulation may be delivered separately or may be delivered substantially contemporaneously (i.e., co- delivery). As will now be apparent to the person of ordinary skill, the gene target of such genome-editing approaches may be the gene of CAMK1D. Alternatively, the gene target of such editing may be another gene that controls the expression, function and/or stability of CAMK1D, for example CaM or CAMKK.

In preferred embodiments of the invention, the compounds for genome editing approaches according to the invention comprise at least the use of a guide RNA or DNA complementary to a region (such as one described above) of a CAMK1D sequence. In some additional embodiments, the compounds for use in genome editing approaches of the invention may include donor sequences homologous to such a region of CAMK1D, as templates for homology directed repair. The donor sequences comprise a mutated sequence of CAMK1D that when used in the CRISPR induced repair mechanism in a target cell, is by homologous recombination inserted/copied into the CAMK1D genomic locus, and therefore yields into a mutated CAMK1D gene which is characterised by a reduced expression, function and/or stability of the expressed CAMK1D. CRISPR/Cas9 genome editing in cancer therapy is reviewed for example in Khan FA et al: "CRISPR/Cas9 therapeutics: a cure for cancer and other genetic diseases." (Oncotarget. 2016 May 26. doi: 10.18632/oncotarget.9646; incorporated by reference in its entirety).

In particular embodiments, an antisense molecule for targeting CAMK1D may be guide RNA or guide DNA for use in CRISPR/Cas9 gene editing, preferably in humans, for exemplary mouse gRNA sequences are shown (eg in Table A2; SEQ ID NO:).

**Table A2: exemplary gRNA sequences used (for knock-out of mouse CAMK1D)**

| Gene | siRNA ID | siRNA sequence | Order number (Thermofisher Scientific) | SEQ ID NO. |
|---|---|---|---|---|
| mCAMKiD | gRNA1 | TCGATCGGATAGTGGAGAAG | CRISPR383273_CR | 7 |
| mCAMKiD | gRNA2 | GGAGATAGTATACGGCATCC | CRISPR411441_CR | 8 |
| mCAMK1D | gRNA3 | TAGCCGAGGAGAAAGCTACT | CRISPR383284_CR | 9 |

### Hetero-bi-functional compounds

In particular embodiments, inhibitors of CAMK1D may be a hetero-bi-functional compound that contains two ligands connected by a linker, wherein one ligand binds to the target protein (in this case, CAMK1D or a gene that controls the expression, amount, function, activity and/or stability of CAMK1D) and the other ligand binds to and/or recruits a component of the cellular protein degradation machinery such as binding to a ubiquitin ligase protein (eg E3 ubiquitin ligase) or such as recruiting a chaperone protein. Examples of such hetero-bi-functional compounds include PROTACs ("PROteolysis TArgeting Chimera) or HyT ("hydrophobic tagging") molecules, in each case designed to bind to the target protein for the present invention. The general principles of PROTACs and HyT molecules are reviewed in Huang & Dixit 2016 (Cell Research 26:484) and exemplified specifically in, for example, WO 2016/146985A1.

A PROTAC that binds to the target protein (eg CAMK1D) with one ligand and with the other ligand to an E3 ubiquitin ligase protein thereby brings the ligase and the target into close proximity. Without being bound by any particular theory it is generally understood that it is this close proximity which in turn triggers the poly-ubiquitination and subsequent proteasome-dependent degradation of the target protein of interest. Supporting evidence for a PROTAC approach on a general level is provided by known proof-of-concept examples where alternative PROTACs have been used to degrade: the Estrogen-receptor (Cyrus et al 2010, Chem Bio Chem 11:1531); the Androgen-receptor (Sakamoto et al 2003, Mol Cell Proteomics 2:1350); methionine aminopeptidease-2 (Sakamoto et al 2001, PNAS 98:8554); as well as the Aryl Hydrocarbon Receptor (Lee et al 2007, Chem Bio Chem 8:2058).

The concept of hydrophobic tagging is similar to that of PROTAC, but instead of using a ligand to recruit a specific E3 ligase, a synthetic hydrophobic group, such as adamantane, linked to a chemical moiety that specifically recognizes the target protein (eg CAMK1D), assumes the role of "recruiter" for the degradation machinery. Upon binding to the target protein, the hydrophobic tag mimics or induces a misfolded state. Without being bound by any particular theory it is generally understood that modification of the target protein with a bulky hydrophobic side-group attracts the chaperone machinery, the primary goal of which is to help refold misfolded proteins. Since the covalent modification cannot be easily removed, the target protein remains unfolded and is eventually cleared by ubiquitin-proteasome mediated degradation.

In certain embodiments of such a hetero-bi-functional compound in the context of the present invention, the ligand contained therein that binds to the target protein (eg CAMK1D) may be a peptide that binds (preferably, specifically) to such target protein; and in alterative embodiments the ligand that binds to the target protein (eg CAMK1D) may be a small molecule such as small molecule CAMK1D inhibitor that binds (preferably, specifically) to such target protein. Exemplary small molecule CAMK1D inhibitors are described elsewhere herein, and as well as such small molecule CAMK1D inhibitors having utility as inhibitors per-se (that is, not contained in a hetero-bi-functional compound, may - in certain embodiments - be comprised in a hetero-bi-functional compound.

### Small molecule CAMK1D inhibitors:

In another particular set of embodiments, the CAMK1D inhibitor may be a small molecule (in particular, a small molecule ligand or a small cell-permeable molecule).

In more particular of such embodiments, a small molecule CAMK1D inhibitor can bind to: (i) CAMK1D and inhibit the function or activity of CAMK1D (eg phosphorylated CAMK1D); or (ii) a gene (such as CaM or CAMKK) that controls the expression, amount function, activity and/or stability of CAMK1D and, example, thereby modulates the expression, amount, function, activity and/or stability of CAMK1D (eg phosphorylated CAMK1D).

An inhibitor of CAMK1D that is a small molecule can be, for example, any chemical structure or compound (molecule) having a CAMK1D inhibitory activity as described herein and a molecular weight of, for example, less than 3000 dalton (Da), preferably less than 1500 Da, most preferably less than 1000 Da

Typically, a small molecule is a compound having a molecular mass of less than about 750 Da, such as less than about 650 or 600 Da, (and in certain embodiments, a small molecule may be less than about 550 or 500 Da). Furthermore, (in particularly for a cell-permeable compound, and especially for an orally active compound), the small molecule can have, in certain embodiments: (i) no more than 5 hydrogen bond donors (the total number of nitrogen-hydrogen and oxygen-hydrogen bonds); (ii) no more than 10 hydrogen bond acceptors (all nitrogen or oxygen atoms); and/or (iii) an octanol-water partition coefficient log P not greater than 5. Accordingly, in some embodiments the CAMK1D inhibitor can be a cell-permeable small organic molecule, such as one that binds to and inhibits the function or activity of CAMK1D (in particular, of phosphorylated CAMK1D).

In one particular embodiment of all aspects of the invention, the CAMK1D inhibitor is QPP, the compound having a structure of the following formula (I): 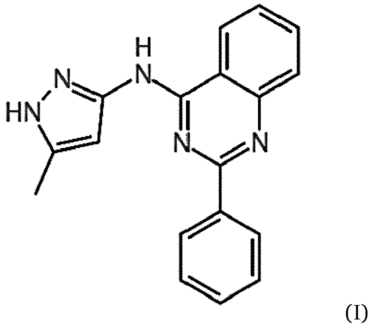 (also known as (5-Methyl-1H-pyrazol-3-yl)-(2-phenylquinazolin-4-yl)amine), and any salt (or a solvate), complex, polymorph, crystalline form, racemic mixture, diastereomers, enantiomer, tautomer, isotopically labelled form, and/or prodrug thereof.

An inhibitor of CAMK1D that is a small molecule has in preferred embodiments a similar inhibitory potential as QPP, and preferably has an IC50 of less than 5µM, more preferably less than 3 µM, less than 1 µM.

### Pharmaceutical compositions:

To be used in therapy, the CAMK1D inhibitor may be formulated into a pharmaceutical composition appropriate to facilitate administration to animals or humans. The term "composition" means a mixture of substances. The term "pharmaceutical composition" means a mixture of substances including a therapeutically active substance (such as a CAMK1D inhibitor) for pharmaceutical use.

To be used in therapy, the CAMK1D inhibitor may be formulated into a pharmaceutical composition appropriate to facilitate administration to animals or humans. The term "composition" means a mixture of substances. The term "pharmaceutical composition" means a mixture of substances including a therapeutically active substance (such as a CAMK1D inhibitor) for pharmaceutical use.

Accordingly, in another aspect, herein provided is a pharmaceutical composition comprising a CAMK1D inhibitor (of, or for use with the invention), and a pharmaceutically acceptable excipient, stabiliser or carrier.

In **another particular aspect,** the invention also relates to **a pharmaceutical composition** including: a CAMK1D inhibitor, preferably the compound according to formula (I): 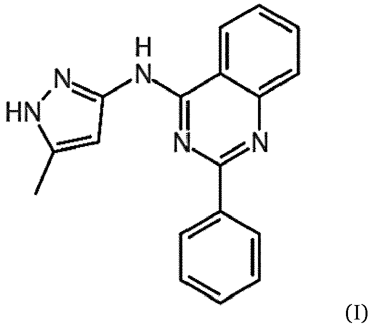 (also known as (5-Methyl-1H-pyrazol-3-yl)-(2-phenylquinazolin-4-yl)amine) or a salt (or a solvate), complex, polymorph, crystalline form, racemic mixture, diastereomers, enantiomer, tautomer, isotopically labelled form, and/or prodrug thereof; and a pharmaceutically acceptable excipient, stabiliser or carrier.

By way of example, the pharmaceutical composition of the invention may comprise between 0.1% and 100% (w/w) active ingredient (for example, a CAMK1D inhibitor), such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, subcutaneous, oral, transdermal (topical) and transmucosal administration.

Solutions or suspensions used for parenteral, intradermal, or subcutaneous application, as well as comprising a compound of (or for use with) the invention (eg CAMK1D inhibitor), can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Kolliphor® EL (formerly Cremophor EL™; BASF, Parsippany, N.J.) or phosphate buffered saline (PBS). In all cases, the injectable composition should, typically, be sterile and be fluid to the extent that easy syringability exists. It should, typically, be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the compound of (or for use with) the invention (e.g., a CAMK1D inhibitor) in the required amount in an appropriate solvent with one or a combination of ingredients described herein, as required, followed by filtered sterilisation. Generally, dispersions are prepared by incorporating the active compound into a sterile vehicle which contains a basic dispersion medium and the required other ingredients from those described herein. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions, as well as comprising a compound of (or for use with) the invention (eg a CAMK1D inhibitor), generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavouring agent such as peppermint, methyl salicylate, or orange flavouring.

Furthermore, the compounds of (or for use with) the invention (eg a CAMK1D inhibitor) can be administrated rectally. A rectal composition can be any rectally acceptable dosage form including, but not limited to, cream, gel, emulsion, enema, suspension, suppository, and tablet. One preferred dosage form is a suppository having a shape and size designed for introduction into the rectal orifice of the human body. A suppository usually softens, melts, or dissolves at body temperature. Suppository excipients include, but are not limited to, theobroma oil (cocoa butter), glycerinated gelatin, hydrogenated vegetable oils, mixtures of polyethylene glycols of various molecular weights, and fatty acid esters of polyethylene glycol.

For administration by inhalation, the compounds of (or for use with) the invention (eg a CAMK1D inhibitor) are typically delivered in the form of an aerosol spray from pressured container or dispenser which contains a suitable propellant, e.g., a gas such as carbon dioxide, or a nebuliser.

Cells, such as immune cells (eg CAR T cells) for use with the invention can be included in pharmaceutical formulations suitable for administration into the bloodstream or for administration directly into tissues or organs. A suitable format is determined by the skilled person (such as a medical practitioner) for each patient, tissue, and organ, according to standard procedures. Suitable pharmaceutically acceptable carriers and their formulation are known in the art (see, e.g. Remington's Pharmaceutical Sciences 16th edition, Osol, A. Ed., 1980). Such cells, when formed in a pharmaceutical composition, are preferably formulated in solution at a pH from about 6.5 to about 8.5. Excipients to bring the solution to isotonicity can also be added, for example, 4.5% mannitol or 0.9% sodium chloride, pH buffered with art-known buffer solutions, such as sodium phosphate. Other pharmaceutically acceptable agents can also be used to bring the solution to isotonicity, including, but not limited to, dextrose, boric acid, sodium tartrate, propylene glycol, polyols (such as mannitol and sorbitol) or other inorganic or organic solutes. In one embodiment, a media formulation is tailored to preserve the cells while maintaining cell health and identity. For example, a premixture including an aqueous solution of anticoagulant (ACD-A), an equal amount of dextrose (50%), and phosphate buffered saline (PBS), or the like is pre-mixed and aliquoted in a volume to typically match or approximate the cellular matrix or environment from which the cell was extracted from the tissue or organ.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions can be formulated into ointments, salves, gels, or creams as generally known in the art.

In certain embodiments, the pharmaceutical composition is formulated for sustained or controlled release of a compound of (or for use with) the invention (eg a CAMK1D inhibitor). Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially (including liposomes targeted to infected cells with monoclonal antibodies to viral antigens) can also be used as pharmaceutically acceptable carriers. These can be prepared according to methods known to those skilled in the art.

It is especially advantageous to formulate oral, rectal or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

In some embodiments, the pharmaceutical composition comprising a CAMK1D inhibitor is in unit dose form of between 10 and 1000 mg CAMK1D inhibitor. In some embodiments, the pharmaceutical composition comprising a CAMK1D inhibitor is in unit dose form of between 10 and 200 mg CAMK1D inhibitor. In some embodiments, the pharmaceutical composition comprising an ABP is in unit dose form of between 200 and 400 mg CAMK1D inhibitor. In some embodiments, the pharmaceutical composition comprising a CAMK1D inhibitor is in unit dose form of between 400 and 600 mg CAMK1D inhibitor. In some embodiments, the pharmaceutical composition comprising a CAMK1D inhibitor is in unit dose form of between 600 and 800 mg CAMK1D inhibitor. In some embodiments, the pharmaceutical composition comprising a CAMK1D inhibitor is in unit dose form of between 800 and 1000 mg CAMK1D inhibitor.

Exemplary unit dosage forms for pharmaceutical compositions comprising CAMK1D inhibitors are tablets, capsules (eg as powder, granules, microtablets or micropellets), suspensions or as single-use pre-loaded syringes. In certain embodiments, kits are provided for producing a single-dose administration unit. The kit can contain both a first container having a dried active ingredient and a second container having an aqueous formulation. Alternatively, the kit can contain single and multi-chambered pre-loaded syringes.

Toxicity and therapeutic efficacy (eg effectiveness) of such active ingredients can be determined by standard pharmaceutical procedures in cell cultures or experimental animals, eg, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Active agents which exhibit large therapeutic indices are preferred. While compounds that exhibit toxic side effects may be used, care should be taken to design a delivery system that targets such compounds to the site of affected tissue in order to minimise potential damage to uninfected cells and, thereby, reduce side effects.

The data obtained from the cell culture assays and animal studies can be used in formulating a range of dosage of the active ingredients (eg a CAMK1D inhibitor or TNR6 ligand or TRAIL, variant of a TNR6 ligand or TRAIL, or agonist of TNR6 or a TRAIL receptor), such as for use in humans. The dosage of such active ingredients lies preferably within a range of circulating concentrations that include the ED50 with little or no toxicity. The dosage may vary within this range depending upon the dosage form employed and the route of administration utilised. For any active ingredients used in the therapeutic approaches of the invention, the (therapeutically) effective dose can be estimated initially from cell culture assays. A dose may be formulated in animal models to achieve a circulating plasma concentration range that includes the IC50 (ie, the concentration of the active ingredients which achieves a half-maximal inhibition of symptoms) as determined in cell culture. Such information can be used to more accurately determine useful (eg effective) amounts or doses, such as for administration to humans. The pharmaceutical compositions can be included in a container, pack, or dispenser together with instructions for administration.

In the context of the invention, an effective amount of the CAMK1D inhibitor or the pharmaceutical composition can be one that will elicit the biological, physiological, pharmacological, therapeutic or medical response of a cell, tissue, system, body, animal, individual, patient or human that is being sought by the researcher, scientist, pharmacologist, pharmacist, veterinarian, medical doctor, or other clinician, eg, lessening of the effects/symptoms of a disorder, disease or condition, such as a proliferative disorder, for example, a cancer or tumour, or killing or inhibiting growth of a cell involved with a proliferative disorder, such as a tumour cell. The effective amount can be determined by standard procedures, including those described below.

In accordance with all aspects and embodiments of the medical uses and methods of treatment provided herein, the effective amount administered at least once to a subject in need of treatment with a CAMK1D inhibitor is, typically, between about 0.01 mg/kg and about 100 mg/kg per administration, such as between about 1 mg/kg and about 10 mg/kg per administration. In some embodiments, the effective amount administered at least once to said subject of a CAMK1D inhibitor is between about 0.01 mg/kg and about 0.1 mg/kg per administration, between about 0.1 mg/kg and about 1 mg/kg per administration, between about 1 mg/kg and about 5 mg/kg per administration, between about 5 mg/kg and about 10 mg/kg per administration, between about 10 mg/kg and about 50 mg/kg per administration, or between about 50 mg/kg and about 100 mg/kg per administration.

For the prevention or treatment of disease, the appropriate dosage of a CAMK1D inhibitor (or a pharmaceutical composition comprised thereof) will depend on the type of disease to be treated, the severity and course of the disease, whether the CAMK1D inhibitor and/or pharmaceutical composition is administered for preventive or therapeutic purposes, previous therapy, the patient's clinical history, age, size/weight and response to the CAMK1D inhibitor and/or pharmaceutical composition, and the discretion of the attending physician. The CAMK1D inhibitor and/or pharmaceutical composition is suitably administered to the patient at one time or over a series of treatments. If such CAMK1D inhibitor and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely.

The amount of the CAMK1D inhibitor and/or pharmaceutical composition administered will depend on variables such as the type and extent of disease or indication to be treated, the overall health, age, size/weight of the patient, the in vivo potency of the CAMK1D inhibitor and/or pharmaceutical composition, and the route of administration. The initial dosage can be increased beyond the upper level in order to rapidly achieve the desired blood-level or tissue level. Alternatively, the initial dosage can be smaller than the optimum, and the daily dosage may be progressively increased during the course of treatment. Human dosage can be optimised, e.g., in a conventional Phase I dose escalation study designed to run from relatively low initial doses, for example from about 0.01 mg/kg to about 20 mg/kg of active ingredient. Dosing frequency can vary, depending on factors such as route of administration, dosage amount and the disease being treated. Exemplary dosing frequencies are once per day, once per week and once every two weeks. Formulation of a CAMK1D inhibitor of (or for use with) the present is within the ordinary skill in the art. In some embodiments of the invention such CAMK1D inhibitor is lyophilised and reconstituted in buffered saline at the time of administration. The CAMK1D inhibitor and/or pharmaceutical composition of may further result in a reduced relapsing of the disease to be treated or reduce the incidence of drug resistance or increase the time until drug resistance is developing; and in the case of cancer may result in an increase in the period of progression-free survival and/or overall survival.

### Characteristics of proliferative disorders for treatment (or diagnosis) in the context of the invention:

The invention is based on the surprising finding that CAMK1D is associated with resistance against anti-tumour immune responses, an in particular resistance against apoptotic stimuli of for example immune cells against a tumour cell, and the inventors herein describe various aspects related to the treatment of proliferative disorders (eg cancers or tumours), in particular by overcoming the resistance of cells involved with the proliferative disorder to a cell-mediated and preferably cytotoxic immune response.

A disorder treatable by the subject matter of the invention is preferably one characterised by a resistance of one or more cells involved in (eg affected by) the disorder (such as the proliferative disorder) against a defence response of the host organism, such as resistance that is associated with a pathological phenotype. A particular example is a resistance of such cells (eg cells of a tumour or cancer) against one or cytotoxic stimuli, such as preferably a stimuli or signal elicited by one or more immune responses, in particular a cell-mediated immune response, mounted by the subject/patient suffering from the disorder.

The term "resistance" refers to an acquired or natural resistance of a cell involved with (eg of or affected by) a proliferative disease, such as tumour or cancer cell, to a patient's own immune response (such as a cell-dependent or cell-independent cytotoxic stimulus, preferably a stimulus capable of inducing death receptor signalling and ultimately apoptosis, including TNR6 or TRAIL signalling), or to immune responses aided by immune therapy/immunotherapy such as adoptive T-cell transfer or treatment with checkpoint blockers. Therefore, a resistant cell (eg a resistant tumour or cancer cell) is more likely to escape and survive humoural and/or cellular immune defence mechanisms in a subject having the disorder (such as the tumour or cancer). A treatment of a resistant proliferative disease, such as tumour/cancer resistance, in context of the invention shall be effective if, compared to a non-treated control, the cell involved with the proliferative disease (such as a cell of the tumour of cancer) becomes more sensitive or susceptible to an immune response (as a cell-dependent or cell-independent cytotoxic stimulus, preferably a stimulus capable of inducing death receptor signalling and ultimately apoptosis, including TNR6 or TRAIL signalling) - in other words will be more likely to be recognised and/or neutralised (preferably by cytotoxic processes such as apoptosis) by the subject's immune response.

Accordingly, in particular embodiments of the invention, cell(s) involved with the proliferative disorder may be resistant against (to) a cell-dependent or cell-independent cytotoxic stimulus, preferably a stimulus capable of inducing death receptor signalling and ultimately apoptosis, including TNR6 or TRAIL signalling cell-mediated, preferably in context of an immune response; and/or such cell(s) may have or display a resistant phenotype.

In preferred embodiments of the invention, the terms "cellular resistance", "cell resistance" and the like refers to a resistance of the subject cell(s) (such as a tumour or cancer cell) to a cell-mediated immune response, such as a cytotoxic T lymphocyte (CTL) response or exposure to death receptor signalling (eg, the tumour or tumour cell being nonresponsive to, or having reduced or limited response to a CTL targeting a tumour cell via TNR6 or TRAIL, or by other death receptor signalling agonists). A tumour cell may show a reduced or limited response when contacted with a CTL specific for an antigen expressed on that tumour cell or when contacted with another agonist of TNR6 or TRAIL signalling. A reduced or limited response is a reduction to a 90% cytotoxic T cell or death receptor mediated responses, preferably a reduction to 80%, 70%, 60%, 50% or more preferably a reduction to 40%, 30%, 20% or even less. In this case, 100% would denote the state wherein the CTLs or cell-independent cytotoxic stimuli can kill all of the subject cells involved with the proliferative disorder in a sample. Whether or not a subject cell (eg a tumour cell) is resistant to a patient's (cell-dependent or cell-independent) immune response may be tested in-vitro by contacting a sample of the subjects such cells (eg autologous tumour cells) with (eg autologous) T-cells or (eg recombinant) death receptor signalling agonists (such as recombinant TNR6 ligands or TRAIL) and thereafter quantifying the survival/proliferation rate of the (eg) tumour cells. As an alternative, the reduction in (cell-mediated) immune response is determined by comparing cancer samples of the same cancer before and after the resistance is acquired (for example induced by therapy), or by comparing with a cancer sample derived from a different cancer which is known to have no resistance to the CTL or to death receptor signalling. On the other hand, the treatments of the present invention include the sensitisation of cells involved with the proliferative disorder against CTL and/or death receptor signalling and therefor to decrease resistance of such cells. A decrease of (eg tumour) cell resistance against CTL and/or death receptor signalling is preferably a significant increase of CTL or death receptor-mediated toxicity, preferably a 10% increase, more preferably 20%, 30%, 40%, 50%, 60%, 70%, 80% or more, even more preferably 2 fold increase, 3 fold, 4 fold, 5 fold or more.

In particular embodiments, a resistant phenotype of the cells involved with the proliferative disorder is displayed by such cells when a subject suffering from the proliferative disorder (eg a cancer or tumour) has been previously treated with an (immune)therapy and, for example, such proliferative disorders has progressed despite such prior (immune)therapy. Accordingly, in certain embodiments, the subject may be distinguished (characterised) as having been previously treated with an immunotherapy and whose tumour has progressed, in particular whose tumour relapsed, recurred or did not respond. For example, a class of subject suitable for the various therapeutic methods of the invention can be those whose tumour (or cancer) has progressed (such as has relapsed or recurred, or has not responded to) after prior treatment with a cancer immunotherapy. Therefore, in certain embodiments, the prior immunotherapy comprised administration of an immune checkpoint inhibitor, such as a prior immunotherapy comprising (administration of) an antibody binding to an immune checkpoint molecule, a non-antibody peptide or a small molecule (in each case, such as described elsewhere). In certain embodiments, such prior treatment may be any immunotherapy as described elsewhere herein, including adoptive immune cell transfer (eg TCR or CAR T cell therapy), an anti-tumour vaccine, an antibody binding to an immune checkpoint molecule (such as CTLA-4, PD-1 or PD-Li), or a non-antibody peptide or small molecule ligand of an immune (inhibitory) checkpoint molecule (in each case, such as described elsewhere. Other immunotherapy (such as may be a prior treatment of a subject) may include: (x) administration of a drug that activates STING protein, which may lead to stimulation of the innate immune system of the subject. Drugs that agonise STING can include cyclic dinucleotides (CDNs), such as compounds Aduro Biotech's ADU-S100, an analogue of 2'3'-cGAMP (Fu et al, 2015; Sci Transl Med 7; 283:ra52); (y) administration of A2aR antagonists such as SCH58261, SYN115, ZM241365 or FSPTP (as reviewed by Leone et al, 2015; Comp Struct Biotech J 13:265); and/or (z) targeting IDO1/TDO and their downstream effectors, such as administration of indoleamine-2,3-dioxygenase which is in clinical trials with the aim at reverting cancer-induced immunosuppression (as reviewed by Platten et al, 2015; Front Immun 5:673).

In other (or further) embodiments, the subject may suffer from a tumour or cancer, and such cancer may have progressed (such as has relapsed or recurred, or has not responded to) after prior radiotherapy. Accordingly, in other embodiments, the subject may be distinguished (characterised) as having a tumour that progressed, in particular relapsed, recurred or did not respond to, prior radiotherapy.

Accordingly, the invention also includes those embodiments where cells involved with the proliferative disorder have been subjected to prior immunotherapy. As one example of such embodiments, the subject may have received (eg treatment by) prior immunotherapy (eg one described elsewhere herein), such as by administration with (a ligand to) an immune checkpoint molecule (eg administration of an immune checkpoint inhibitor), and/or any other prior immunotherapy such as described elsewhere herein.

A disorder treatable by the subject matter of the invention is, in certain embodiments, one characterised by expression of CAMK1D; in particular, one characterised by such expression that is aberrant, for example over- (or under-) expression or representation or activity of CAMK1D (in particular of phosphorylated CAMK1D) in a given cell or tissue (such as those cells or tissues involved with the proliferative disease of the subject) compared to that in a healthy subject or a normal cell.

Accordingly, the invention includes those embodiments wherein cells involved with the proliferative disorder (eg cells of the tumour) are characterised by expression and/or activity of CAMK1D (in particular, such cells express mRNA and/or protein of CAMK1D, and/or are positive for such CAMK1D expression and/or activity). In particular of such embodiments, the subject is distinguished (eg, can be characterised) - such as being suitable for the treatment methods of the present invention, by having cells involved with the proliferative disorder characterised by expression and/or activity of CAMK1D, in particular such cells express mRNA and/or protein of CAMK1D, and/or are positive for such CAMK1D expression and/or activity.

Furthermore, the invention includes those embodiments wherein cells involved with the proliferative disorder (eg cells of the tumour) are characterised by expression and/or activity of CAMK1D and a death receptor (in particular, such cells express mRNA and/or protein of CAMK1D and a death receptor, and/or are positive for such CAMK1D and a death receptor expression and/or activity). In particular of such embodiments, the subject is distinguished (eg, can be characterised) - such as being suitable for the treatment methods of the present invention, by having cells involved with the proliferative disorder characterised by expression and/or activity of CAMK1D and a death receptor, in particular such cells express mRNA and/or protein of CAMK1D and a death receptor, and/or are positive for such CAMK1D and a death receptor expression and/or activity. A death receptor is preferably TNR6 or a TRAIL receptor as disclosed herein elsewhere.

A disorder treatable by the subject matter of the invention may, in other certain embodiments, be one characterised by (such as a disorder that is further characterised by) one or more applicable biomarkers.

The term "applicable biomarker" means any one (or more) of the genes (as well as CAMK1D) expressed by the cell involved with the proliferative disorder that the inventors have surprisingly found are involved in the CAMK1D-mediated cellular resistance against an immune response (eg a cell-mediated immune response such as mediated by death receptor signalling, in particular TNR6/TRAIL). Such genes include (as well as CAMK1D, in particular phosphorylated CAMK1D):
- TNR6, such as the presence of (or an amount of) or expression and/or activity of TNR6 in cells of the tumour;
- TNFRSF10A or TNFRSF10B, such as the presence of (or an amount of) or expression and/or activity of TNFRSF10A or TNFRSF10B, in cells of the tumour;
- A binding of a Ca2+/Calmodulin complex to CAMK1D;
- A binding of CAMKK to CAMK1D;
- A phosphorylation of CAMK1D, preferably by CAMKK, such as an increase in phosphorylated CAMK1D in the cell or cell-free system;
- Caspases-3, -6, and/or -7, in particular the presence of phosphorylated and thereby inactivated or inhibited caspases -3, -6, and/or -7; in particular a presence of a phosphorylation of caspase-3 at SER150, and/or a phosphorylation of caspase-6 at SER256;
- A binding of CAMK1D to a caspase, such as caspases-3, -6, and/or -7;

In certain embodiments of all aspects of the invention, the proliferative disorder may be a tumour selected from the group consisting of: head and neck cancer, squamous cell carcinoma, multiple myeloma, solitary plasmacytoma, renal cell cancer, retinoblastoma, germ cell tumours, hepatoblastoma, hepatocellular carcinoma, melanoma, rhabdoid tumour of the kidney, Ewing Sarcoma, chondrosarcoma, any haemotological malignancy (e.g., chronic lymphoblastic leukemia, chronic myelomonocytic leukemia, acute lymphoblastic leukemia, acute lymphocytic leukemia, acute myelogenous leukemia, acute myeloblasts leukemia, chronic myeloblastic leukemia, Hodgekin's disease, non- Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, marginal zone lymphoma, Burkitt Lymphoma, mycosis fungoides, seary syndrome, cutaneous T-cell lymphoma, peripheral T cell lymphoma, chronic myeloproliferative disorders, myelofibrosis, myeloid metaplasia, systemic mastocytosis), and central nervous system tumours (eg, brain cancer, glioblastoma, non- glioblastoma brain cancer, meningioma, pituitary adenoma, vestibular schwannoma, a primitive neuroectodermal tumour, medulloblastoma, astrocytoma, anaplastic astrocytoma, oligodendroglioma, ependymoma and choroid plexus papilloma), myeloproliferative disorders [e.g., polycythemia vera, thrombocythemia, idiopathic myelfibrosis), soft tissue sarcoma, thyroid cancer, endometrial cancer, carcinoid cancer, and liver cancer; in particular one or the forgoing that is a solid tumour; or the cell(s) involved with the proliferative disorder is(are) one of or derived from one of such tumours.

In particular of such embodiments, the proliferative disorder may be a tumour selected from the group consisting of: B cell malignancies, such as multiple myeloma, (uveal) melanoma, ovarian cancer, oesophageal cancer, sarcoma and colorectal cancer; or the cells involved with the proliferative disorder are those of or derived from one of such tumours.

In preferred embodiments of all aspects of the invention the proliferative disorder to be treated is characterized by the expression of Fas and/or TRAIL receptor (DR4/DR5) in cells involved with the proliferative disorder, such as tumour cells.

In yet other embodiments of the therapeutic methods of the invention, administration of, exposure to or cell contact with the CAMK1D inhibitor, when in a subject, may be associated with one or more of:
- an increase in tumour infiltrating lymphocytes (TIL)/marrow infiltrating lymphocytes (MIL) activity in said subject;
- an increase in TIL/MIL survival in said subject;
- an increase in TIL/MIL number in said subject;
- an increase in TNR6 ligand, TRAIL, or TNR6-ligand expressing CTLs, production in said subject;
- an increase in the ratio of anti-tumour and tumour-suppressive immune cells (eg, the ratio of CD8 T cells/Tregs or the ratio of Teff/MDSC);
- an increase in infiltration of TILs/MILs into the tumour of said subject;
- an increase in INF-gamma production in said subject;
- an increase in IL2 production in said subject;
- a decrease in production of TGF-beta in said subject:
- a decrease in production of IL6 in said subject and/or
- a decrease in production of IDO (indoleamine-pyrrole 2,3-dioxygenase) in said subject.

In yet further embodiments of the therapeutic methods of the invention, administration of, exposure to or cell contact with the CAMK1D inhibitor, when in a subject, may enhance a cell-mediated immune response in the subject; in particular wherein said enhancement is associated with one or more of the features as set forth above.

### Detection/diagnostic/monitoring methods and kits:

CAMK1D can be used for diagnostic purposes to detect, diagnose, or monitor diseases and/or conditions associated with a resistance against cell dependent or cell independent cytotoxic stimulus; and in particular aberrant and/or localised expression/activity of CAMK1D (in particular phosphorylated CAMK1D) can be so used. In preferred embodiments of the detection and diagnosis methods of the invention, the diseases, disorders or conditions is a cancer or tumour (such as a liquid or solid tumour), including one or more of those described elsewhere herein; more preferably one or more of the disorders described elsewhere herein, such as multiple myeloma, uveal melanoma, ovarian cancer, oesophageal cancer, glioblastoma, sarcoma and colorectal cancer.

Accordingly, in **a seventh aspect,** the invention pertains to a method for determining whether a subject has, or is at risk of, developing a proliferative disorder, such as a tumour, that is associated with cellular resistance against a cell-dependent or cell-independent cytotoxic stimulus, such as of a cell-mediated immune response, the method comprising the step of:
- detecting an applicable biomarker in a biological sample from said subject;
wherein the detection of the applicable biomarker in the sample indicates the proliferative disorder, or a risk of developing the proliferative disorder, in the subject; and
wherein the applicable biomarker is one or more selected from the group consisting of:
- CAMK1D, in particular the presence (or an amount) of or expression and/or activity of CAMK1D, preferably of phosphorylated CAMK1D;
- A death receptor, in particular the presence (or an amount) of or expression and/or activity of a death receptor;
- A death receptor ligand or a cell expressing a death receptor ligand, such as a FAS ligand, in particular the presence (or an amount) of or expression and/or activity of a death receptor ligand:
Preferably, wherein the applicable biomarker is detected in cells involved with the proliferative disorder contained in the biological sample.

In certain embodiments of such aspect, the detection of the applicable biomarker may comprise determining the presence or an amount of CAMK1D (and in particular phosphorylated CAMK1D), or activity thereof, in the sample, in particular such CAMK1D associated with or of tumour cells of the subject. In other (alternative or further) embodiments, the detection of the applicable biomarker may comprise determining the presence or an amount of one or more of the other applicable biomarkers as described above.

In certain embodiments of such aspect, the detection of the applicable biomarker may comprise determining the presence or an amount of a death receptor (and in particular FasR (or CD95) or TRAIL receptor such as DR4 or DR5), or activity thereof, in the sample, in particular in cells associated with the proliferative disorder, such as of tumour cells of the subject. Preferably, certain embodiments of such aspect may include the detection of both a CAMK1D (and in particular phosphorylated CAMK1D) and a death receptor (and in particular FasR (or CD95) or TRAIL receptor such as DR4 or DR5), or activity thereof, in particular in cells associated with the proliferative disorder, such as of tumour cells of the subject.

In certain embodiments of such aspect, the detection of the applicable biomarker may comprise determining the presence or an amount of a death receptor ligand (and in particular FasL (or CD95L) or TRAIL, or a functional equivalent or analog thereof), or activity thereof, in the sample, in particular in cells associated with the proliferative disorder, such as of tumour cells of the subject.

In **an eighth aspect,** the invention pertains to a method for determining whether a subject has, or has a risk of developing, a disease, disorder or condition that is associated with resistance against pro apoptotic stimuli, such as pro apoptotic stimuli elicited by cell-mediated immune responses, and wherein the proliferative disorder is associated with expression or activity of CAMK1D, the method comprising the steps of:
- contacting cells of the subject suspected to be involved with the disease, disorder or condition with a CAMK1D inhibitor in the presence of a pro apoptotic signal: (i) immune cells capable of eliciting or eliciting pro apoptotic stimuli towards cells involved with the proliferative disease, disorder or condition, such as lymphocytes, T-cells, CTLs and TILs; or (ii) a pro apoptotic stimulus such as a soluble or substrate bound death receptor agonist or ligand; and
- determining initiation of apoptosis in the cells involved with the proliferative disorder of the subject,
wherein an enhancement of the initiation of apoptosis in the cells of the subject indicates that the subject has or has a risk of developing such disease, disorder or condition.

In particular embodiments, such detection and/or determination methods can be practiced as a method of diagnosis, such as a method of diagnosis whether a mammalian subject (such as a human subject or patient) has a disease, disorder or condition, in particular (the presence of) a proliferative disorder such as a cancer or tumour (or has a risk of developing such a disease, disorder or condition) that is associated with cellular resistance against a cytotoxic stimulus, for example such stimulus in context of a cell-mediated immune response, and/or that is associated with (aberrant) expression or activity of CAMK1D; in particular a tumour, such as one having cellular resistance against a cell-mediated immune response.

In certain embodiments of these detection, determination and/or diagnostic methods, the cellular resistance against a cell-mediated immune response is cellular resistance against a T cell-mediated immune response, in particular cellular resistance to the killing effect of FasL and/or TRAIL, or respectively of death receptor signalling, such as signalling of Fas and/or a TRAIL receptor such as DR4 or DR5.

In some embodiments, these detection, determination and/or diagnostic methods may be a computer-implemented method, or one that is assisted or supported by a computer. In some embodiments, information reflecting the presence or an amount of the applicable biomarker (eg CAMK1D) to be determined (or activity thereof) in a sample is obtained by at least one processor, and/or information reflecting the presence or an amount of such marker (or activity thereof) in a sample is provided in user readable format by another processor. The one or more processors may be coupled to random access memory operating under control of or in conjunction with a computer operating system. The processors may be included in one or more servers, clusters, or other computers or hardware resources, or may be implemented using cloud-based resources. The operating system may be, for example, a distribution of the Linux™ operating system, the Unix™ operating system, or other open-source or proprietary operating system or platform. Processors may communicate with data storage devices, such as a database stored on a hard drive or drive array, to access or store program instructions other data. Processors may further communicate via a network interface, which in turn may communicate via the one or more networks, such as the Internet or other public or private networks, such that a query or other request may be received from a client, or other device or service. In some embodiments, the computer-implemented method of detecting the presence or an amount of the applicable biomarker (or activity thereof) in a sample is provided as a kit.

Such detection, determination and/or diagnosis methods can be conducted as an in-vitro (eg ex-vivo) method, and can be, for example, practiced using the kit of the present invention (or components thereof). An in-vitro method may use, involve or be practised on immortalised cell lines (such as those replicated, cultured or indefinitely maintained outside of the body of an animal or human), or it may be use, involve or be practised in-vitro using cells (such as primary cells) directly or freshly obtained from the body of an animal of human (eg, practised as a so-called "ex-vivo" method).

In some embodiments of these detection, determination and/or diagnosis methods, the biological sample is a tissue sample from the subject, such as a sample of a tumour or a cancer from the subject. As described above, such tissue sample may be a biopsy sample of the tumour or a cancer such as a needle biopsy sample, or a tumour biopsy section or an archival sample thereof. Such a tissue sample may comprise living, dead or fixed cells, such as from the tumour or a cancer, and such cells may be suspected of expressing (e.g. aberrantly or localised) the applicable biomarker to be determined.

In some embodiments, determination and/or diagnosis method of the invention can comprise, such as in a further step, comparing the detected amount (or activity of) of (eg protein or mRNA of) the applicable biomarker (eg CAMK1D, and in particular phosphorylated CAMK1D) with a standard or cut-off value; wherein a detected amount greater than the standard or cut-off value indicates a phenotype (or a risk of developing a phenotype) that is associated with cellular resistance against the cell-mediated immune response in the subject and/or is associated with is associated with (aberrant) expression or activity of CAMK1D in the subject. Such a standard or cut-off value may be determined from the use of a control assay, or may be pre-determined from one or more values obtained from a study or a plurality of samples having known phenotypes. For example, a cut-off value for a diagnostic test may be determined by the analysis of samples taken from patients in the context of a controlled clinical study, and determination of a cut-off depending on the desired (or obtained) sensitivity and/or specificity of the test.

Examples of methods useful in the detection of (such as the presence or absence of, or an amount of) the applicable biomarker (such as CAMK1D, and in particular phosphorylated CAMK1D) include immunoassays, such as the enzyme linked immunosorbent assay (ELISA) and the radioimmunoassay (RIA), which employ an antigen binding protein ("ABP") such as an antibody or an antigen-binding fragment thereof, that specifically binds to such applicable biomarker.

An "antigen binding protein" ("ABP") as used herein includes the meaning of a protein that specifically binds one or more epitope(s) displayed by or present on a target antigen. Typically, an antigen binding protein is an antibody (or a fragment thereof), however other forms of antigen binding protein are also envisioned by the invention. For example, the ABP may be another (non-antibody) receptor protein derived from small and robust non-immunoglobulin "scaffolds"; such as those equipped with binding functions for example by using methods of combinatorial protein design (Gebauer & Skerra, 2009; Curr Opin Chem Biol, 13:245). Particular examples of such non-antibody ABPs include: Affibody molecules based on the Z domain of Protein A (Nygren, 2008; FEBS J 275:2668); Affilins based on gamma-B crystalline and/or ubiquitin (Ebersbach et al, 2007; J Mo Biol, 372:172); Affimers based on cystatin (Johnson et al, 2012; Anal Chem 84:6553); Affitins based on Sac7d from Sulfolobus acidcaldarius (Krehenbrink et al, 2008; J Mol Biol 383:1058); Alphabodies based on a triple helix coiled coil (Desmet et al, 2014; Nature Comms 5:5237); Anticalins based on lipocalins (Skerra, 2008; FEBS J 275:2677); Avimers based on A domains of various membrane receptors (Silverman et al, 2005; Nat Biotechnol 23:1556); DARPins based on an ankyrin repeat motif (Strumpp et al, 2008; Drug Discov Today, 13:695); Fynomers based on an SH3 domain of Fyn (Grabulovski et al, 2007; J Biol Chem 282:3196); Kunitz domain peptides based on Kunitz domains of various protease inhibitors (Nixon et al, Curr opin Drug Discov Devel, 9:261) and Monobodies based on a 10th type III domain of fibronectin (Koide & Koide, 2007; Methods Mol Biol 352:95).

Alternatively, the presence of the applicable biomarker (eg CAMK1D, and in particular phosphorylated CAMK1D) may be detected by detection of the presence of mRNA that encodes such applicable biomarker, or fragments of such mRNA. Methods to detect the presence of such mRNA (or fragments) can include, PCR (such as quantitative RT-PCR), hybridisation (such as to Illumina chips), nucleic-acid sequencing etc. Such methods may involve or comprise steps using one or more nucleic acids as described herein, such as PCR primers or PCR probes, or hybridisation probes, that bind (eg specifically) to such mRNA.

In **a ninth aspect,** the invention pertains to a method for stratifying a subject that suffers from a proliferative disorder into a patient group that is distinguished by having a poor prognosis or into a patient group that does not have a poor prognosis, the method comprising the steps of:
- detecting an applicable biomarker in a biological sample from said subject, in particular wherein the biological sample comprises cells involved with the proliferative disorder;
wherein the detection of the applicable biomarker in the sample indicates that the subject is stratified into the group of patients having a poor prognosis, and wherein no detection of the applicable biomarker in the sample indicates that the subject is stratified into the group of patients not having a poor prognosis; and
wherein the applicable biomarker is one, preferably both, selected from the group consisting of:
- CAMK1D, in particular the presence (or an amount) of or expression and/or activity of CAMK1D, preferably of phosphorylated CAMK1D; or
- a death receptor, in particular the presence (or an amount) of or expression and/or activity of a death receptor;
Preferably, wherein the applicable biomarker is detected in cells involved with the proliferative disorder contained in the biological sample.

In **a tenth aspect,** the invention pertains to a use of an antigen binding protein (ABP) capable of binding specifically to CAMK1D or phosphorylated CAMK1D in an *in-vitro* diagnosis of a proliferative disease, disorder or condition in a subject; wherein the proliferative disease, disorder or condition is associated with resistance against a pro apoptotic signal, such as pro apoptotic stimuli elicited by cell-mediated immune responses, and wherein the proliferative disease, disorder or condition is associated with expression or activity of CAMK1D.

In **an eleventh aspect,** the invention pertains to a use of a kit in an *in-vitro* diagnosis of a proliferative disease, disorder or condition in a subject, wherein the proliferative disease, disorder or condition is associated with resistance against a pro apoptotic stimuli, such as pro apoptotic stimuli elicited by cell-mediated immune responses, for example which are mediated by a TNR6 ligand positive immune cell, and wherein the proliferative disease, disorder or condition is associated with expression or activity of CAMK1D, wherein the kit comprises: (a) either (x) a nucleic acid capable of binding specifically to CAMK1D, or (y) an ABP binding specifically to CAMK1D; and (b) optionally (i) instructions describing how to use the ABP or a nucleic acid or kit for detecting CAMK1D activity in a sample obtained from a subject; and/or (ii) one or more other item, component, reagent or other means useful for the use of the kit or the detection of CAMK1D activity in the sample.

A kit for use in a diagnostic method for determining whether a subject has, or has a risk of developing, a disease, disorder or condition that is associated with resistance against a pro apoptotic stimulus, such as a cell-mediated response mediated by a TNR6 ligand positive immune cell, and that is associated with expression or activity of CAMK1D; wherein:
- the diagnostic method comprises a step of surgically obtaining a sample from the subject; and
- the kit comprises: (a) either (x) a nucleic acid capable of binding specifically to CAMK1D, or (y) an ABP binding specifically to CAMK1D; and (b) optionally (i) instructions describing how to use the ABP or a nucleic acid or kit for detecting CAMK1D activity in the sample; and/or (ii) one or more other item, component, reagent or other means useful for the use of the kit or the detection of CAMK1D activity in the sample.

In certain embodiments of the kit, an additional component may comprise instructions describing how to use the ABP or a nucleic acid or kit, for detecting the presence of the applicable biomarker in the sample, such as by detecting binding between the ABP and protein such applicable biomarker, and/or detecting binding between the nucleic acid and mRNA of such applicable biomarker. Such instructions may consist of a printed manual or computer readable memory comprising such instructions, or may comprise instructions as to identify, obtain and/or use one or more other components to be used together with the kit.

In other certain embodiments of the kit, the additional component may comprise one or more other item, component, reagent or other means useful for the use of the kit or practice of a detection method of the invention, including any such item, component, reagent or means disclosed herein useful for such practice. For example, the kit may further comprise reaction and/or binding buffers, labels, enzymatic substrates, secondary antibodies and control samples, materials or moieties etc.

In a particular such embodiment, the additional component may comprise means of detecting the presence of protein of the applicable biomarker (eg CAMK1D, and in particular the phosphorylated CAMK1D), such as detecting binding between the ABP and such protein.

### Screening methods of the invention:

The present invention identifies CAMK1D as a checkpoint inhibitor in tumour resistance against immune cell induced death receptor signalling. Accordingly, the invention is in particular useful to screen for candidate therapeutic agents that reduce such resistance of tumour cells. Since CAMK1D checkpoint function is based on its actions within the death receptor signalling induced apoptotic pathway, the present invention allows for many possible screening approaches for the identification of inhibitors of CAMK1D mediated resistance against cytotoxic stimuli, and in particular such stimuli elcited in context, and preferably by, a cell-mediated immune response.

Therefore, in **a twelfth aspect,** the invention pertains to a method for identifying and/or characterising a compound suitable for a treatment of a disease, disorder or condition that is characterised by resistance against death receptor signalling, in particular resistant against a cell-mediated immune response, and that is characterised by expression or activity of CAMK1D, the method comprising the steps of:
(a) bringing into contact a first cell or cell-free system which comprises and/or expresses CAMK1D mRNA or protein and (i) a candidate compound, or (ii) a candidate compound and a cell-dependent or cell-independent cytotoxic stimulus; and
(b) determining (i) the expression, activity, function and/or stability of the (eg protein or mRNA of) CAMK1D, in the first cell or cell-free system; and/or (ii) the cytotoxicity of the cell-dependent or cell-independent cytotoxic stimulus against the first cell or cell-free system;
wherein: (i) a reduced expression, activity function and/or stability of the (eg protein or mRNA of) CAMK1D, in said first cell or cell-free system contacted with the candidate compound compared to said first cell or cell-free system not contacted with said candidate compound; and/or (ii) an enhanced cytotoxicity of the cell-dependent or cell-independent cytotoxic stimulus against the first cell or cell-free system contacted with the candidate compound compared to the cytotoxicity of the cell-dependent or cell-independent cytotoxic stimulus against the first cell or cell-free system not contacted with the candidate compound; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.

In **a thirteenth aspect,** the invention pertains to a method for identifying and/or characterising a compound suitable for a treatment of a disease, disorder or condition that is characterised by resistance against death receptor signalling, in particular resistant against a cell-mediated immune response, and that is characterised by expression or activity of CAMK1D, the method comprising the steps of:
(a) bringing into contact a first cell or cell free system which comprises and/or expresses CAMK1D mRNA or protein and one or more effector caspase(s) - in particular caspase-3, -6 and/or -7 - mRNA or protein, and (i) a candidate compound, or (ii) a candidate compound, and a cell-dependent or cell-independent cytotoxic stimulus; and
(b) determining (i) the expression, activity, function and/or stability of the (eg protein or mRNA of) of the one or more effector caspase(s) - in particular caspase-3, -6 and/or -7 - in the first cell or cell-free system; and/or (ii) the cytotoxicity of the cell-dependent or cell-independent cytotoxic stimulus against the first cell or cell-free system;
wherein: (i) an increased expression, activity function and/or stability of the (eg protein or mRNA of) one or more effector caspase(s) - in particular caspase-3, -6 and/or -7 - in the first cell or cell-free system, in said first cell or cell-free system contacted with the candidate compound compared to said first cell or cell-free system not contacted with said candidate compound; and/or (ii) an enhanced cytotoxicity of the cell-dependent or cell-independent cytotoxic stimulus against the first cell or cell-free system contacted with the candidate compound compared to the cytotoxicity of the cell-dependent or cell-independent cytotoxic stimulus against the first cell or cell-free system not contacted with the candidate compound; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.

In **a fourteenth aspect,** the invention pertains to a method for identifying and/or characterising a compound suitable for a treatment of a disease, disorder or condition that is characterised by resistance against death receptor signalling, in particular resistant against a cell-mediated immune response, and that is characterised by expression or activity of CAMK1D, the method comprising the steps of:
(a) bringing into contact a first cell or cell free system which comprises and/or expresses CAMK1D mRNA or protein and calmodulin mRNA or protein, and (i) a candidate compound, or (ii) a candidate compound, and a cell-dependent or cell-independent cytotoxic stimulus; and
(b) determining or detecting (i) the expression, activity, function and/or stability of the (eg protein or mRNA of) of calmodulin in the first cell or cell-free system; and/or (ii) the specific binding of a Ca2+/calmodulin protein complex to CAMK1D protein;
wherein: (i) an increased expression, activity function and/or stability of the (eg protein or mRNA of) calmodulin in the first cell or cell-free system, in said first cell or cell-free system contacted with the candidate compound compared to said first cell or cell-free system not contacted with said candidate compound; and/or (ii) a reduced specific binding of a Ca2+/calmodulin protein complex to CAMK1D protein in the presence of the candidate compound compared to the absence of the candidate; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.

In **a fifteenth aspect,** the invention pertains to a method for identifying and/or characterising a compound suitable for a treatment of a disease, disorder or condition that is characterised by resistance against death receptor signalling, in particular resistant against a cell-mediated immune response, and that is characterised by expression or activity of CAMK1D, the method comprising the steps of:
(a) bringing into contact a first cell or cell free system which comprises and/or expresses CAMK1D mRNA or protein and CAMK kinase (CAMKK) mRNA or protein, and (i) a candidate compound, or (ii) a candidate compound, and a cell-dependent or cell-independent cytotoxic stimulus; and
(b) determining or detecting (i) the expression, activity, function and/or stability of the (eg protein or mRNA of) of CAMKK in the first cell or cell-free system; and/or (ii) the specific binding of a CAMKK protein to CAMK1D protein;
wherein: (i) an increased expression, activity function and/or stability of the (eg protein or mRNA of) CAMKK in the first cell or cell-free system, in said first cell or cell-free system contacted with the candidate compound compared to said first cell or cell-free system not contacted with said candidate compound; and/or (ii) a reduced specific binding of a CAMKK protein to CAMK1D protein in the presence of the candidate compound compared to the absence of the candidate; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.

In **a sixteenth aspect,** the invention pertains to a method for identifying and/or characterising a compound suitable for a treatment of a disease, disorder or condition that is characterised by resistance against death receptor signalling and that is characterised by expression or activity of CAMK1D, the method comprising the steps of:
(a) bringing into contact a first cell which comprises and/or expresses CAMK1D mRNA or protein and (i) a candidate compound, or (ii) a candidate compound and a death receptor stimulating agent; and
(b) determining (i) the expression, activity, function and/or stability of the (eg protein or mRNA of) CAMK1D, in the first cell; and/or (ii) the cytotoxicity of the agonist of death receptor signalling against the first cell,
wherein: (i) a reduced expression, activity function and/or stability of the (eg protein or mRNA of) CAMK1D, in said first cell contacted with the candidate compound compared to said first cell not contacted with said candidate compound; and/or (ii) an enhanced cytotoxicity of agonist of death receptor signalling against the first cell contacted with the candidate compound compared to the cytotoxicity of the death receptor stimulating agent against the first cell not contacted with the candidate compound; indicates that the candidate compound is a compound suitable for the treatment of the disease, disorder or condition that is characterised by cellular resistance against death receptor signalling and that is characterised by expression or activity of CAMK1D.

In certain embodiments of such screening aspects of the invention, the activity of the (eg protein or mRNA of) CAMK1D in the first cell or cell free system may be determined directly (eg, by antibody detection of CAMK1D protein or by PCR of CAMK1D mRNA), or may be determined by determining the presence or an amount of phosphorylated CAMK1D or of a phosphorylated effector caspase, preferably caspase-3, -6, and/or -7, in the first cells or first cell free system, in particular in the cytoplasm of the first cell. For example, a reduction in phosphorylated effector caspases, preferably of caspase-3, -6, and/or -7, in the cytoplasm of such first cell would indicate reduced activity of CAMK1D in such cell. In particular embodiments, the detection of a specific binding of CAMK1D to one of its substrate caspases indicates activation and/or function of CAMK1D in the first cell or cell free system. Specific binding of CAMK1D to one or more of its substrate caspases may be determined by co-immunoprecipitation, or other methods for the detection of protein-protein interaction (eg yeast-two-hybrid systems).

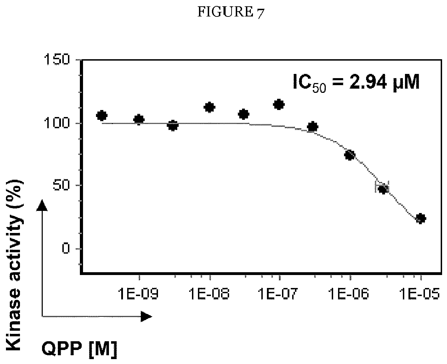

In certain embodiments the activity of the (eg protein or mRNA of) CAMK1D in the first cell or cell free system may be determined using a kinase assay as described herein in figure 7, that was used to determine the IC₅₀ of QPP inhibition of CAMK1D.

In certain embodiments of such aspects, the methods also include the step of providing (such as by obtaining) the first cell and/or the candidate compound and/or (components of) the cytotoxic stimulus, such as and including a cell-mediated immune response, in particular where each of such steps is conducted prior to the contacting step.

Typically, the methods of such aspects will be in-vitro (or ex-vivo) methods; that is, typically the methods are those not practiced on the body of a human or animal. For example, one or more of the steps of such methods may be in-vitro steps. Also, typically, such methods are practiced to identify a suitable candidate compound for purposes of further drug development. That is, in typical (but not all) embodiments, such methods are practiced (especially, if an ex-vivo methods) not to determine (eg diagnose) if a compound is a candidate for the treatment of a particular human or animal.

The reduction (or enhancement) of expression, activity function and/or stability or CAMK1D (in particular, of phosphorylated CAMK1D), or the enhancement (or reduction) in cytotoxicity is, preferably, identified by reference to a control method. In one example, the control method may be one practiced in the absence of any candidate compound, or with compound having a known effect on such expression, function, activity and/or stability (such as a positive or negative control), and/or one practiced in the absence of (one or more components of) a cell-mediated immune response.

In certain embodiments of the screening method, the (components of) a such as and including a cell-mediated immune response is a second cell which is a cytotoxic immune cell, for example a cytotoxic T-lymphocyte (CTL), capable of immunologically recognising the first cell. Accordingly, the containing step of such embodiment comprises bringing into contact a first cell and the candidate compound and a second cell, wherein the first cell expresses CAMK1D (eg, a protein or mRNA of CAMK1D) and the second cell is a cytotoxic immune cell, for example a cytotoxic T-lymphocyte (CTL), capable of immunologically recognising the first cell.

In related but alternative embodiments of the screening method, the (components of) a cytotoxic stimulus such as and including a cell-mediated immune response, is a cell-free medium that comprises soluble cell-independent cytotoxic stimuli, natural or artificial, which when brought into contact with the first cell are capable of inducing a death receptor mediated signalling, preferably via CD95 or a TRAIL receptor such as DR4 or DR5. Accordingly, the contacting step of such embodiment comprises bringing into contact a first cell and the candidate compound and a cell-free medium, wherein the first cell expresses CAMK1D (eg, a protein or mRNA of CAMK1D) and the cell-free medium comprises soluble cell-independent cytotoxic stimuli, natural or artificial, which when brought into contact with the first cell are capable of inducing a death receptor mediated signalling.

In other related but alternative embodiments of the screening method, the (components of) cell-mediated immune response is an agonist of death receptor signalling, such as CD95L or TRAIL, or functional analogs thereof. Accordingly, the contacting step of such embodiment comprises bringing into contact a first cell and the candidate compound and agonist of death receptor signalling, wherein the first cell expresses CAMK1D (eg, a protein or mRNA of CAMK1D).

In particular of such embodiments, the agonist of death receptor signalling is soluble FasL or TRAIL (such as rHuFasL or rHuTRAIL), optionally brought into contact with the first cell at a concentration of between about 0.01 ng/mL and about 1000 ng/mL. For example, the FasL or TRAIL may be brought into contact with the first cell at a concentration of about 0.05, 0.1, 0.5, 1.0, 5.0, 10, 50, 100, or 500 ng/mL, in particular between about 5 and 50 ng/mL or 50 and 200 ng/mL FasL or TRAIL.

The first cell is preferably a cell involved with a proliferative disorder (such as a tumour), eg a cell derived from a tumour. The tumour or cell thereof, may be one or, or derived from, one of the tumours described elsewhere herein.

A reduced expression, activity function and/or stability of the (eg protein or mRNA of) CAMK1D, in said first cell may be determined using varying signal read-outs that are associated with expression, activity function and/or stability of the (eg protein or mRNA of) CAMK1D. Most preferred are any assays for CAMK1D activity, expression and/or function as disclosed herein in the example section. Such assays include but are not limited to biochemical assay for kinase activity, detection of CAMK1D phosphorylation, detection of protein-protein interaction of CAMK1D with any of its target effector caspases, or with any of its upstream activating compounds, such as CaM or CAMKK. Expression may be determined using antibodies specific for CAMK1D protein, nucleic acid based methods such as sequencing, PCR and/or hybridization techniques usable for the detection of CAMK1D genetic sequences, CAMK1D promoter methylation and/or CAMK1D mRNA expression or stability. Assays useful as read-outs for the present invention and in details disclosed in the example section (see Material and Methods) include, but the invention shall not be limited to these approaches: luciferase-based cytotoxicity assay, real-time live-cell imaging assay, ELISA, flow cytometry (FACS), Calcium Imaging, functional neutralization assay, blocking assays, and luminex assays

The candidate compound used in the screening methods may be one selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules.

In particular embodiments, the candidate compound is a small molecule, such as one described elsewhere herein.

In further particular embodiments, the candidate compound is a CAMK1D inhibitor, such as one described elsewhere herein. For example, in certain of such embodiments, the candidate compound inhibits CAMK1D preferentially to inhibiting CAMK1A, CAMK1B and/or CAMK1C.

Such CAMK1D inhibiting candidate compounds, in certain embodiments, may have been identified (or characterised) as a CAMK1D inhibitor (such as a CAMK1D-specific inhibitor, eg a CAMK1D selective inhibitor) prior to it being included in a screening method of the invention. For example, a candidate compound may, as a prior step, be subjected to a CAMK1D biochemical assay - such as those provided by Promega, ProQuinase or ThermoFisher - to identify or characterise that the candidate compound is a CAMK1D inhibitor (such as a CAMK1D-specific inhibitor, eg a CAMK1D selective inhibitor).

Alternatively, for the screening purposes, the invention further comprises as CAMK1D inhibitors such compounds which have a direct inhibitory function on a direct upstream component of CAMK1D, such as CaM or CAMKK.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
Item 1: **A Calcium/calmodulin-dependent protein kinase type 1D (CAMK1D) inhibitor for use in a treatment of a proliferative disorder** in a subject, wherein the treatment involves inhibiting an activity, function, expression and/or stability of CAMK1D, preferably thereby sensitising cells involved with the proliferative disorder to a cell-dependent or cell-independent cytotoxic stimulus; wherein the treatment comprises administering the CAMK1D inhibitor to the subject.
Item 2: The CAMK1D inhibitor for use of item 1, wherein the CAMK1D inhibitor is administered in a therapeutically effective amount that is effective to reduce an activity, function, expression and/or stability of CAMK1D; preferably of CAMK1D in the cells involved with the proliferative disorder.
Item 3: The CAMK1D inhibitor for use of item 1 or 2, wherein the cell-dependent or cell-independent cytotoxic stimulus is capable of inducing or increasing apoptosis in the cells involved with the proliferative disorder.
Item 4: The CAMK1D inhibitor for use of any one of items 1 to 3, wherein the cell-dependent cytotoxic stimulus is cell-mediated immune response, such as a cytotoxic T-lymphocyte (CTL) response, and wherein preferably the cell-mediated immune response involves the expression and/or secretion, such as a cell-surface expression or secretion, of at least one immune cell effector molecule (e.g. TNR6 ligand or soluble or membrane bound TRAIL).
Item 5: The CAMK1D inhibitor for use of any one of items 1 to 4, wherein the cell-dependent or cell-independent cytotoxic stimulus is capable of inducing death receptor signalling dependent apoptosis in cells involved with the proliferative disorder, preferably wherein the death receptor is of the Tumour Necrosis Factor Receptor (TNFR) superfamily.
Item 6: The CAMK1D inhibitor for use of item 5, wherein the receptor of the TNFR superfamily is selected from Tumour necrosis factor receptor superfamily 6 (TNR6) or a Tumour necrosis factor-related apoptosis-inducing ligand (TRAIL) receptor, such as Tumour necrosis factor receptor superfamily member 10A (TNFRSF10A) or Tumour necrosis factor receptor superfamily member 10B (TNFRSF10B).
Item 7: The CAMK1D inhibitor for use of any one of items 1 to 5, wherein the treatment is administered by inhibiting an activity, function, expression and/or stability of CAMK1D protein and/or CAMK1D mRNA.
Item 8: The CAMK1D inhibitor for use of any one of items 1 to 7, wherein cells involved with the proliferative disorder are exposed to the cell-dependent or cell-independent cytotoxic stimulus, such as to a cell-mediated immune response (e.g. a CTL response).
Item 9: The CAMK1D inhibitor for use of any one of items 1 to 8, wherein the cell-dependent or cell-independent cytotoxic stimulus is selected from a substance or composition capable of binding to, and activating or increasing an activity of, a death receptor signalling pathway in the cells involved with the proliferative disorder, for example selected from (i) an agonist of TNR6 signalling (such as an agonistic anti-TNR6 antibody, a membrane bound or soluble TNR6 ligand (FAS ligand), or (ii) an agonist of TRAIL receptor signalling, such as a TRAIL or an agonistic anti-TRAIL receptor (DR4 or DR5) antibody.
Item 10: The CAMK1D inhibitor for use of any one of items 1 to 9, wherein the administration of the CAMK1D inhibitor is associated with a reduced inhibition of, in particular by a reduced phosphorylation of, effector caspases in the cells involved with the proliferative disorder, such as caspase-3, caspase-6 and/or caspase-7.
Item 11: **A CAMK1D inhibitor for use in a treatment of a proliferative disorder in a subject, the treatment comprising exposing cells involved with the proliferative disorder** in the subject to: (i) cell-dependent or cell-independent cytotoxic stimulus; and (ii) the CAMK1D inhibitor.
Item 12: The CAMK1D inhibitor for use of item 11, wherein the cell-dependent or cell-independent cytotoxic stimulus is capable of inducing or increasing apoptosis in the cells involved with the proliferative disorder, in particular death receptor mediated apoptosis.
Item 13: The CAMK1D inhibitor for use of item 11 or 12, wherein the cell-dependent or cell-independent cytotoxic stimulus is an agent that when exposed to the cells involved with the proliferative disorder induces or increases apoptosis in the cells involved with the proliferative disorder by activating, or by increasing the activation of, death receptor signalling, such as signalling by a receptor of the Tumour necrosis factor receptor superfamily.
Item 14: The CAMK1D inhibitor for use of item 13, wherein the receptor of the Tumour necrosis factor receptor superfamily is selected from Tumour necrosis factor receptor superfamily 6 (TNR6) or a Tumour necrosis factor-related apoptosis-inducing ligand (TRAIL) receptor, such as Tumour necrosis factor receptor superfamily member 10A (TNFRSF10A) or Tumour necrosis factor receptor superfamily member 10B (TNFRSF10B).
Item 15: The CAMK1D inhibitor for use of any one of items 1 to 14, wherein in (i) the cells involved with the proliferative disorder are exposed to the cell-dependent or cell-independent cytotoxic stimulus by
   (a) a cell-mediated immune response, such as CTL response, wherein the immune cells express and/or secrete a cell-dependent or cell-independent cytotoxic stimulus (for example a TNR6 ligand or TRAIL), in particular wherein the cells involved with the proliferative disorder are exposed to the immune cells such as CTL;
   (b) an administration of immune cells, such as CTL, which express and/or secrete a cell-dependent or cell-independent cytotoxic stimulus (for example a TNR6 ligand or TRAIL); and/or
   (c) an administration of a substance or composition eliciting the cell-dependent or cell-independent cytotoxic stimulus to the subject.
Item 16: The CAMK1D inhibitor for use of any one of items 11 to 15, wherein in (ii), exposing a cell involved with the proliferative disorder in the subject to the CAMK1D inhibitor is sensitising cells involved with the proliferative disorder to a pro apoptotic stimulus, and wherein the treatment comprises administering the CAMK1D inhibitor to the subject
Item 17: The CAMK1D inhibitor for use of any one of items 11 to 16, wherein the treatment comprises that the CAMK1D inhibitor is administered to the subject.
Item 18: The CAMK1D inhibitor for use of any one of items 11 to 17, wherein the treatment comprises that the CAMK1D inhibitor is administered to the subject in a therapeutically effective amount effective to inhibit CAMK1D, in particular CAMK1D in the cells involved with the proliferative disorder.
Item 19: The CAMK1D inhibitor for use of any one of items 11 to 18, wherein the treatment comprises that the CAMK1D inhibitor is administered to the subject in a therapeutically effective amount to inhibit CAMK1D mediated phosphorylation of caspase 3, 6 and/or 7.
Item 20: The CAMK1D inhibitor for use of any one of items 11 to 19, wherein the treatment comprises that a TNR6 agonist, such as an agonistic anti-TNR6 antibody or a TNR6 ligand, or a functional variant thereof, is administered to the subject.
Item 21: A **CAMK1D inhibitor for use in a treatment of a proliferative disorder** in a subject, wherein the treatment is for sensitizing cell involved with the proliferative disorder to a cell-dependent or cell-independent cytotoxic stimulus, the treatment comprising administering the CAMK1D inhibitor to the subject.
Item 22: A **CAMK1D inhibitor for use in a treatment for increasing the therapeutic index of treatment with a cell-dependent or cell-independent cytotoxic stimulus in a subject being treated therewith for a proliferative disorder,** the method comprising administering the inhibitor of CAMK1D to the subject.
Item 23: **A CAMK1D inhibitor for use in a treatment for the sensitisation of a subject suffering from a proliferative disorder to a therapy involving the administration of a cell-dependent or cell-independent cytotoxic stimulus to the subject,** the method comprising administering the CAMK1D inhibitor to the subject
Item 24: The CAMK1D inhibitor for use of any one of items 1 to 23, wherein the cell-dependent or cell-independent cytotoxic stimulus is selected from a substance or composition capable of binding to, and activating or increasing an activity of, a death receptor signalling pathway in the cells involved with the proliferative disorder, for example selected from (i) an agonist of TNR6 signalling (such as an agonistic anti-TNR6 antibody, a membrane bound or soluble TNR6 ligand (FAS ligand), or (ii) an agonist of TRAIL receptor signalling, such as a TRAIL or an agonistic anti-TRAIL receptor (anti-"DR4" or anti-"DR5") antibody.
Item 25: The CAMK1D inhibitor for use of any one of item 1 to 24, wherein the cell-dependent or cell-independent cytotoxic stimulus is capable of inducing apoptosis in the cells involved with the proliferative disorder via activation of one or more caspases, preferably wherein the one or more caspases is/are selected from caspases 3, 6 and 7.
Item 26: The CAMK1D inhibitor for use of any one of items 1 to 25, wherein the cells involved with the proliferative disorder in the subject are cells characterized by the expression, preferably by a cell surface expression, of a death receptor protein, in particular wherein the death receptor protein is selected from the TNFR superfamily, and preferably is Tumour necrosis factor receptor superfamily 6 (TNR6), or a Tumour necrosis factor-related apoptosis-inducing ligand (TRAIL) receptor, such as Tumour necrosis factor receptor superfamily member 10A (TNFRSF10A) or Tumour necrosis factor receptor superfamily member 10B (TNFRSF10B) [also known as DR4/DR5].
Item 27: The CAMK1D inhibitor for use of any one of items 1 to 26, wherein the subject is distinguished by having cells involved in the proliferative disorder which are characterised by expression and/or activity of CAMK1D, in particular such cells express mRNA and/or protein of CAMK1D, and/or are positive for such CAMK1D expression and/or activity.
Item 28: The CAMK1D inhibitor for use of item 27, wherein the subject is further distinguished by having cells involved in the proliferative disorder characterised by expression, and/or activity of, a death receptor, for example TNR6 or a TRAIL receptor, in particular such cells express mRNA and/or protein of the death receptor, and/or are positive for a cell surface expression of death receptor protein.
Item 29: The CAMK1D inhibitor for use of item 28, wherein the subject is distinguished as having been previously treated with an immunotherapy and whose tumour has progressed, in particular whose tumour relapsed, recurred or did not respond.
Item 30: The CAMK1D inhibitor for use of item 29, wherein the immunotherapy involved the use of a PD-L1/PD-1 inhibitor, preferably wherein the tumour is a PD-L1/PD-1 inhibitor sensitive tumour or is a PD-L1/PD-1 inhibitor refractory tumour.
Item 31: The CAMK1D inhibitor for use of any one of items 1 to 30, wherein the proliferative disorder is a tumour, such as a solid or a liquid tumour.
Item 32: The CAMK1D inhibitor for use of item 31, wherein the proliferative disorder is a PD-L1/PD-1 inhibitor refractory tumour.
Item 33: The CAMK1D inhibitor for use of any one of items 1 to 32, wherein the proliferative disorder is characterized by expression of (i) mRNA and/or protein of CAMK1D, or (ii) expression of mRNA and/or protein CAMK1D and expression of a death receptor, in particular such as TNR6 (Fas) or a TRAIL receptor (DR4/DR5); in the cells involved with the proliferative disorder, and thus preferably tumour cells.
Item 34: The CAMK1D inhibitor for use of any one of items 1 to 33, wherein the proliferative disorder is a tumour selected from the group consisting of a liquid or solid tumour, and preferably is selected from the group consisting of: melanoma, in particular uveal melanoma (UVM), ovarian cancer, stomach and oesophageal cancer, glioblastoma, sarcoma and colorectal cancer; or a liquid tumour such as a B cell malignancy, such as multiple myeloma (MM), or cells involved with the proliferative disorder are those of or derived from one of such tumours.
Item 35: The CAMK1D inhibitor for use of any one of items 1 to 34, wherein the CAMK1D inhibitor is a CAMK1D-specific inhibitor.
Item 36: The CAMK1D inhibitor for use of any one of items 1 to 35, wherein the CAMK1D inhibitor inhibits CAMK1D more potently than it inhibits CAMK1 alpha, CAMK1 beta and/or CAMK1 gamma.
Item 37: The CAMK1D inhibitor for use of any one of items 1 to 36, wherein the CAMK1D inhibitor is a small molecule, in particular, a small molecule ligand or a small cell-permeable molecule.
Item 38: The CAMK1D inhibitor for use of any one of items 1 to 37, wherein the CAMK1D inhibitor is a molecule of the following formula (I): 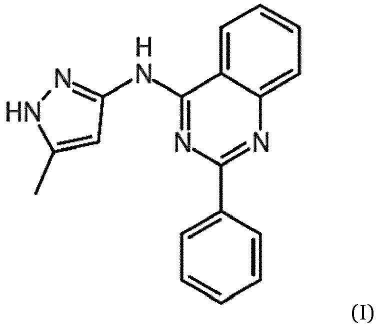 (also known as (5-Methyl-1H-pyrazol-3-yl)-(2-phenylquinazolin-4-yl)amine) and solvates, salts, complexes, stereoisomers, polymorphs, crystalline forms, racemic mixtures, diastereomers, enantiomers, tautomers, isotopically labelled forms, prodrugs, and combinations thereof.
Item 39: The CAMK1D inhibitor for use of any one of items 1 to 36, wherein the CAMK1D inhibitor is selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a hetero-bi-functional compound (such as a PROTAC or a HyT molecule); a carbohydrate such as a polysaccharide or oligosaccharide and the like, including variants or derivatives thereof; a lipid such as a fatty acid and the like, including variants or derivatives thereof.
Item 40: **A CAMK1D inhibitor for use in the treatment of a proliferative disorder** in a subject in need of such treatment, the treatment comprising a step of administering to the subject the CAMK1D inhibitor recited in any one of the preceding items, in an effective amount to sensitise cells involved with the proliferative disorder in the subject to a death receptor stimulating agent.
Item 41: The CAMK1D inhibitor of item 40, further comprising a step of administering to the subject a therapeutically effective amount of a death receptor stimulating agent.
Item 42: The CAMK1D inhibitor of item 40 or 41, wherein the death receptor is a death receptor recited in any one of the preceding items.
Item 43: The CAMK1D inhibitor of any one of items 40 to 42, wherein the death receptor stimulating agent is a substance or composition capable of binding to, and activating or increasing activity of, a death receptor signalling pathway in the cells involved with the proliferative disorder, and preferably which is an agonist of TNR6 signalling, such as an membrane bound or soluble TNR6 ligand (FAS ligand), or an agonist of TRAIL receptor signalling, such as TRAIL.
Item 44: **A method for determining whether a subject has, or is at risk of, developing a proliferative disorder,** such as a tumour, that is associated with cellular resistance against a cell-dependent or cell-independent cytotoxic stimulus, such as of a cell-mediated immune response, the method comprising the step of:
   (a) detecting an applicable biomarker in a biological sample from said subject;
      wherein the detection of the applicable biomarker in the sample indicates the proliferative disorder, or a risk of developing the proliferative disorder, in the subject; and
      wherein the applicable biomarker is one or more selected from the group consisting of:
      (i) CAMK1D, in particular the presence (or an amount) of or expression and/or activity of CAMK1D, preferably of phosphorylated CAMK1D;
      (ii) A death receptor, in particular the presence (or an amount) of or expression and/or activity of a death receptor;
      (iii) A death receptor ligand or a cell expressing a death receptor ligand, such as a FAS ligand, in particular the presence (or an amount) of or expression and/or activity of a death receptor ligand:
   Preferably, wherein the applicable biomarker is detected in cells involved with the proliferative disorder contained in the biological sample.
Item 45: The method according to item 38, wherein the detection of the applicable biomarker comprises determining the presence or an amount of CAMK kinase (CAMKK), or activity thereof, in the sample, in particular in the cytoplasm in cells comprised in the sample.
Item 46: **A method for determining** whether a subject has, or has a risk of developing, a disease, disorder or condition that is associated with resistance against pro apoptotic stimuli, such as pro apoptotic stimuli elicited by cell-mediated immune responses, and wherein the proliferative disorder is associated with expression or activity of CAMK1D, the method comprising the steps of:
   - independent cytotoxic stimulus, such as of a cell-mediated immune response, the method comprising the step of:
      (a) contacting cells of the subject suspected to be involved with the disease, disorder or condition with a CAMK1D inhibitor in the presence of a pro apoptotic signal: (i) immune cells capable of eliciting or eliciting pro apoptotic stimuli towards cells involved with the proliferative disease, disorder or condition, such as lymphocytes, T-cells, CTLs and TILs; or (ii) a pro apoptotic stimulus such as a soluble or substrate bound death receptor agonist or ligand; and
      (b) determining initiation of apoptosis in the cells involved with the proliferative disorder of the subject,
   wherein an enhancement of the initiation of apoptosis in the cells of the subject indicates that the subject has or has a risk of developing such disease, disorder or condition.
Item 47: The method of item 36, wherein the initiating of apoptosis is determined by determining caspase activation, such as activation of caspase 3, 6 and/or 7.
Item 48: **A method for stratifying a subject** that suffers from a proliferative disorder into a patient group that is distinguished by having a poor prognosis or into a patient group that does not have a poor prognosis, the method comprising the steps of:
   (a) detecting an applicable biomarker in a biological sample from said subject, in particular
      wherein the biological sample comprises cells involved with the proliferative disorder;
      wherein the detection of the applicable biomarker in the sample indicates that the subject is stratified into the group of patients having a poor prognosis, and wherein no detection of the applicable biomarker in the sample indicates that the subject is stratified into the group of patients not having a poor prognosis; and
      wherein the applicable biomarker is one, preferably both, selected from the group consisting of:
      (i) CAMK1D, in particular the presence (or an amount) of or expression and/or activity of CAMK1D, preferably of phosphorylated CAMK1D; or
      (ii) a death receptor, in particular the presence (or an amount) of or expression and/or activity of a death receptor;
   Preferably, wherein the applicable biomarker is detected in cells involved with the proliferative disorder contained in the biological sample.
Item 49: **A use of an antigen binding protein (ABP)** capable of binding specifically to CAMK1D or phosphorylated CAMK1D in an in-vitro diagnosis of a proliferative disease, disorder or condition in a subject; wherein the proliferative disease, disorder or condition is associated with resistance against a pro apoptotic signal, such as pro apoptotic stimuli elicited by cell-mediated immune responses, and wherein the proliferative disease, disorder or condition is associated with expression or activity of CAMK1D.
Item 50: The use according to item 49, wherein the cell-mediated immune response is a response mediated by a TNR6-ligand positive immune cell, such as a TNR6-ligand positive T-cell.
Item 51: **A use of a kit in an in-vitro diagnosis** of a proliferative disease, disorder or condition in a subject, wherein the proliferative disease, disorder or condition is associated with resistance against a pro apoptotic stimuli, such as pro apoptotic stimuli elicited by cell-mediated immune responses, for example which are mediated by a TNR6 ligand positive immune cell, and wherein the proliferative disease, disorder or condition is associated with expression or activity of CAMK1D, wherein the kit comprises: (a) either (x) a nucleic acid capable of binding specifically to CAMK1D, or (y) an ABP binding specifically to CAMK1D; and (b) optionally (i) instructions describing how to use the ABP or a nucleic acid or kit for detecting CAMK1D activity in a sample obtained from a subject; and/or (ii) one or more other item, component, reagent or other means useful for the use of the kit or the detection of CAMK1D activity in the sample.
Item 52: **A kit for use in a diagnostic method** for determining whether a subject has, or has a risk of developing, a disease, disorder or condition that is associated with resistance against a pro apoptotic stimulus, such as a cell-mediated response mediated by a TNR6 ligand positive immune cell, and that is associated with expression or activity of CAMK1D; wherein:
   - the diagnostic method comprises a step of surgically obtaining a sample from the subject; and
   - the kit comprises: (a) either (x) a nucleic acid capable of binding specifically to CAMK1D, or (y) an ABP binding specifically to CAMK1D; and (b) optionally (i) instructions describing how to use the ABP or a nucleic acid or kit for detecting CAMK1D activity in the sample; and/or (ii) one or more other item, component, reagent or other means useful for the use of the kit or the detection of CAMK1D activity in the sample.
Item 53: The method of any one of items 44 to 48, the use of any one of items 49 to 51, or the kit of item 52, wherein the disease, disorder or condition is, and/or is determined to be, one, or the subject is distinguished as one, suitable for treatment with a CAMK1D inhibitor, or a treatment with a CAMK1D inhibitor and a substance or composition capable of inducing death receptor dependent apoptosis.
Item 54: The method of any one of items 44 to 48 or 53, the use of any one of items 49 to 51 or 53, or the kit of item 52 or 53, wherein the subject is distinguished as having been previously treated with an immunotherapy and whose tumour has progressed, in particular whose tumour relapsed, recurred or did not respond.
Item 55: The method, the use, or the kit, of item 54, wherein the immunotherapy is a therapy with an inhibitor of PD-L1/PD-1.
Item 56: **A method for identifying and/or characterising** a compound suitable for a treatment of a disease, disorder or condition that is characterised by resistance against death receptor signalling, in particular resistant against a cell-mediated immune response, and that is characterised by expression or activity of CAMK1D, the method comprising the steps of:
   (a) bringing into contact a first cell or cell-free system which comprises and/or expresses CAMK1D mRNA or protein and (i) a candidate compound, or (ii) a candidate compound and a cell-dependent or cell-independent cytotoxic stimulus; and
   (b) determining (i) the expression, activity, function and/or stability of the (eg protein or mRNA of) CAMK1D, in the first cell or cell-free system; and/or (ii) the cytotoxicity of the cell-dependent or cell-independent cytotoxic stimulus against the first cell or cell-free system;
   wherein: (i) a reduced expression, activity function and/or stability of the (eg protein or mRNA of) CAMK1D, in said first cell or cell-free system contacted with the candidate compound compared to said first cell or cell-free system not contacted with said candidate compound; and/or (ii) an enhanced cytotoxicity of the cell-dependent or cell-independent cytotoxic stimulus against the first cell or cell-free system contacted with the candidate compound compared to the cytotoxicity of the cell-dependent or cell-independent cytotoxic stimulus against the first cell or cell-free system not contacted with the candidate compound; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.
Item 57: **A method for identifying and/or characterising a compound** suitable for a treatment of a disease, disorder or condition that is characterised by resistance against death receptor signalling, in particular resistant against a cell-mediated immune response, and that is characterised by expression or activity of CAMK1D, the method comprising the steps of:
   (a) bringing into contact a first cell or cell free system which comprises and/or expresses CAMK1D mRNA or protein and one or more effector caspase(s) - in particular caspase-3, -6 and/or -7 - mRNA or protein, and (i) a candidate compound, or (ii) a candidate compound, and a cell-dependent or cell-independent cytotoxic stimulus; and
   (b) determining (i) the expression, activity, function and/or stability of the (eg protein or mRNA of) of the one or more effector caspase(s) - in particular caspase-3, -6 and/or -7 - in the first cell or cell-free system; and/or (ii) the cytotoxicity of the cell-dependent or cell-independent cytotoxic stimulus against the first cell or cell-free system;
   wherein: (i) an increased expression, activity function and/or stability of the (eg protein or mRNA of) one or more effector caspase(s) - in particular caspase-3, -6 and/or -7 - in the first cell or cell-free system, in said first cell or cell-free system contacted with the candidate compound compared to said first cell or cell-free system not contacted with said candidate compound; and/or (ii) an enhanced cytotoxicity of the cell-dependent or cell-independent cytotoxic stimulus against the first cell or cell-free system contacted with the candidate compound compared to the cytotoxicity of the cell-dependent or cell-independent cytotoxic stimulus against the first cell or cell-free system not contacted with the candidate compound; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.
Item 58: The method of item 57, wherein the cell or cell-free system comprises mRNA or protein of caspase-3, -6 and -7.
Item 59: The method of item 57 and 58, wherein step (b) comprises a determination of the function and/or activity of the one or more effector caspases by determining or detecting (i) a specific binding of CAMK1D protein to one or more of the effector caspases, and/or (ii) a CAMK1D dependent phosphorylation of the one or more effector caspases, such as preferably comprising a phosphorylation of a serine residue more preferably SER150 of caspase-3, and/or SER257 of caspase-6.
Item 60: **A method for identifying and/or characterising a compound** suitable for a treatment of a disease, disorder or condition that is characterised by resistance against death receptor signalling, in particular resistant against a cell-mediated immune response, and that is characterised by expression or activity of CAMK1D, the method comprising the steps of:
   (a) bringing into contact a first cell or cell free system which comprises and/or expresses CAMK1D mRNA or protein and calmodulin mRNA or protein, and (i) a candidate compound, or (ii) a candidate compound, and a cell-dependent or cell-independent cytotoxic stimulus; and
   (b) determining or detecting (i) the expression, activity, function and/or stability of the (eg protein or mRNA of) of calmodulin in the first cell or cell-free system; and/or (ii) the specific binding of a Ca2+/calmodulin protein complex to CAMK1D protein;
   wherein: (i) an increased expression, activity function and/or stability of the (eg protein or mRNA of) calmodulin in the first cell or cell-free system, in said first cell or cell-free system contacted with the candidate compound compared to said first cell or cell-free system not contacted with said candidate compound; and/or (ii) a reduced specific binding of a Ca2+/calmodulin protein complex to CAMK1D protein in the presence of the candidate compound compared to the absence of the candidate; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.
Item 61: **A method for identifying and/or characterising a compound** suitable for a treatment of a disease, disorder or condition that is characterised by resistance against death receptor signalling, in particular resistant against a cell-mediated immune response, and that is characterised by expression or activity of CAMK1D, the method comprising the steps of:
   (a) bringing into contact a first cell or cell free system which comprises and/or expresses CAMK1D mRNA or protein and CAMK kinase (CAMKK) mRNA or protein, and (i) a candidate compound, or (ii) a candidate compound, and a cell-dependent or cell-independent cytotoxic stimulus; and
   (b) determining or detecting (i) the expression, activity, function and/or stability of the (eg protein or mRNA of) of CAMKK in the first cell or cell-free system; and/or (ii) the specific binding of a CAMKK protein to CAMK1D protein;
   wherein: (i) an increased expression, activity function and/or stability of the (eg protein or mRNA of) CAMKK in the first cell or cell-free system, in said first cell or cell-free system contacted with the candidate compound compared to said first cell or cell-free system not contacted with said candidate compound; and/or (ii) a reduced specific binding of a CAMKK protein to CAMK1D protein in the presence of the candidate compound compared to the absence of the candidate; indicates that the candidate compound is a compound suitable for the treatment of the disease, disorder or condition that is characterised by resistance against death receptor signalling, in particular resistance against a cell-mediated immune response, and that is characterised by expression or activity of CAMK1D.
Item 62: The method of item 62, wherein the activity or function of CAMKK is an activity of CAMKK to phosphorylate CAMK1D, and thereby, to activate an activity or function of CAMK1D.
Item 63: The method of any one of items 56 to 62, wherein the cell-dependent or cell-independent cytotoxic stimulus is a substance or composition capable of binding to, and activating or increasing activity of, a death receptor signalling pathway, or a downstream component of death receptor signalling, in the cell or cell-free, and preferably is an agonist of TNR6 signalling, such as a membrane bound or soluble TNR6 ligand (FAS ligand), or is an agonist of TRAIL receptor signalling, such as TRAIL.
Item 64: The method of item 63, wherein the cell-dependent or cell-independent cytotoxic stimulus is selected from (i) a protein, such as a death receptor ligand (e.g. a FAS ligand), an agonistic antibody binding to a death receptor, or a small molecular agonist of a death receptor; or (ii) a cell, such as an immune cell (e.g. cytotoxic T lymphocyte) and/or a cell expressing or comprising a protein according to (i).
Item 65: **A method for identifying and/or characterising a compound** suitable for a treatment of a disease, disorder or condition that is characterised by resistance against death receptor signalling and that is characterised by expression or activity of CAMK1D, the method comprising the steps of:
   (a) bringing into contact a first cell which comprises and/or expresses CAMK1D mRNA or protein and (i) a candidate compound, or (ii) a candidate compound and a death receptor stimulating agent; and
   (b) determining (i) the expression, activity, function and/or stability of the (eg protein or mRNA of) CAMK1D, in the first cell; and/or (ii) the cytotoxicity of the agonist of death receptor signalling against the first cell,
   wherein: (i) a reduced expression, activity function and/or stability of the (eg protein or mRNA of) CAMK1D, in said first cell contacted with the candidate compound compared to said first cell not contacted with said candidate compound; and/or (ii) an enhanced cytotoxicity of agonist of death receptor signalling against the first cell contacted with the candidate compound compared to the cytotoxicity of the death receptor stimulating agent against the first cell not contacted with the candidate compound; indicates that the candidate compound is a compound suitable for the treatment of the disease, disorder or condition that is characterised by cellular resistance against death receptor signalling and that is characterised by expression or activity of CAMK1D.

### BRIEF DESCRIPTION OF THE FIGURES AND SEQUENCES

The figures show:

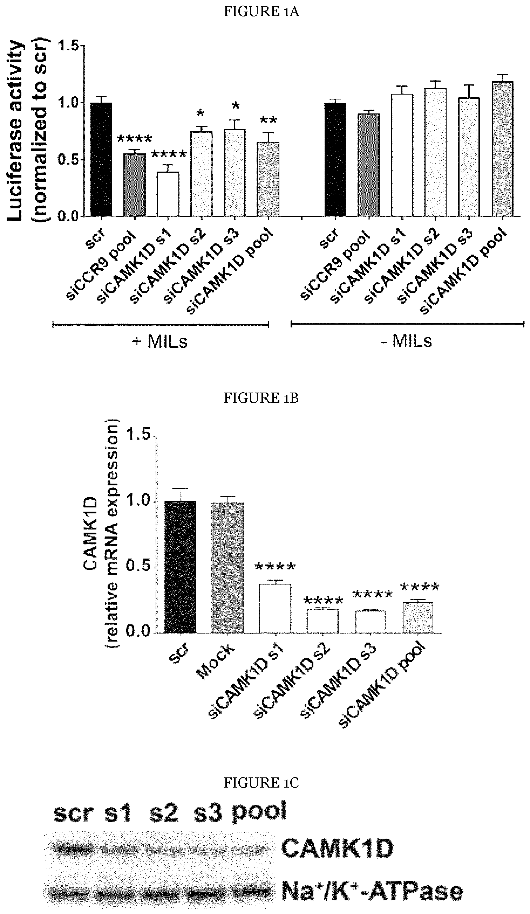

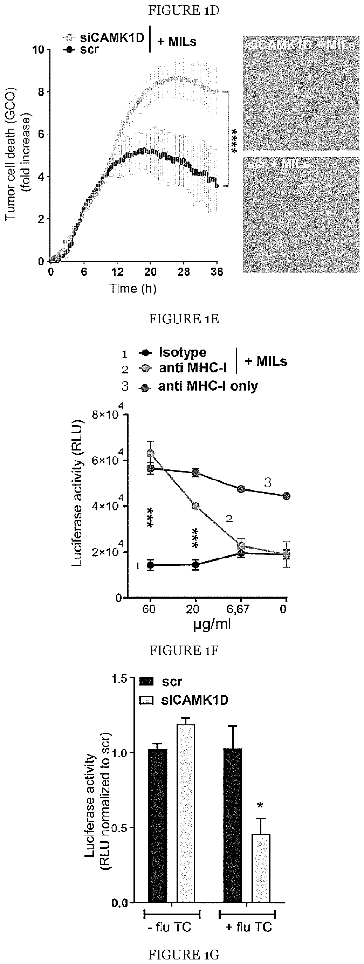

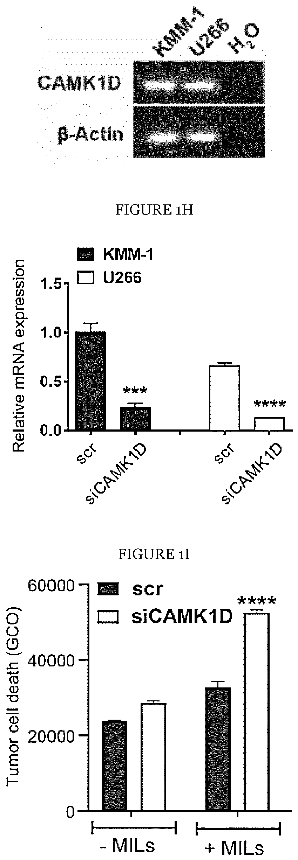

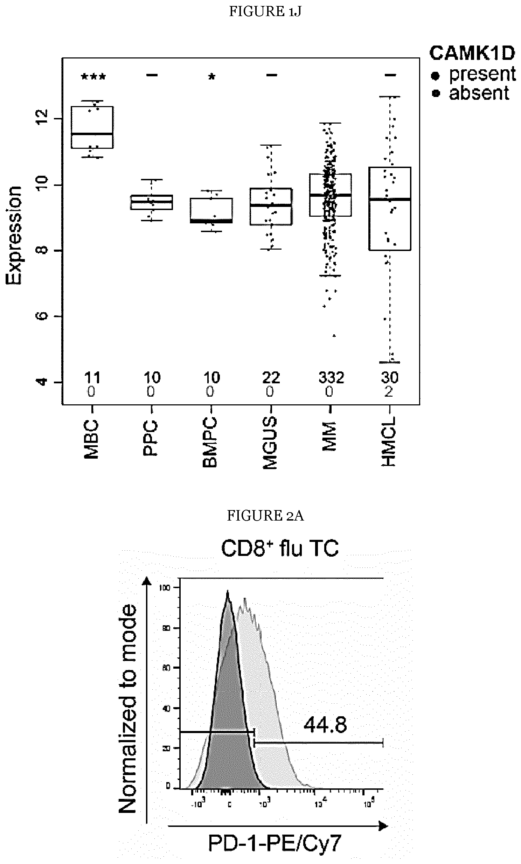

**Figure 1****:** shows the effect of siCAMKiD on cytotoxic T-cell responses. (A) KMM-1-luc cells were transfected using single (s1, s2, s3) or pooled non-overlapping siRNAs targeting CAMK1D. Control siRNA (scr) was used as a negative control, whereas pooled siCCR9 served as positive control. Transfected cells were co-cultured with MILs at 10:1 E:T ratio for the cytotoxicity setting. For the viability setting, only culture medium was added instead of T cells. T cell-mediated cytotoxicity was measured using the luciferase-based cytotoxicity assay. Values were normalized to scr control in each setting. **(B, C)** KMM-1 cells were transfected with single (s1, s2, s3) or pooled siRNAs and 48h later (B) mRNA expression levels were determined by qPCR where the results are presented in terms of fold change after normalizing to β-actin mRNA and (C) protein levels were measured via western blot analysis where the Sodium-Potassium ATPase was used as housekeeping gene. **(D)** Live cell-imaging analysis. Tumour cells were transfected with siCAMKiD or scr siRNA sequences and co-cultured with MILs. A fluorescent dye (YOYO-1) was added as an indicator of apoptosis and the graph shows the green object counted (GCO). The experiment is representative of three independent experiments. Right: Representative pictures from the live-cell microscopy. Top: siCAMKiD transfected KMM-1 cells with the addition of MILs. Bottom: scr-transfected KMM-1 cells with the addition of MILs. YOYO-i was added in the co-culture to detect apoptotic cells. Apoptotic cells are indicated by the green colour. **(E)** Luciferase-based killing assay for detection of T cell-mediated cytotoxicity in the presence of the indicated concentrations of anti-MHC-I antibody (red line) and IgG2a isotype as positive control (black line). Anti-MHC-I antibody was added to KMM-1 cells in the absence of T cells as negative control (grey line). **(F)** KMM-1-luc were pulsed with 0,005µg/ml of HLA-A*02 matched flu peptide for 1h before co-culture with flu-specific T cells or medium control (viability setting) for 20h. T cell-mediated lysis or viability impact of target knockdown was measured by luciferase assay. **(G)** End-point PCR analysis of CAMK1D expression in U266 cells. KMM-1 cells were used as positive control. β-actin was used as housekeeping gene. H₂O served as no template control. **(H)** Quantitative PCR (qPCR) showing CAMK1D knockdown efficiency in KMM-i and U266 cell lines. Results are presented in terms of fold change after normalization to β-actin mRNA. **(I)** Live cell-imaging analysis. U266 tumour cells were transfected with siCAMKiD or scr siRNA sequences and co-cultured with MILs. No MILs -condition served as viability control. Tumour cell death was measured by the addition of the YOYO-i dye. Columns show the green object counted (GCO). **(J)** CAMK1D expression by gene expression profiling (probe set 235626_at) in human MBC, PPC, BMPC, MGUS, MM and HMCL.*; BMPC showed significantly lower CAMK1D expression than PPC, MGUS, MM and HMCL (p < 0.05; each). ***; MBC showed significantly higher CAMK1D expression than BMPC, PPC, MGUS, MM and HMCL (p < 0.001; each). **(A**, **B)** Graphs show mean +/- SEM. Cumulative data of at least two independent experiments. **(D)** Graph shows mean +/- SEM. P-value was calculated using paired two-tailed student's t-test. **(E)** Representative data of at least three independent experiments. Graph shows mean +/- SD. **(F**, **H, I)** Representative data of at least two independent experiments. Graphs show mean +/- SEM. P-values were calculated using unpaired two-tailed student's t-test. * p < 0.05, ** p < 0.01, *** p < 0.001, **** p< 0.0001

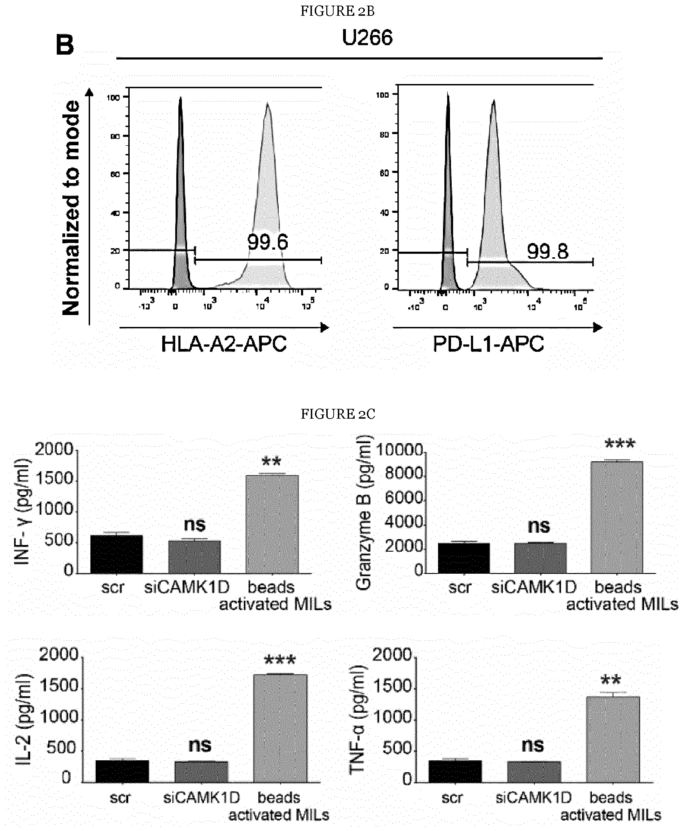

**Figure 2****:** shows the phenotypic characterization of T cells and tumour cells. **(A)** FACS-analysis of PD-1 (light blue histogram) expression on flu TC. Isotype is shown as dark grey histogram. **(B)** FACS-analysis of HLA-A2 (light grey histogram) and PD-L1 (blue histogram) on U266 tumour cells. Isotype control is shown as dark grey histogram. **(C)** KMM-1 cells were transfected with scr or CAMK1D siRNA sequences for 48h. Afterwards MILs were added at an E:T ratio of 10:1 and INF-γ, Granzyme B, IL-2 and TNF-α secretion was measured 20h after co-culture. Anti-CD3/anti-CD28 magnetic beads stimulation served as a positive control. Representative data of two independent experiments. Columns show mean +/- SD. P-values were calculated using unpaired two-tailed student's t-test. * p ≤ 0.05; ** p ≤ 0.01; *** p ≤ 0.001.

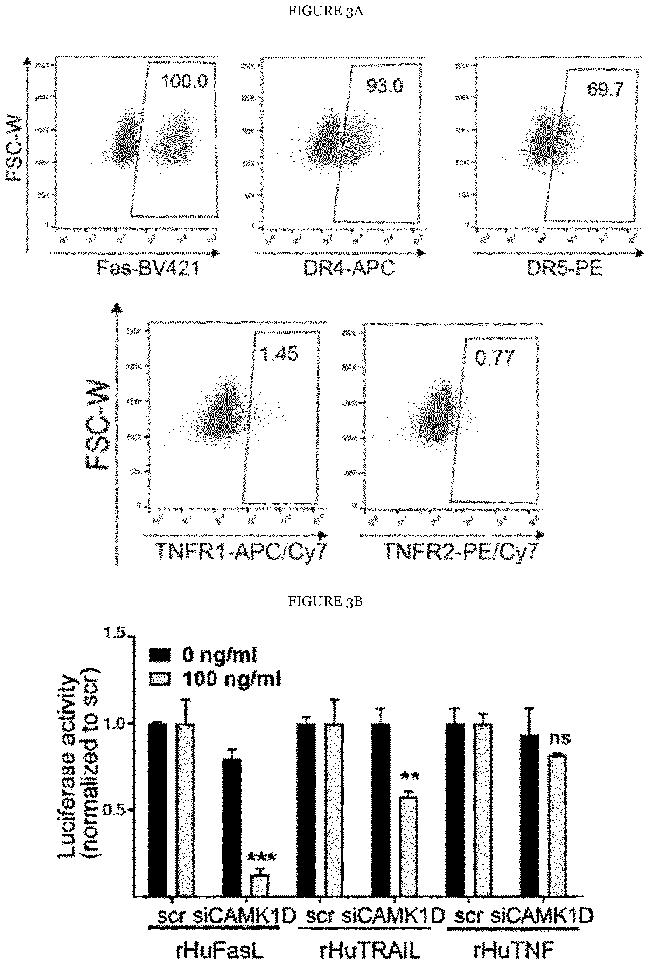

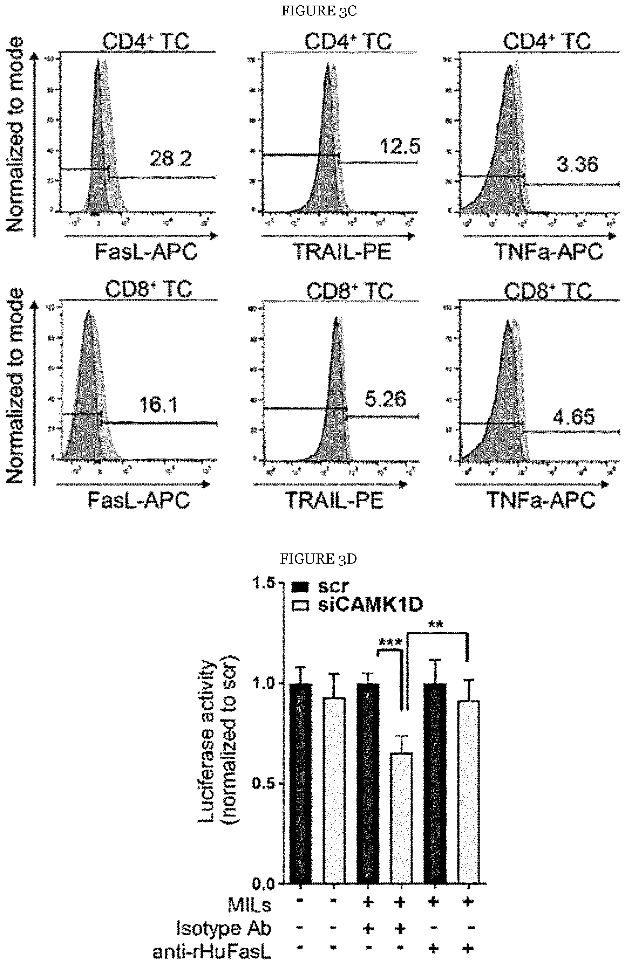

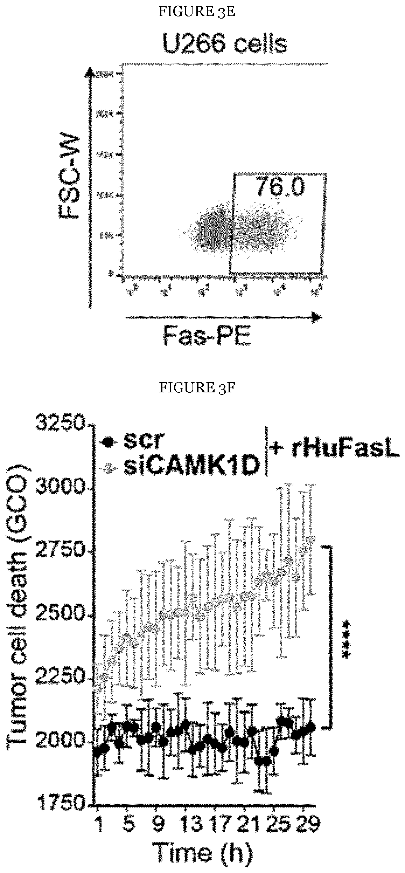

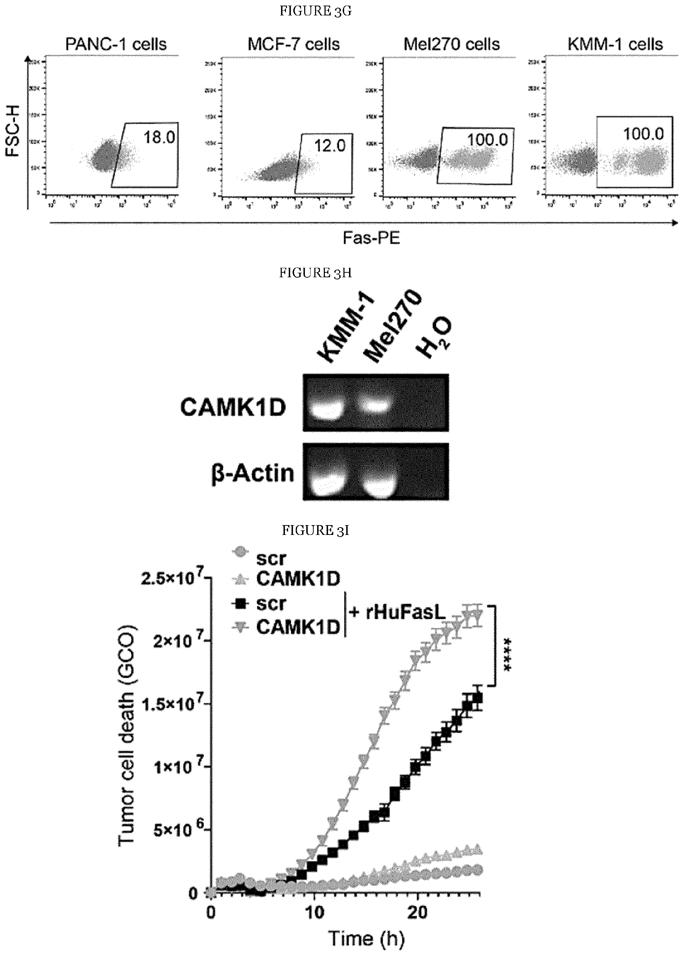

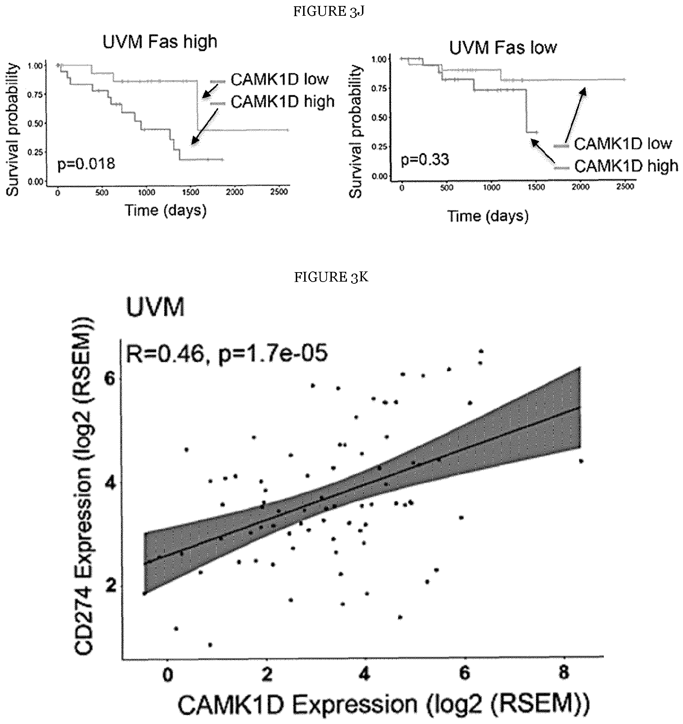

**Figure 3****:** shows the effect of CAMK1D knockdown in different tumour entities. **(A)** Representative FACS analysis of Fas, DR4, DR5, TNFR1 and TNFR2 expression in KMM-1 cells. Cell surface expression was measured by flow cytometry. Positive tumour cells are marked in orange while isotype is shown as grey dots. **(B)** KMM-1-luc cells were transfected with scr or CAMK1D siRNAs and treated with recombinant FasL, TRAIL or TNF. Luciferase activity was measured after 20h of treatment. Experiments were performed in triplicates and representative results of three independent experiments are shown. **(C)** Representative FACS analysis of FasL, TRAIL and membrane bound TNFa expression on CD4 and CD8 -positive MILs. Isotype controls are shown as grey histograms. **(D)** Luciferase-based assay: scr or siCAMKiD transfected KMM-1 were co-cultured with MILs in the presence of a FasL neutralizing (anti-FasL) antibody or with an isotype control. Loss of luciferase activity was measured. **(E)** Representative FACS analysis of Fas expression in U266 cells. Positive tumour cells are marked in orange while isotype is shown as grey dots. **(F)** Live cell-imaging analysis. U266 tumour cells were transfected with siCAMKiD or scr siRNA sequences and treated with rHuFasL. The YOYO-i dye was added as an indicator of tumour cell death. The experiment is representative of three independent experiments and shows the green objects counted (GCO). **(G)** Representative FACS analysis of Fas expression in PANC-i, MCF-7, Mel270 and KMM-1 cells. Tumour cells were stained with conjugated antibodies against Fas. Cell surface expression was measured by flow cytometry. Positive tumour cells are marked in orange while isotype is shown as grey dots. **(H)** End-point PCR showing CAMK1D expression in the uveal melanoma cell line Mel270. KMM-1 multiple myeloma cells were used as positive control. β-actin was used as housekeeping gene. Water served as no template control. **(I)** Live cell-imaging analysis showing uveal melanoma cells transfected with siCAMKiD or scr siRNA sequence upon exposure to rHuFasL or medium control. A fluorescent dye (YOYO-1) was added as an indicator of apoptosis measured as green object counted (GCO). The experiment is representative of two independent experiments. Values denote mean ± SEM. **(J)** Correlation between CAMK1D and Fas expression on patients' survival in UVM. Fas high and Fas low UVM patients were divided in CAMK1D high and low expression according to the median of CAMK1D expression. Kaplan-Meier curves showing the correlation between CAMK1D expression and patients' survival probability were generated using TCGA clinical data. Significance was calculated using the log-rank test. **(K)** CAMK1D and PD-L1 correlation is depicted. **(B**, **D)** Graphs show mean +/- SD. P-values were calculated using unpaired two-tailed student's t-test. **(F)** Graph shows mean +/- SD. P-value was calculated using paired two-tailed student's t-test. **(I)** Representative data of at least two independent experiments. Graph shows mean +/- SEM. P-value was calculated using paired two-tailed student's t-test* p < 0.05, ** p < 0.01, *** p < 0.001, **** p< 0.0001

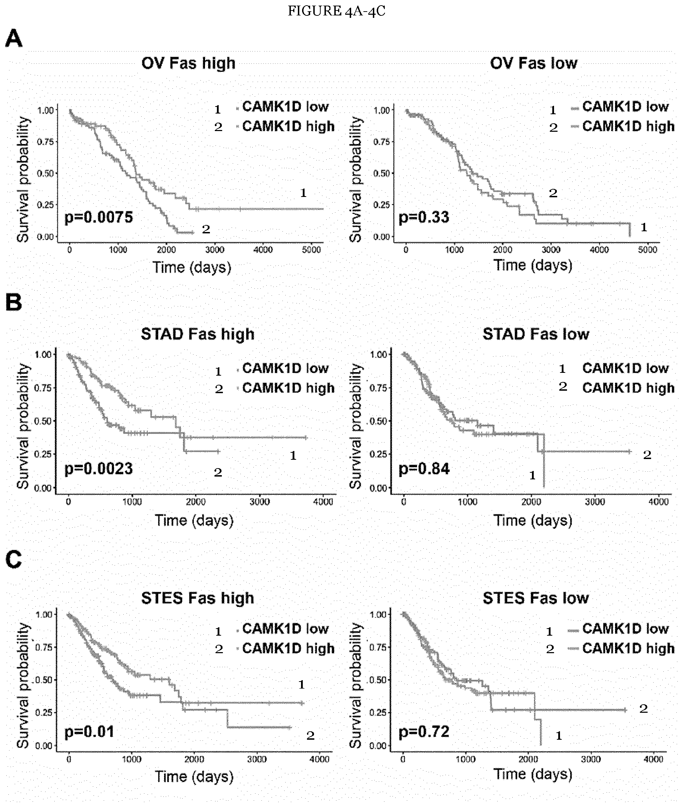

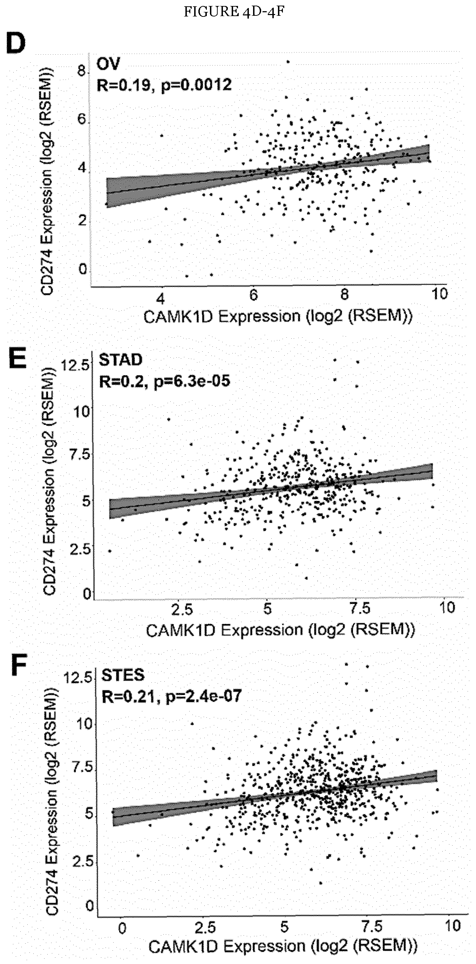

**Figure 4****:** shows TCGA analysis for patient survival probability correlating with Fas, CAMK1D and PD-L1. Correlation between CAMK1D and Fas expression on patient survival in **(A)** Ovarian serous cystadenocarcinoma (OV), **(B)** Stomach adenocarcinoma (STAD) and **(C)** Stomach and Esophageal carcinoma (STES). Fas high and Fas low OV, STAD and STES patients were divided in CAMK1D high and low expression according to the median of CAMK1D expression. Kaplan-Meier curves showing the correlation between CAMK1D expression and patients' survival probability were generated using TCGA clinical data. Significance was calculated using the log-rank test. **(D, E**, **F)** Graphs show CAMK1D and PD-L1 correlation in OV, STAD and STES.

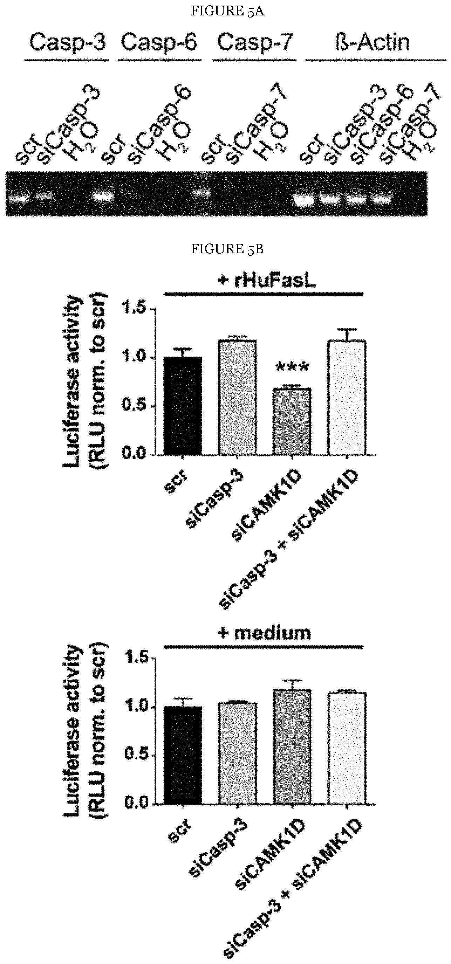

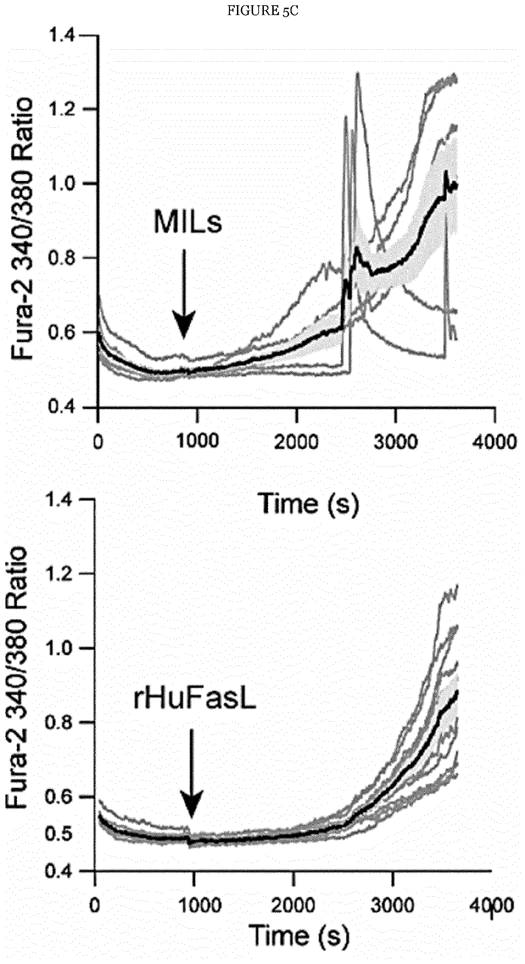

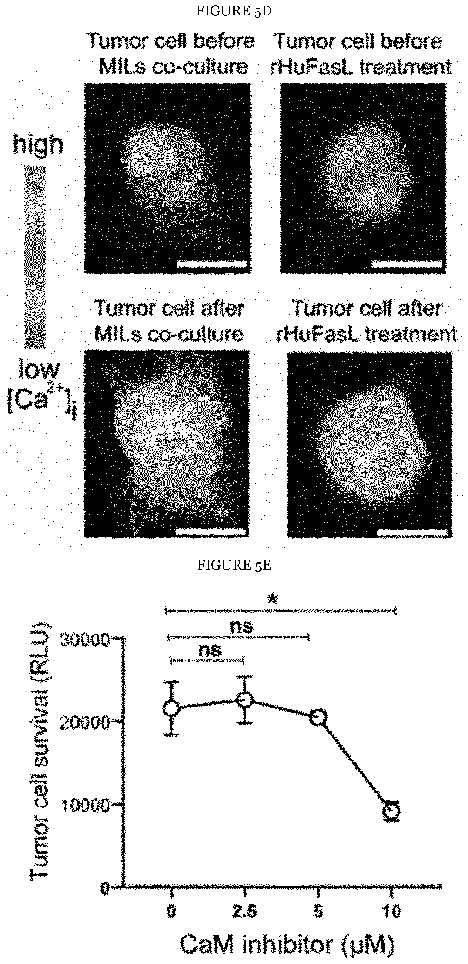

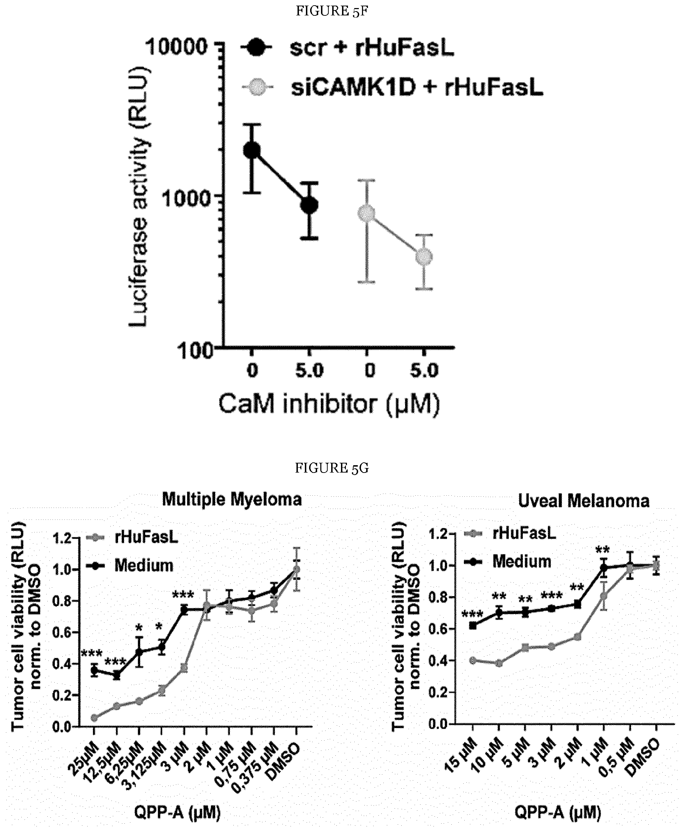

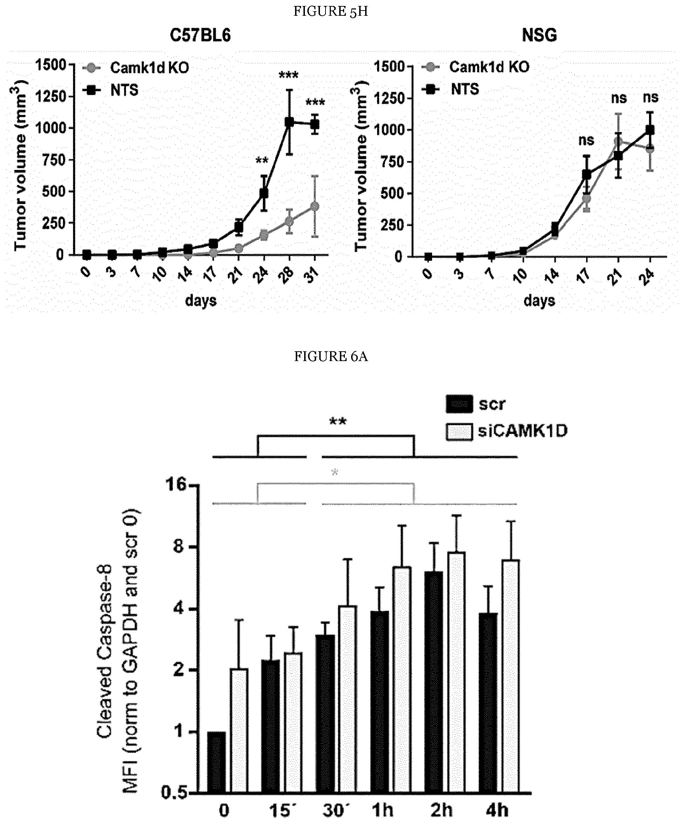

**Figure 5****:** shows CAMK1D regulation. **(A)** Caspase-3, -6 and -7 expression and knockdown in KMM-1 cells was measured via end-point PCR. **(B)** Effector caspases were knocked down alone or in combination with CAMK1D and stimulated with rHuFasL or with medium control. Luciferase activity was measured after 20h of treatment. Experiments were performed in quadruplicates and representative results of three independent experiments are shown. **(C)** Intracellular calcium response in KMM-1 cells upon (top) MILs co-culture and (bottom) rHuFasL treatment. **(D)** Representative picture of intracellular free Ca²⁺ measurement in KMM-1 scr-transfected cells before (top) and after (bottom) co-culture with MILs or treated with rHuFasL. **(E)** KMM-1 cells were treated with different concentrations of CaM inhibitor (W-7 hydrochloride) and tumour cell survival was measured by luciferase intensity. **(F)** scr and siCAMKiD KMM-1 transfected cells were treated with rHuFasL together with the indicated concentration of CaM inhibitor. **(G)** KMM-1 (left) and Mel270 (right) cells were treated with the indicated concentration of CAMK1D inhibitor (QPP) and exposed to rHuFasL or medium. Tumour cell survival was measured by luciferase intensity. **(H)** C57BL6 (n=12) and NOD/SCID gamma chain (NSG) mice (n=12) were s.c injected with 1 x 10⁵ MC38 Camkid KO and MC38 NTS cells each into the right and left flank of one mouse, respectively. Tumour growth was measured twice a week. Graphs show mean ± SEM and statistical significance was calculated using two-way ANOVA Bonferroni post test. (B) Graphs show mean ± SD. Statistical significance was calculated using unpaired, two-tailed Student's t-test. (E, F, G) Experiments were performed in triplicates and representative results of three independent experiments are shown. Graphs show mean ± SEM and statistical significance was calculated using unpaired, two-tailed Student's t-test. * p ≤ 0.05; ** p ≤ 0.01; *** p ≤ 0.001; **** p ≤ 0.0001.

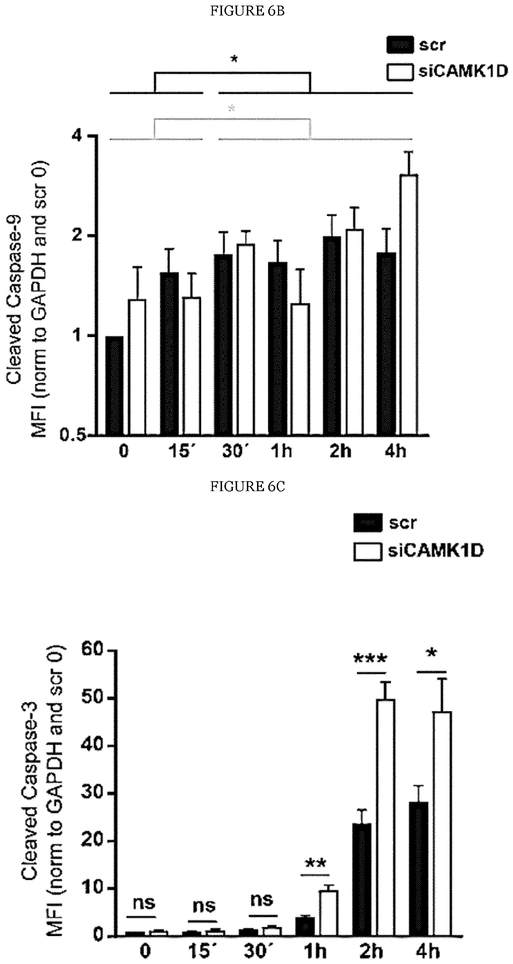

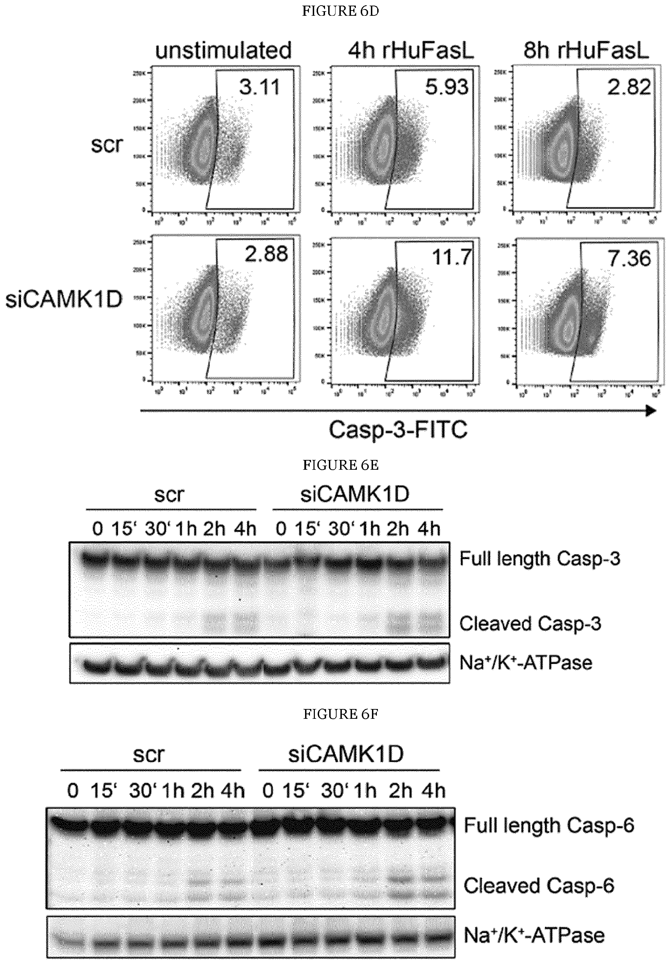

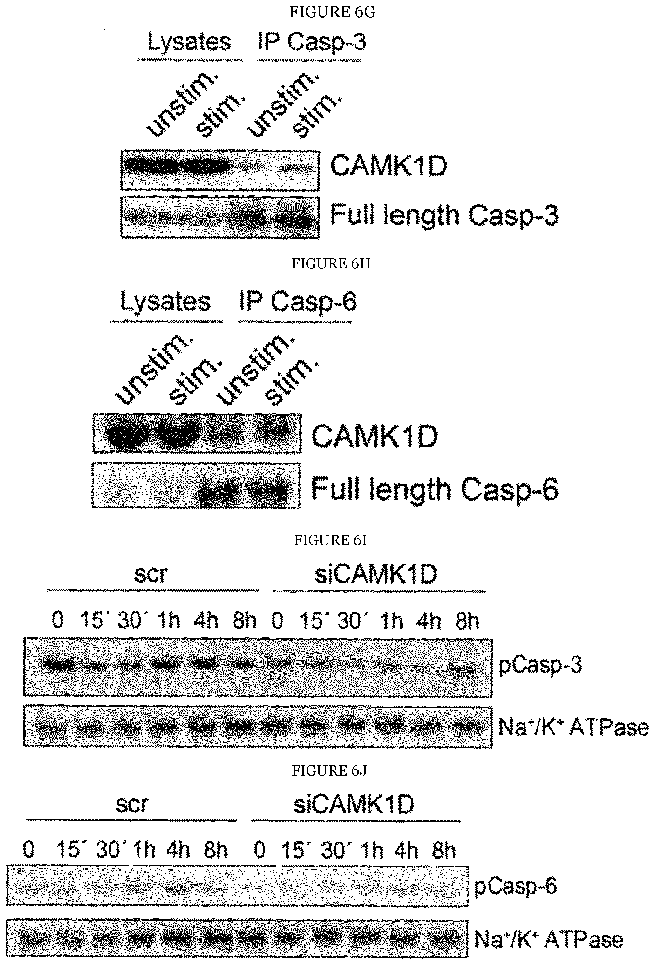

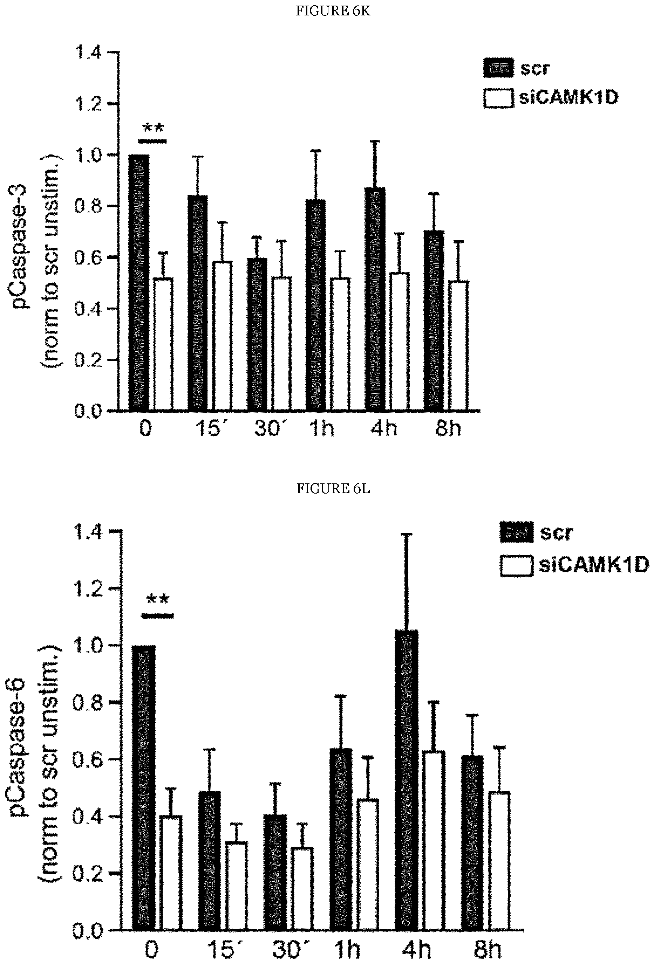

**Figure 6****:** shows pathways which are regulated by CAMK1D. **(A-C)** Luminex assays measuring apoptosis proteins. CAMK1D-proficient and -deficient cells were stimulated with rHuFasL for the indicated time frames. Protein levels were normalized to GAPDH and compared to scr-unstimulated cells. The amount of (A) cleaved caspase-8 (B) cleaved caspase-9 and (C) cleaved caspase-3 was measured. Graphs show cumulative data of at least two independent experiments. **(D)** FACS analysis of scr and siCAMKiD transfected KMM-1 cells treated for the given time frames with rHuFasL. Gate marks active caspase-3 labeled cells. **(E, F)** KMM-1 cells were treated as in (A-C) and full-length and cleaved (E) caspase-3 and (F) caspase-6 were measured via western blot. The Sodium-Potassium ATPase was used as housekeeping gene. Representative results of at least two independent experiments. **(G, H)** Representative blots showing co-immunoprecipitation of (G) caspase-3 and CAMK1D, (H) caspase-6 and CAMK1D. KMM-1 cells were stimulated with rHuFasL for 4h. Unstimulated cells were used as negative control. Unstimulated and stimulated cell lysates were used as positive control for CAMK1D detection. **(I, J)** Western blot measuring phosphorylated caspase-3 and caspase-6 upon rHuFasL stimulation. The Sodium-Potassium ATPase was used as housekeeping gene. **(K, L)** Quantification of (K) phosphorylated caspase-3 and (L) phosphorylated caspase-6 upon rHuFasL stimulation for the indicated time frames. Graphs show cumulative data of four independent experiments. (A, B, K, L) Graphs show mean ± SEM and statistical significance was calculated using unpaired, two-tailed Student's t-test. (C) Graph shows mean ± SD and statistical significance was calculated using unpaired, two-tailed Student's t-test. * p ≤ 0.05; ** p ≤ 0.01; *** p ≤ 0.001; **** p ≤ 0.0001.
**Figure 7****:** shows CAMK1D kinase inhibition by QPP; biochemical kinase inhibition assay was performed at ProQinase (Freiburg, Germany); a radiometric protein kinase assay (33PanQinase® Activity Assay) was used for measuring the kinase activity of CAMK1D; all kinase assays were performed in 96-well FlashPlates from PerkinElmer (Boston, MA, USA) in a 50 µl reaction volume, IC50 values were measured by testing 10 concentrations (1 x 10-05 M to 3 x 10 10 M) of the compound in singlicate.

The sequences show:
**SEQ ID NO: 1** (CAMK1D; UniProt identifier: Q8IU85-1, database entry of May 13, 2020): 
        10         20         30         40         50
MARENGESSS SWKKQAEDIK KIFEFKETLG TGAFSEVVLA EEKATGKLFA
        60         70         80         90        100
VKCIPKKALK GKESSIENEI AVLRKIKHEN IVALEDIYES PNHLYLVMQL
 
110 120 130 140 150
VSGGELFDRI VEKGFYTEKD ASTLIRQVLD AVYYLHRMGI VHRDLKPENL
160 170 180 190 200
LYYSQDEESK IMISDFGLSK MEGKGDVMST ACGTPGYVAP EVLAQKPYSK
210 220 230 240 250
AVDCWSIGVI AYILLCGYPP FYDENDSKLF EQILKAEYEF DSPYWDDISD
260 270 280 290 300
SAKDFIRNLM EKDPNKRYTC EQAARHPWIA GDTALNKNIH ESVSAQIRKN
310 320 330 340 350
FAKSKWRQAF NATAVVRHMR KLHLGSSLDS SNASVSSSLS LASQKDCLAP
360 370 380
STLCSFISSS SGVSGVGAER RPRPTTVTAV HSGSK
**SEQ ID NO: 2** (CAMK1D; UniProt identifier: Q8IU85-1, database entry of May 13, 2020): 
        10          20          30          40          50
MARENGESSS SWKKQAEDIK KIFEFKETLG TGAFSEVVLA EEKATGKLFA
        60          70          80          90         100
VKCIPKKALK GKESSIENEI AVLRKIKHEN IVALEDIYES PNHLYLVMQL
       110         120         130         140         150
VSGGELFDRI VEKGFYTEKD ASTLIRQVLD AVYYLHRMGI VHRDLKPENL
       160         170         180         190         200
LYYSQDEESK IMISDFGLSK MEGKGDVMST ACGTPGYVAP EVLAQKPYSK
       210         220         230         240         250
AVDCWSIGVI AYILLCGYPP FYDENDSKLF EQILKAEYEF DSPYWDDISD
       260         270         280         290         300
SAKDFIRNLM EKDPNKRYTC EQAARHPWIA GDTALNKNIH ESVSAQIRKN
       310         320         330         340         350
FAKSKWRQAF NATAVVRHMR KLHLGSSLDS SNASVSSSLS LASQKDCAYV

AKPESLS
**SEQ ID NO: 3** (siRNA sequence targeting CAMK1D)
   UGAAGUGUAUCCCUAAGAA
**SEQ ID NO: 4** (siRNA sequence targeting CAMK1D)
   CAAAUCACCUGUACUUGGU
**SEQ ID NO: 5** (siRNA sequence targeting CAMK1D)
   CCGAAAAUCUCUUGUACUA
**SEQ ID NO: 6** (siRNA sequence targeting CAMK1D)
   GAGAAGGACCCGAAUAAAA
**SEQ ID NO: 7** (gRNA sequence for targeting CAMK1D by gene editing)
   TCGATCGGATAGTGGAGAAG
**SEQ ID NO: 8** (gRNA sequence for targeting CAMK1D by gene editing)
   GGAGATAGTATACGGCATCC
**SEQ ID NO: 9** (gRNA sequence for targeting CAMK1D by gene editing)
   TAGCCGAGGAGAAAGCTACT
**SEQ ID NO: 10** (gene editing target sequence at locus: Chr.2: 5362021 - 5362043 on GRCm38)
   GGAGATAGTATACGGCATCC
**SEQ ID NO: 11** (Calmodulin (CaM); UniProt identifier: PoDP23, database entry of May 15, 2020): 
        10          20          30          40          50

        60          70          80          90         100

       110         120         130         140

**SEQ ID NO: 12** (human Calcium/calmodulin-dependent protein kinase kinase 1 isoform 1; UniProt identifier: Q8N5S9-1, database entry of May 15, 2020): 
10 20 30 40 50
MEGGPAVCCQ DPRAELVERV AAIDVTHLEE ADGGPEPTRN GVDPPPRARA
60 70 80 90 100
ASVIPGSTSR LLPARPSLSA RKLSLQERPA GSYLEAQAGP YATGPASHIS
110 120 130 140 150
PRAWRRPTIE SHHVAISDAE DCVQLNQYKL QSEIGKGAYG VVRLAYNESE
160 170 180 190 200
DRHYAMKVLS KKKLLKQYGF PRRPPPRGSQ AAQGGPAKQL LPLERVYQEI
210 220 230 240 250
AILKKLDHVN VVKLIEVLDD PAEDNLYLVF DLLRKGPVME VPCDKPFSEE
260 270 280 290 300
QARLYLRDVI LGLEYLHCQK IVHRDIKPSN LLLGDDGHVK IADFGVSNQF
310 320 330 340 350
EGNDAQLSST AGTPAFMAPE AISDSGQSFS GKALDVWATG VTLYCFVYGK
360 370 380 390 400
CPFIDDFILA LHRKIKNEPV VFPEEPEISE ELKDLILKML DKNPETRIGV
410 420 430 440 450
PDIKLHPWVT KNGEEPLPSE EEHCSVVEVT EEEVKNSVRL IPSWTTVILV
460 470 480 490 500
KSMLRKRSFG NPFEPQARRE ERSMSAPGNL LVKEGFGEGG KSPELPGVQE

DEAAS
**SEQ ID NO:** 13 (human Calcium/calmodulin-dependent protein kinase kinase 1 isoform 2; UniProt identifier: Q8N5S9-2, database entry of May 15, 2020):

10 20 30 40 50
MEGGPAVCCQ DPRAELVERV AAIDVTHLEE ADGGPEPTRN GVDPPPRARA
60 70 80 90 100
ASVIPGSTSR LLPARPSLSA RKLSLQERPA GSYLEAQAGP YATGPASHIS
110 120 130 140 150
PRAWRRPTIE SHHVAISDAE DCVQLNQYKL QSEIGKGAYG VVRLAYNESE
160 170 180 190 200
DRHYAMKVLS KKKLLKQYGF PRRPPPRGSQ AAQGGPAKQL LPLERVYQEI
210 220 230 240 250
AILKKLDHVN VVKLIEVLDD PAEDNLYLAL QNQAQNIQLD STNIAKPHSL
260 270 280 290 300
LPSEQQDSGS TWAARSVFDL LRKGPVMEVP CDKPFSEEQA RLYLRDVILG
310 320 330 340 350
LEYLHCQKIV HRDIKPSNLL LGDDGHVKIA DFGVSNQFEG NDAQLSSTAG
360 370 380 390 400
TPAFMAPEAI SDSGQSFSGK ALDVWATGVT LYCFVYGKCP FIDDFILALH
410 420 430 440 450
RKIKNEPVVF PEEPEISEEL KDLILKMLDK NPETRIGVPD IKLHPWVTKN
460 470 480 490 500
GEEPLPSEEE HCSVVEVTEE EVKNSVRLIP SWTTVILVKS MLRKRSFGNP
510 520
FEPQARREER SMSAPGNLLV

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: CAMK1D protects PD-L1+ tumour cells against death receptor signalling by cytotoxic T cells

In order to identify novel genes involved in immune escape mechanisms of cancer cells, a high-throughput screening approach recently developed (45) was adapted. The HLA-A2 positive human multiple myeloma cell line KMM-1 was used as a tumour model in this study because KMM-1 cells express high levels of PD-L1 and also lower levels of another recently characterized immune-checkpoint molecule, CCR9 (45). As a reporter system for tumour cell survival the inventors stably transfected KMM-1 cells with e-GFP-firefly luciferase, allowing to apply luminescence imaging as a reliable parameter for immune mediated tumour cell destruction in a HTP format. As a source of tumour-reactive T cells marrow-infiltrating, PD-1 positive T cells (MILs) from an HLA-A2-matched patient were used. These MILs were not terminally exhausted as they showed strong IFN-gamma secretion after polyclonal stimulation, which even exceeded that of a well-established tumour antigen specific CD8+ cytotoxic T cell clone, SK-1 (Survivin TC) (45). Moreover, they recognized and reacted by substantial IFN-gamma secretion also against KMM-1 tumour cells, despite high levels of PD-L1 expression on KMM-1. However, they exerted only limited capacity to kill KMM-1 cells (20 % killing at 10:1 E:T ratio with 5000 KMM-1-luc cells; not shown) suggesting the presence of resistance mechanisms against T cell attack in KMM-1 cells. Silencing of firefly-luciferase (siFLuc) was used as positive control for siRNA transfection efficacy, while silencing of genes essential for tumour cell survival, such as ubiquitin C (UBC) or transfection with a mixture of siRNAs inducing cell death (siCD) resulted in strong reduction of luciferase expression, indicating appropriate gene silencing and sensitivity of the luciferase-based readout. The strongest immune modulatory effect (high impact on T cell killing and no viability impact) was elicited by the serine/threonine calcium/calmodulin-dependent protein kinase 1D (CAMK1D)

Based on the strong immune resistance phenotype associated with CAMK1D expression in the screens for immune checkpoint inhibitors, the inventors focused on validation and characterization of the immune regulatory role of the intracellular kinase CAMK1D. So far, an immune-related function of CAMK1D in cancer evasion has not been studied. As a first experiment, the inventors de-convoluted the pool of CAMK1D targeting siRNAs from used in the HTP-screen to exclude potential dominant off-target effects of single siRNAs within the pool. Three out of four CAMK1D targeting siRNAs (s1, s2 and s3) and the pool of all siRNAs increased T cell mediated cytotoxicity, while no viability impact of the individual or pooled siRNAs *per se* was detected (Figure 1A) and all of them significantly reduced CAMK1D expression at mRNA and protein level (Figure 1B, 1C). In a luciferase-independent assay, employing live cell-imaging of tumour cell apoptosis using a fluorescence apoptosis dye (YOYO-1) a strong increase of MIL-induced apoptosis in CAMK1D-deficient KMM-1 cells was confirmed (Figure 1D). This could be inhibited by MHC-I blocking antibodies, indicating that tumour cell apoptosis was induced by MHC-I-restricted CD8+ MILs in a T cell receptor-dependent manner (Figure 1E). To corroborate this further, KMM-1 cells were pulsed with an HLA-A2-restricted peptide of influenza-matrix protein and co-cultured them with PD-1 positive, influenza (flu)-peptide-specific CD8+ cytotoxic T cells (flu TC) (Figure 2A). Again, CAMK1D silencing but not PD-L1 silencing (data not shown) resulted in a significant increase of T cell-mediated tumour cell lysis (Figure 1F), demonstrating that CAMK1D mediates resistance of KMM-1 cells towards an attack by antigen specific cytotoxic T cells and that this effect occurs independent of the T cell source. Of note, CAMK1D mediated immune protection not only in KMM-1 cells but to a comparable degree also in an additional PD-L1+, HLA-A2+ multiple myeloma cell line, U266 (Figure 1G-I and Figure 2B).

Next, CAMK1D expression was studied in a large cohort of CD138-purfied malignant plasma cells from multiple myeloma patients with monoclonal gammopathy of unknown significance (MGUS), human myeloma cell lines (HMCL), memory B cells (MBC), plasmablasts (PPC) and normal bone marrow plasma cells (BMPC). CAMK1D expression was highest in MBC but it was also expressed in all MM, MGUS, PPC, and in 30/32 HMCL samples and these showed higher expression than normal bone marrow plasma cells (BMPCs) (Figure 1J). Thus, these data indicate that CAMK1D is consistently expressed in human multiple myelomas and confers resistance against cytotoxic T cell attack. Classical immune-checkpoint molecules expressed by tumour cells regulate the activity of cytotoxic T cells mostly through engagement of inhibitory receptors on T cells. Since CAMK1D is an intracellular kinase, it may indirectly regulate T cell activity. Parameters of T cell effector function upon contact with CAMK1D proficient or deficient KMM-1 cells were studied next, including the secretion of the T cell effector cytokines INF-γ, Granzyme B, IL-2 or TNF-α. Although consistently increased T cell-mediated tumour cell killing was detected after CAMK1D knockdown in KMM-1 cells, functional analysis of T cells did not reveal any increased T cell function after interaction with CAMK1D-deficient compared to wt tumour cells (Figure 2C). Therefore, it is concluded that CAMK1D expression in tumour cells does not affect type 1 effector T cell function and hypothesized that it may instead regulate the sensitivity of tumour cells towards cytotoxic T cell attack.

### Example 2: CAMK1D immune checkpoint function blocks FasL and TRAIL induced T cell cytotoxicity

KMM-1 cells were then exposed to the cytotoxic agents FasL (rHuFasL), TRAIL (rHuTRAIL) or TNF (rHuTNF) commonly used by T cells to kill their target cells. The respective cell death-mediating receptors for FasL and TRAIL, Fas, DR4 and DR5 were strongly expressed on KMM-1 cells while the TNF receptors TNFR1 and TNFR2 were not expressed (Figure 3A). While CAMK1D-proficient KMM-1 cells were resistant against all tested cytotoxic agents, CAMK1D-deficient tumour cells were dramatically reduced after exposure to FasL and, to a much lesser degree, after exposure to recombinant TRAIL (Figure 3B). In line, the inventors detected FasL on 28.2% and 16.1% of CD4+ and CD8+ MILs, respectively (Figure 3C) and on 12.7% of flu TC (not shown). TRAIL expression was detected only on 12.5% and 5.3% of CD4+ and CD8+ MILs, while membrane bound TNF was hardly detectable (Figure 3C). Neutralization of FasL by monoclonal antibodies completely abrogated the CAMK1D-induced protection against cytotoxic activity of MILs (Figure 3D). Thus, CAMK1D mediates intrinsic tumour resistance against activated T cells through interfering with Fas-mediated death signalling. In line with this, U266 myeloma cells strongly express Fas (Figure 3E) and similar to KMM-1 cells they are protected by CAMK1D expression against Fas-mediated cell death (Figure 3F).

### Example 3: CAMK1D has immune checkpoint function in multiple tumour entities and allows for patient stratification

Since Fas-FasL interactions represent a major cytotoxic principle in tumour immunology, we wondered whether CAMK1D might protect not only multiple myeloma but also solid tumour cells against immune rejection. Therefore Fas expression was analysed on several human cancer cell lines. Fas expression was low in the pancreatic cancer cell line PANC-i and in the breast cancer cell line MCF-7. However, strong Fas and CAMK1D expression was found in Mel270, which is a PD-L1⁺ human uveal melanoma (UVM) cell line (Figure 3G, and H). UVM is a highly treatment-refractory and anti-PD-1-resistant subtype of malignant melanoma (59). In line with the observation in the myeloma cell lines, silencing of CAMK1D significantly increased the cytolytic response of Mel270 towards FasL exposure (Figure 3I), indicating that uveal melanomas can exploit CAMK1D for resistance against T cell attack. In contrast, although expressed, CAMK1D silencing in the Fas negative tumour cell lines PANC-i and MCF-7 did not sensitize these cells towards T cell killing (data not shown). These data indicate a strong rationale for CAMK1D inhibition in particular in the context of Fas-positive tumours to achieve significant anti-tumour immune response.

The clinical outcome of a cohort of uveal melanoma patients together with genome-wide RNA expression data from their tumour tissue is available at the TCGA database and allows an analysis of the prognostic impact of CAMK1D in this highly immunotherapy-refractory patient population. CAMK1D expression in UVM might protect those tumours with strong Fas receptor expression against immune rejection. Therefore the inventors stratified patients in this cohort according to expression levels of CAMK1D and Fas (above/below median). Kaplan-Meier analyses show that overexpression of CAMK1D in Fas receptor^{high} tumours but not in Fas receptor^{low} tumours correlate with poor patient prognosis (Figure 3J). This suggests that CAMK1D exerts a tumour protective effect in particular in the context of Fas receptor activation during an immune response. T cell activity in tumours is characterized by IFN-gamma secretion and thus correlates with PD-L1 upregulation. It was found that over-expression of CAMK1D and PD-L1 was tightly co-regulated in UVM melanomas (Figure 3K). Thus, under conditions of immune activation and PD-L1 expression CAMK1D represents another level of immune resistance in tumour cells. Furthermore, the present study with PD-L1 expressing yet refractory tumour models shows that CAMK1D supersedes the PD-L1 axis in mediating immune-suppression.

Using the TCGA database CAMK1D and PD-L1 co-regulation was studied in other tumour entities that are largely unresponsive to anti-PD-1 treatment, specifically in ovarian, pancreatic, colorectal, stomach and esophageal cancer and in glioblastoma. Among them, CAMK1D and PD-L1 were co-expressed in ovarian, pancreatic, stomach and esophageal cancer. As observed in UVM, significant correlations of CAMK1D and Fas receptor expression with poor outcome in these cancers was detected (Figure 4A-F) with the exception of pancreatic cancer. However, pancreatic cancer is characterized by defective Fas receptor signalling (60, 61) and thus CAMK1D-mediated immune protection may not be activated in this tumour entity. Taken together, the data indicate that in several PD-1-therapy refractory tumours, CAMK1D is co-regulated with PD-L1 and controls tumour rejection after Fas receptor activation.

### Example 4: CAMK1D regulates the activity of effector caspases -3, -6 and -7 after Fas receptor activation

FasL binding to Fas receptor results in complex signalling events. This induces on the one hand the caspase cascade that finally activates endonucleases to initiate apoptosis by DNA fragmentation and on the other hand stimulates Ca²⁺ influx into the cytoplasm, which ultimately triggers CAMK1D activation. CAMK1D might thus interfere with the cellular apoptotic cascade to mediate its tumour protective effect. To clarify this assumption, the impact of CAMK1D expression on tumour cell killing in the absence of effector caspases was analysed. Indeed, silencing of each of the individual downstream effector caspase (caspase -3, -6 and -7) completely abrogated the increased lysis of CAMK1D-deficient tumour cells after FasL exposure (Figure 5A, B). Thus, CAMK1D selectively regulates cellular sensitivity towards apoptotic cell death. Besides, these results demonstrate the necessity of simultaneous activity of all three effector caspases for efficient induction of apoptotic cell death after Fas activation.

CAMK1D activation depends on binding to calmodulin (CaM) which upon Ca²⁺ influx induces a conformational change allowing the CAMK-kinase (CAMKK) to phosphorylate and fully activate CAMK1D (62, 63). FasL-expressing MILs thus might trigger Ca²⁺ release in KMM-1 cells sufficient for CAMK1D activation. Hence, the intracellular Ca²+ in KMM-1 cells on single cell level was compared after exposure to MILs or rHuFasL and it was found found that both procedures induced a similar, robust increase of intracellular Ca²⁺ shortly after treatment (Figure 5C, D).

Activation of CAMK1D requires binding to Ca²⁺/calmodulin complexes which can be inhibited by W-7 hydrochloride (64). Treatment with 5 µM W-7 hydrochloride is not toxic to KMM-1 cells (Figure 5E) and sharply recapitulated the effect of CAMK1D silencing on FasL induced tumour cell apoptosis, suggesting CAMK1D to be the decisive target of calmodulin for mediating FasL resistance (Figure 5F). Since both CAMK1D silencing (50%-75% knockdown efficiency) and W-7 hydrochloride treatment only incompletely blocked CAMK1D, it was explored whether their combination further reduced cell viability after FasL exposure. Indeed, this combinatorial treatment resulted in a 3-fold further increase of FasL-mediated tumour cell killing (Figure 5F). To further corroborate these findings, a CAMK1D -specific inhibitor (QPP) was applied to the multiple myeloma as well as the uveal melanoma cell line.QPP has the following structure of formula (I) and was identified as a strong inhibitor of CAMK1D. 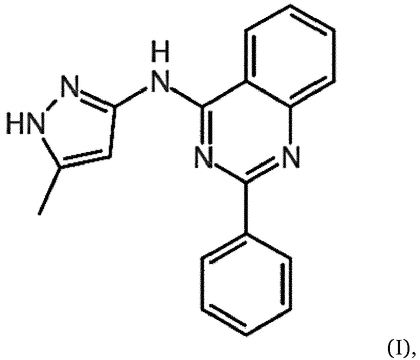 QPP is also known under CAS ID # CAS-404828-08-6 and has a IUPAC name (5-Methyl-1H-pyrazol-3-yl)-(2-phenylquinazolin-4-yl)amine.

QPP was identified as CAMK1D inhibitor in a radiometric protein kinase assay (PanQinase® Activity Assay) used for measuring the kinase activity of a recombinantly expressed CAMK1D protein kinase. All assays were performed with a BeckmanCoulter/SAGIAN™ Core System. Using varying concentrations an IC50 of 2,94E-06 was determined for QPP (shown in Figure 7).

The additional treatment with recombinant FasL induced a significant tumour cell viability, confirming that CAMK1D plays a substantial role in conferring resistance towards tumour cell apoptosis (Figure 5G). Taken together, these results demonstrate that CAMK1D activation in cancer cells is (i) triggered by cytotoxic T cells via FasL-induced Ca²⁺ release and (ii) is required to control Fas-induced tumour cell apoptosis.

### Example 5: CAMK1D knock-out reduces tumour growth in-vivo

To further confirm the role of CAMK1D in mediating cancer resistance against immune attack in *vivo,* the inventors knocked out Camkid in the murine colon adenocarcinoma cell line MC38 using the CRISPR/Cas9 technique. *In vitro* analysis of MC38 Camkid-deficient tumour cells already revealed sensitivity towards FasL as well as TRAIL mediated apoptosis. Thus, MC38 Camkid KO as well as MC38 NTS (non-targeting sequence) cells were injected into the left and right flank of the same mouse of both immunodeficient NSG and immunocompetent C57BL6 mice. MC38 Camkid KO and MC38 NTS tumours outgrew in a similar manner in NSG mice, while a significant difference was observed in the immunocompetent C57BL6 mice, where Camkid-deficient tumours grew as in the NSG mice, while the growth of Camkid-deficient tumours was significantly retarded (Figure 5H).

### Example 6: CAMK1D binds and phosphorylates effector caspases

To elucidate mechanistic aspects of CAMK1D involvement in the Fas-signalling cascade, activation of caspase-8 and -9, the prototypic initiator caspases of the extrinsic and intrinsic apoptotic pathway, respectively (65). FasL-induced activation of caspase-8 and -9 was comparably effective in CAMK1D proficient and -deficient KMM-1 cells (Figure 6A, B). Thus, CAMK1D controls initiation of the apoptotic cascade downstream of death inducing signalling complex (DISC) assembly or activation of initiator caspases. Consequently, CAMK1D might regulate the activity of effector caspases -3, -6 or -7. To this end, the activation of the central executioner caspase-3 was studied through various techniques, including Luminex analysis, flow cytometry and western blot. Indeed, a strong increase in caspase-3 activation was observed in CAMK1D-deficient KMM-1 cells after FasL treatment (Figure 6C-E). In addition, increased cleavage of the effector caspases -6 and -7 in CAMK1D-deficient tumour cells was detected (Figure 6F). Moreover, the phosphorylation and thus activation level of the transcription factor cAMP response element-binding protein (CREB), was increased in CAMK1D-proficient cells, which was responsible for the transcription of the anti-apoptotic molecule Bcl-2. At early time-points (15min, 30min and 1h) of rHuFasL stimulation the phosphorylation levels of the Extracellular Signal-regulated Kinases (ERK1/2) was observed, were enhanced in wild-type cells, while the knockdown of CAMK1D reestablished basal levels. The altered activation of the presented proteins implies that CAMK1D plays a role in interfering with the apoptotic machinery of KMM-1 cells leading to tumour cell resistance towards FasL-positive T cells. CAMK1D has thus far not been established as a regulator of effector caspase activity. Notably, in silico analysis (using the webtools KinaseNet and UniProt (http://www.kinasenet.ca/showProtein: https://www.uniprot.org/uniprot/P42574) predicted a binding motif for CAMK1D on caspase-3 and also on caspase-6 (regions in Caspase 3: amino acids R147 and S150; caspase 6: R254 and S257). For caspase-7, however, no binding motifs were predicted. To confirm these results, a co-immunoprecipitation (co-IP) experiment was performed. Notably, CAMK1D co-immunoprecipitated with caspase-3, caspase-6, and caspase-7 and the levels of CAMK1D interaction with all three effector caspases increased upon rHuFasL treatment (Figure 6G, H). A direct CAMK1D/effector caspase interaction could on the one hand result in stoichiometric inhibition of caspase cleavage by initiator caspases. Alternatively, the effector caspases may also serve as targets of CAMK1D kinase activity. Phosphorylation of inhibitory serine residues 150 (caspase-3) or 257 (caspase-6) impedes their activation, proteolytic activity and ultimately hampers apoptosis induction (66).

Notably, the inhibitory Ser150 phosphorylation site of caspase-3 (67) and the corresponding Ser257 of caspase-6 (68) are located in the kinase-function critical distance of up to 4 amino acids apart from the predicted binding site for CAMK1D. CAMK1D might thus be able to phosphorylate Ser150 and Ser257 of caspases -3 and -6. Indeed, CAMK1D deficient KMM-1 cells showed a strongly reduced phosphorylation level of inhibitory serine residues of both caspase-3 and -6 already at steady-state conditions (Figure 6I-L). In KMM-1 wt cells, phosphorylation levels transiently decreased 15min - 30min after FasL treatment (which has been attributed to transient stimulation of phosphatases (69)), but recovered to pre-stimulation levels within 1h (caspase-3) to 4h (caspase-6). In contrast, caspase-3 and -6 phosphorylation was persistently low in CAMK1D-deficient KMM-1 cells throughout the entire observation period, resulting in overall much lower caspase inactivation compared to CAMK1D wt cells. This demonstrates that CAMK1D is required for steady-state inactivation of effector caspases through phosphorylation and for the rapid restoration of caspase-3 and -6 phosphorylation after FasL stimulation.

Taken together, these results demonstrate that in tumours, CAMK1D upon its activation through FasL regulates activation and activity of all effector caspases after cytotoxic T cell encounter. These results further suggest that this is at least partially achieved by the inhibitory phosphorylation of the effector caspases.

### Materials and Methods:

### Experimental model and subject details: Patients, healthy donors, and samples:

Patients presenting with previously untreated multiple myeloma (n=332) or monoclonal gammopathy of unknown significance (MGUS; n=22) at the University Hospitals of Heidelberg and Montpellier as well as 10 healthy normal donors have been included in the study approved by the ethics committee (#229/2003 and S-152/2010) after written informed consent. Patients were diagnosed, staged and response to treatment assessed according to standard criteria (33-35).

Samples: Normal bone marrow plasma cells and myeloma cells were purified using anti-CD138 microbeads (Miltenyi Biotec, Bergisch Gladbach, Germany) (36-40). Peripheral CD27⁺ memory B-cells (n=11) were FACS-sorted as described (41). The human myeloma cell lines U266, RPMI-8226, LP-1, OPM-2, SK-MM-2, AMO-1, JJN-3, NCI-H929, KMS-12-BM, KMS-11, KMS-12-PE, KMS-18, MM1.S, JIM3, KARPAS-620, L363 and ANBL6 were purchased from the German Collection of Microorganisms and Cell Cultures (Braunschweig, Germany) and the American Type Cell Culture (Wesel, Germany), the XG-lines were generated at INSERM U1040 (Montpellier, France) (42). KMM-1 cells were obtained from the National Institutes of Biomedical Innovation, Health and Nutrition (Osaka, Japan). Cell line identity was assessed for proprietary cell lines by DNA-fingerprinting, mycoplasma-contamination excluded by PCR-based assays, and EBV-infection status by clinical routine PCR-based diagnostics. Polyclonal plasmablastic cells (n=io) were generated as published (38, 43, 44). The human uveal melanoma cell line Mel270 was established, characterized and provided by Prof. Griewank (University Hospital Essen) (45). KMM-1-luc cells were generated after transfection with a pEGFP-luc plasmid (provided by Dr. Rudolf Haase, LMU Munich, Germany) and selected for the G418-resistance gene. Lipofectamine LTX was used as transfection reagent according to the manufacturer s instructions. Transfected cells were selected for 14 days with G418-containing medium (0.6 mg/mL). KMM-1-luc cells were sorted twice for the expression of GFP by flow cytometry and cultured in the presence of 0.6 mg/mL G418. Cell sorting was conducted in collaboration with the DKFZ sorting core facility, using the FACSARIA II cell sorter (BD). KMM-1, U266 and Mel270 were cultured under standard conditions in RPMI media supplemented with 10% fetal calf serum, 100 U/mL penicillin G and 100 µg/ml streptomycin at 37 °C in a humidified atmosphere under 5% CO₂.

### MILs isolation

Marrow-infiltrating lymphocytes were isolated from the bone marrow of a multiple myeloma patient. Briefly, T cells were isolated from the negative fraction of CD138-sorted bone marrow cells using untouched Human T cells Dynabeads (Invitrogen). Cells were stained for anti-CD3 (Pacific Blue™ anti-human CD3 (Clone OKT3), Biolegend), anti-CD4 (APC/Cy7 mouse anti-human CD4 (Clone RPA-T4), BD Biosciences) and anti-CD8 (Pacific Blue™ mouse anti-human CD8 (Clone RPA-T8), BD Biosciences), tested for HLA-A2 positivity (APC mouse anti-human HLA-A2 Clone BB7.2 (RUO), BD Biosciences) and subsequently expanded using the rapid expansion protocol.

### MILs expansion

MILs cultures were ex-vivo expanded using a modified version of the Rapid Expansion Protocol (REP) (46, 47). 2X106 of freshly isolated MILs were diluted to 6 x10⁵ cell/mL in CLM supplemented with 3000 U/mL rHuIL2 (Novartis Pharma). Cells were incubated in 25 cm² tissue culture flask for 48h at 37°C and 5% CO₂. PBMCs from three different buffy coats (at a ratio of 1:1:1) were irradiated with 60 Gy (Gammacell 1000) and used as feeder cells to support MILs expansion. 2x10⁶ MILs were co-incubated with 2x10⁸ feeder cells (in a ratio 1:100) in 400 mL of MIL expansion medium (CLM/AIM-V) with 30 ng/mL OKT3 antibody (Thermo Scientific) and 3000 IU/mL IL-2 for 5 days in a G-Rex 100 cell culture flask. Afterwards, 250 mL of supernatant was changed with 150 mL of fresh media and IL-2 was replenished to keep the concentration at 3000 IU/mL. On day 7, MILs were divided into three G-Rex 100 flasks in a final volume of 250 mL medium each and media was again replenished on day 11. On day 14 of the expansion, MILs were counted and frozen in aliquots of 40x10⁶ cells/mL in freezing media A (60% AB serum and 40% RPMI1640) and B (80% AB serum and 20% DMSO).

### Generation of flu-antigen specific CD8+ T cells

For the generation of flu-specific CD8+ T cells (flu TC), PBMCs from HLA-A2 healthy donors were isolated. Total CD8+ T cells were sorted from PBMCs by magnetic separation (Miltenyi) (day 0) and expanded in the presence of A2-matched flu peptide (GILGFVFTL) for 14 days. Irradiated autologous CD8- fraction was used as feeder cells during the first 7 days of expansion. Afterwards, irradiated T2 cells were used as fresh feeder cells. On day 1 and day 8, 100 IU/mL IL2 (Novartis Pharma) and 5 ng/µL IL15 (R&D Systems) were added to the expansion. The percentage of flu-antigen specific T cells was determined by pentamer staining (GILGFVFTL-APC, Prolmmune) on day 7 and 14 via flow cytometry analysis. After antigen-specific expansion, flu TC were sorted by FACS and expanded further for 14 days by using rapid expansion protocol.

### PCR and qPCR

Gene expression was measured using end-point PCR. Briefly, total RNA was isolated from cell pellets using the RNeasy Mini kit (Qiagen) according to the manufacturer's guidelines. 1 µg of RNA was reverse transcribed to complementary DNA (cDNA) using the QuantiTect reverse transcription kit (Qiagen) according to the manufacturer's protocol. Synthesized cDNA was amplified using conventional PCR. PCR samples were set up in a 25 µL volume using 2x MyTaq HS Red Mix (Bioline), 500 nM of gene-specific primer mix and 100 ng of template cDNA. The PCR program was set as the following: 95°C for 3 min, 35 cycles of 3 repetitive steps of denaturation (95°C for 30 s), annealing (60°C for 30 s) and extension (72°C for 30 s), and a final step at 72°C for 5 min. PCR products were run on a 2% agarose gel in TAE buffer using a gel electrophoresis system (Thermo Scientific) and DNA bands were visualized using UV light of myECL Imager (Thermo Scientific). Knockdown efficiency of siRNA sequences was measured by quantitative PCR (qPCR). For qPCR, 10 ng of template cDNA, 2x QuantiFast SYBR Green PCR mix (Qiagen) and 300 nM of gene-specific primer mix was used per 20 µL reaction and each sample was prepared in triplicates. Reactions were run using the QuantStudio 3 (Applied Biosystems). Expression of several genes was normalized to the expression of β-actin gene and the analysis was performed using comparative Ct method.

Gene expression profiling using U133 2.0 plus arrays (Affymetrix, Santa Clara, CA, USA) was performed as published (36, 37, 48). Expression data are deposited in ArrayExpress under accession numbers E-MTAB-317.

### Survival and correlation analysis using The Cancer Genome Atlas (TCGA)

Transcriptomic normalized RNA-Seq by Expectation-Maximization (RSEM) and clinical data from different tumor entities was downloaded using the TCGA2STAT package for R (49). Log2-normalized expression values were correlated (Person's r) using the ggpubr package for R. Survival curves were generated using survminer package for R. FAS expression was cut at the median to generate Fas high and low sets. Similarly, CAMK1D expression was cut at the median for the Kaplan-Meier survival curves. Significance was calculated using the log-rank test.

### Reverse siRNA transfection

Gene knockdown in tumor cells was induced using reverse siRNA transfection with Lipofectamine RNAiMAX (Thermo Scientific). Briefly, 200 µL of 250 nM siRNA solution was added to each well of a 6-well plate. 4 µl of RNAiMAX transfection reagent was diluted in 196 µL of RPMI (Sigma-Aldrich) and incubated for 10 min at room temperature (RT). 400 µL of additional RPMI was added and 600 µL of RNAiMAX mix was given to the siRNA coated wells and incubated for 30 min at RT. 3,5 x 10⁵ KMM-1 (WT or luc) cells were resuspended in 1,2 mL of antibiotic-free RPMI culture medium supplemented with 10% FCS, seeded in the siRNA-RNAiMAX containing wells and incubated for 48 h at 37°C, 5% CO₂. Final siRNA concentration was 25 nM in all cases.

### Phospho-Protein Isolation

To isolate phosphorylated proteins from cells, tumor cells were pelleted at 0,5 x g for 5 min and washed once with PBS at 4°C. The cell pellets were lysed with one pellet volume of Phosphoplex Lysis Buffer (Merck Millipore) containing protease inhibitor cocktail (Cabliochem, 1:100) and phosphatase inhibitor cocktail (Sigma-Aldrich, 1:100) at 4 °C for 15 min on a rotator. Samples were centrifuged at 17000 g at 4°C for 15 min. Supernatants containing the protein lysates were collected into fresh tubes and quantified using the Pierce BCA Protein Assay Kit (Thermo Scientific) according to the manufacturer's protocol. Proteins were stored at -20 °C.

### SDS-PAGE

30 µg of protein lysates were denaturated in 4x NuPAGE LDS Sample Buffer (Thermo Scientific) containing 10% B-mercaptoethanol (PAN) at 70°C for 10 min. Samples were spun down and separated on NuPAGE 4-12% Bis-Tris Gels (Thermo Scientific) along with PageRuler Prestained Protein Ladder (Thermo Scientific) and run at 115-150 V for 90 min.

### Semi-Dry Western Blot

Proteins were transferred from the gel to a PVDF membrane (Millipore) using a semi-dry western blot method. The PVDF blotting membrane (Merck Millipore) was activated in 100% methanol (Merck Millipore) for 1 min and afterwards placed in Transfer Buffer (Thermo Science) until use. Blots were assembled from anode to cathode into the Pierce Power Blot cassette (Thermo Scientific) and run at 24 V for 10 min. Membranes were washed in 1x TBS and then placed in blocking solution (5% BSA / 0.05% TBST) for 2 h. Primary antibodies (anti-CAMKiD (Abcam) 1:20000, anti-caspase-3 (Abcam) 1:750, anti-caspase-6 (Abcam) 1:2000, anti-caspase-7 (Thermo Scientific) 1:1000, anti-caspase-3 (phospho S150) (Abcam) 1:850, anti-caspase-6 (phospho S257) (Abcam) 1:250 and sodium potassium ATPase (Abcam) 1:20000) were diluted in 5% BSA / 0.05% TBST and kept on the membrane overnight at 4°C on a rotator. Membranes were then washed three times for 10 min with 1 % BSA / 0.05% TBST. Afterwards, HRP-conjugated secondary antibodies (anti-rabbit 1:4000, Santa Cruz or anti-mouse 1:4000, Santa Cruz) were added to 1% BSA/TBST and kept on the membrane at room temperature for 1h on a shaker. Thereafter, the membranes were washed for 10 min with 1% BSA/TBST, then TBST and lastly with TBS. The blots were incubated with the ECL Detection Reagent (Reagent A and Reagent B, 1:1, GE Healthcare) for 4 min and the chemiluminescence was detected with myECL Imager (Thermo Scientific).

### Co-immunoprecipitation assay

For detection of direct protein-protein interaction, co-immunoprecipitation was performed. Briefly, 10 x 10⁶ tumor cells were seeded in 10 cm² petri dishes. The next day, cells were stimulated for 4 h with 100 ng/mL rHuFasL (Biolegend). Unstimulated cells were used as negative control. Afterwards, tumor cells were detached, resuspended in ice cold TBS and centrifuged at 400 g for 6 min at 4°C. Supernatant was discarded, cell pellet was resuspended in 1,5 mL TBS and centrifuged at 500 g for 8 min at 4°C. Cell pellet was lysed with 1,5 mL lysis buffer (50 mM Tris-HCl, 150 mM NaCl, 0,5% NP40 or Triton-X) containing protease inhibitor (Roche complete 25x) and kept on a rotator for 1 h at 4°C. Afterwards, cells were centrifuged for 20 min at 20000 g at 4°C. Supernatant was collected and centrifuged for further 5 min at 20000 g at 4°C. Meanwhile, protein-G agarose (Sigma-Aldrich) was washed with 1 mL TBS and centrifuged for 1 min at 12000 g. 1 mL of cell supernatant containing cytoplasmatic proteins was added to 60 µL protein-G agarose, incubated with anti-caspase-3 (1:50) (Cell Signaling), anti-caspase-6 (1:50) (Abcam) or anti-caspase-7 (1:100) (Cell Signaling) antibodies and incubated overnight on a rotator at 4°C. 90 µL of cell lysates were frozen at -20°C. The next day, the immunoprecipitated samples were centrifuged at 12000 g at 4°C for 1 min. Supernatant was discarded and protein-G agarose was washed three times with lyses buffer and centrifuged at 12000 g at 4°C for 1 min. 2x LDS containing 10% β-mercaptoethanol was added to the immunoprecipitated samples, while 4x LDS containing 10% β-mercaptoethanol was added to the lysates. Samples were denaturated for 10 min at 95°C on a thermocycler. Samples were spun down and separated on NuPAGE 4-12% Bis- Tris Gels (Thermo Scientific) along with PageRuler Prestained Protein Ladder (Thermo Scientific) and run at 115-150 V for 90 min. After electrophoresis, proteins were transferred on a PVDF membrane (Millipore). Anti-CAMKiD antibody (1:10000) was diluted in 5% BSA / 0.05% TBST and kept on the membrane overnight at 4°C on a rotator. Membranes were then washed three times for 10 min with 1% BSA / 0.05% TBST. Afterwards, HRP-conjugated secondary antibodies (anti-rabbit 1:3000) (Santa-Cruz) was added to 1% BSA / TBST and kept on the membrane at room temperature for 1 h on a shaker. The membrane was washed. The blot was incubated with the ECL Detection Reagent (Reagent A and Reagent B, 1:1, GE Healthcare) for 4 min and the chemiluminescence was detected with myECL Imager (Thermo Scientific).

### Plasmid transfection

To generate KMM-1-luc cells, 3,5 x 10⁵ KMM-1 WT cells were seeded in a 6 well plate and incubated at 37°C overnight. 15 µL Lipofectamine LTX reagent were diluted in 150 µL Opti-MEM medium (Gibco). Simultaneously, 3.5 µg of pEGFP-Luc plasmid was diluted in 175 µL Opti-MEM medium and 3.5 µL of PLUS Reagent was added. 150 µL of diluted DNA was added to 150 µL diluted Lipofectamine LTX (Life Technologies) reagent and incubated for 5 min at RT. DNA-lipid complex was then added to the growth medium of the myeloma cells. Cells were incubated at 37°C for 48 h before investigation of transfection efficacy by flow cytometry.

### Luciferase-based cytotoxicity assay

KMM-1-luc cells were reverse transfected with the desired siRNA sequences in white 96-well-plate (Perkin Elmer) and incubated for 48 h at 37°C, 5% CO₂. At the same day of transfection MILs were thawn and treated with benzonase (100 IU/mL) (Merck). Cell density was adjusted to 0,6 x 10⁶ cells/mL in CLM supplemented with 3000 IU/mL rhuIL-2 (Novartis) for 48 h. IL-2 was depleted 24 h before the co-culture. Flu TC were thawn 6 h before co-culture. For the cytotoxicity setting, MILs, flu TC, the supernatant of activated MILs or rHuFasL were added to transfected tumor cells at desired E:T ratio/concentration, and incubated for 20 h at 37°C, 5% CO₂. For the viability setting, only CLM was added to the tumor cells. After co-culture, supernatant was removed, remaining tumor cells were lysed using 40 µL/well of cell lysis buffer for 10 min. After tumor cell lysis, 60 µL/well of luciferase assay buffer was added and luciferase intensity was measured by using the Spark 20M plate reader (Tecan) with a counting time of 100 msec. Luciferase activities (relative luminescence units = RLUs) were either represented as raw luciferase values or as normalized data to scramble or unstimulated controls.

### Real-time live-cell imaging assay

Target genes in KMM-1 or U266 tumor cells were knocked down with reverse siRNA transfection for 48 h. The reverse siRNA transfection was performed using transparent 96 well microplates (TPP). In parallel, MILs were thawn and prepared as previously described. After 48 h, MILs (E:T 10:1) or rHuFasL (100 ng/mL) were added to the target cells in CLM with YOYO-1 (final concentration 1:5000) and co-cultured at 37°C. For viability controls the according amount of CLM with YOYO-1 (final concentration 1:5000) was added. MILs or rHuFasL-mediated tumor lysis was imaged on the green channel using an IncuCyte ZOOM live cell imager (ESSEN BioScience) for the indicated time points at a 10x magnification. Data were analyzed with the Incucyte ZOOM 2016A software by creating a top-hat filter-based mask for the calculation of the area of YOYO-1 incorporating cells (indicating dead cells).

### ELISA

Tumor cells were transfected with the indicated siRNAs in a 96-well plate. Afterwards, T cells were added at the indicated E:T ratio for 20 h and 100 µL of supernatants were harvested for the detection of IFN-γ (Human IFN-γ ELISA Set; BD OptEIA), IL-2 (Human IL-2 ELISA Set; BD OptEIA), Granzyme B (Human Granzyme B ELISA development kit; Mabtech) and TNF (Human TNF ELISA Set; BD OptEIA). Experiments were performed according to the manufacturer's instructions. Polyclonal stimulation (Dynabeads Human T-Activator CD3/CD28, Invitrogen) was used as positive control. Absorbance was measured at λ = 450 nm, taking λ = 570 nm as reference wavelength using the Spark microplate reader (TECAN).

### Flow cytometry (FACS)

Flow cytometry was used for the detection of proteins expressed on the plasma membrane of tumor and T cells. Intracellular staining was performed for the detection of caspase-3 (FITC Active Caspase-3 Apoptosis Kit, BD Bioscience) according to manufacturer's instruction. Tumor cells were detached from plates using PBS-EDTA, centrifuged at 500 x g for 5 min and resuspended in FACS buffer (5 x 105 cells/tube). Live T cell and tumor cells were distinguished by using Live/Dead Fixable Yellow dead Cell Stain (Life Technologies) followed by blocking with kiovig (human plasma-derived immunoglobulin, Baxter, Deerfield, Illinois, USA) at a concentration of 100 µg/mL in FACS buffer (PBS, 2% FCS) for 15 min in the dark on ice. Samples were washed two times in FACS buffer and incubated with either fluorophore-conjugated primary antibodies or isotype control (APC anti-human CD274 (PD-L1) (Clone 29E.2A3), Biolegend; Alexa Fluor 647 Mouse anti-human CCR9 (Clone 112509 (RUO), BD Biosciences; Brilliant Violet 421 anti-human CD95 (Fas) (Clone DX2), Biolegend; PE anti-human CD95 (Fas) (Clone DX2), Biolegend; APC anti-human CD261 (DR4, TRAIL-Ri) (Clone DJR1), Biolegend; PE anti-human CD262 (DR5, TRAIL-R2) (Clone DJR2), Biolegend; Biotin anti-human CD120a (TNFR1) (Clone W15099A), Biolegend; PE/Cy7 anti-human CD120b (TNFR2) (Clone 3G7A02), Biolegend; PE/Cy7 anti-human CD279 (PD-1) Antibody, Biolegend); APC mouse anti-human CD178 (Clone NOK-1), BD Biosciences; PE anti-human CD253 (TRAIL) (Clone RIK2), Biolegend; APC anti-human TNF-a (Clone Mab11), Biolegend for 20 min on ice in the dark. Afterwards, cells were washed twice and acquired with the FACS Canto II cell analyzer machine (BD Bioscience) or FACSLyrics Flow cytometer and data were analyzed using FlowJo (Tree Star).

### Calcium Imaging

KMM-1 cells grown on coverslips were washed with Ringer solution (118 mM NaCl, 5 mM KCl, 1.2 mM MgCl2, 1.2 mM NaHPO₄, 2 mM NaH₂PO₄, 1.8 mM CaCl2, 5 mM glucose, 9.1 mM HEPES, pH 7.4, with NaOH) and loaded with Fura-2-AM ester (Thermo Fisher Scientific, Waltham, USA) for 45 min. After 15 min, MILs or rHuFasL (50 ng/ml) was added to scr siRNA transfected cells and recording of the intracellular free Ca²⁺ was continued for further 30 minutes. Experiments were performed using a ZEISS live cell imaging setup based on an inverse microscope (Axio Observer Z.1) equipped with Fluar 40x/1.3 objective lens (ZEISS, Germany). Fura 2-AM-loaded KMM-1 cells were illuminated with light of 340 nm or 380 nm (BP 340/30 HE, BP 387/15 HE) using a fast wavelength switching and excitation device (Lambda DG-4, Sutter Instrument), and fluorescence was detected at 510 nm (BP 510/90 HE and FT 409) using an AxioCam MRm LCD camera (ZEISS). Data were recorded and analyzed with ZEN 2012 software (ZEISS, Jena, Germany).

### Generation of supernatants of activated MILs

For the generation of the supernatant of polyclonally activated MILs, 1 x 10⁶ MILs were suspended in 1 mL of CLM collected in a 15 mL tube and stimulated with 25 µL of Dynabeads Human T-Activator CD3/CD28 (Thermo Scientific). Afterwards, only the supernatant (100 µL/well) of activated T cells was added to knocked down tumor cells and incubated overnight at 37°C, 5% CO₂. Luciferase-based cytotoxicity assay was performed. Alternatively, MILs were stimulated with tumor cells at an E:T ratio of 10:1. After 20 h co-culture, plates were centrifuged at 450 g for 5 min and 100 µL/well of the supernatant was collected for cytokines detection (ELISA).

### Functional neutralization

For the functional neutralization experiment, anti-FasL (Biolegend) or isotype control (Biolegend) were pre-incubated with MILs for 1 h at 37°C, 5% CO₂. As negative control, antibodies were cultivated in the absence of T cells. Afterwards, antibody-containing supernatants were used to stimulate KMM-1-luc cells, which were reverse transfected with the indicated siRNAs. The final concentration of the neutralizing antibodies was 100 ng/mL for anti-FasL and isotype control. As positive control recombinant FasL protein (100 ng/mL, Biolegend) was added to the tumor cells instead of T cells. 20 h after co-culture, luciferase intensity was measured.

### Blocking assays

For the experiments using the anti-Calmodulin (W7) (Tocris) inhibitor, 1 x 10⁴ KMM-1-luc (scr or CAMK1D-transfected) cells/well were seeded in white 96 well plates (Perkin Elmer) in 100 µL of RPMI 10 % FCS. The small molecule inhibitor was added at the indicated concentrations for 1 h at 37°C, before 100 ng/mL rHuFasL or medium control was added. DMSO treatment served as negative control. After 20 h stimulation, luciferase-based cytotoxicity assay was performed. For CAMK1D inhibition, 1 x 10⁴ KMM-1-luc or 1 x 10⁴ Mel270 cells/well were incubated overnight in a 96 well plate. OMX2001 was added at the indicated concentrations 1h before rHuFasL stimulation (100 ng/mL) or medium control. DMSO treatment served as negative control. After 20h stimulation, luciferase-based cytotoxicity assay was performed.

### Luminex assays

Tumor cells were stimulated with rHuFasL (100 ng/mL) for 15 min, 30 min, 1 h, 2 h, 4 h and 8 h. Unstimulated cells served as control. For the detection of intracellular phosphorylated analytes, a general pathway (MILLIPLEX MAP Multi-Pathway Magnetic Bead 9-Plex kit, Millipore) was used. For the detection of proteins involved in the activation of apoptosis the MILLIPLEX MAP Early Phase Apoptosis 7-plex-kit (Millipore) together with active caspase-3 Magnetic Bead MAPmate (Millipore) was used. Beads specific for GAPDH served as normalization control. 20 µg of protein lysates were used for the detection of ERK/MAP kinase 1/2 (Thr185/Tyr187), Akt (Ser473), STAT3 (Ser727), JNK (Thr183/Tyr185), p70 S6 kinase (Thr412), NF-kB (Ser536), STAT5A/B (Tyr694/699), CREB (Ser133), and p38 (Thr180/Tyr182) phosphorylated Akt (Ser473), JNK (Thr183/Tyr185), Bad (Ser112), Bcl-2 (Ser70), p53 (Ser46), cleaved caspase-8 (Asp384), cleaved caspase-9 (Asp315) and active caspase-3 (Asp175). The assay was performed according to the manufacturer 's instructions and samples were measured using the MAGPIX Luminex instrument (Merck Millipore).

### In vivo experiment

Experiments were performed in two cohorts of mice: C57BL6 (n=12) and NOD/SCID gamma chain (NSG) mice (n=12) were subcutaneously injected with 1 x 10⁵ MC38 Camkid KO (g3 clone 11) or 1 x 105 MC38 NTS (clone 12) cells each into the right and left flank of one mouse, respectively. Tumor growth was measured twice a week and the volume was determined using the following formula: Tumor volume (mm³) = (Width² x Length) x (π / 6). Mice were sacrificed when tumors exceeded 1.5cm in diameter.

### Statistics

For statistical analysis, GraphPad Prism software v6.0 (GraphPad Software, La Jolla, CA, USA was used. If not differently stated, statistical differences between the control and the test groups were determined by using two-tailed unpaired Student's t-test. In all statistical tests, a p-value ≤ 0.05 was considered significant with * = p ≤ 0.05, ** = p ≤ 0.01, *** = p ≤ 0.001 and **** = p ≤ 0.0001.

### REFERENCES

The references are:
1. Choi C, Witzens M, Bucur M, Feuerer M, Sommerfeldt N, Trojan A, Ho A, Schirrmacher V, Goldschmidt H, and Beckhove P. Enrichment of functional CD8 memory T cells specific for MUC1 in bone marrow of patients with multiple myeloma. Blood. 2005;105(5):2132-4.
2. Safi S, Yamauchi Y, Rathinasamy A, Stamova S, Eichhorn M, Warth A, Rauch G, Dienemann H, Hoffmann H, and Beckhove P. Functional T cells targeting tumour-associated antigens are predictive for recurrence-free survival of patients with radically operated non-small cell lung cancer. Oncoimmunology. 2017;6(11):e1360458.
3. Schmitz-Winnenthal FH, Volk C, Z'Graggen K, Galindo L, Nummer D, Ziouta Y, Bucur M, Weitz J, Schirrmacher V, Buchler MW, et al. High frequencies of functional tumour-reactive T cells in bone marrow and blood of pancreatic cancer patients. Cancer research. 2005;65(21):10079-87.
4. Sommerfeldt N, Schutz F, Sohn C, Forster J, Schirrmacher V, and Beckhove P. The shaping of a polyvalent and highly individual T-cell repertoire in the bone marrow of breast cancer patients. Cancer research. 2006;66(16):8258-65.
5. Galon J, Costes A, Sanchez-Cabo F, Kirilovsky A, Mlecnik B, Lagorce-Pages C, Tosolini M, Camus M, Berger A, Wind P, et al. Type, density, and location of immune cells within human colorectal tumours predict clinical outcome. Science. 2006;313(5795):1960-4.
6. Reissfelder C, Stamova S, Gossmann C, Braun M, Bonertz A, Walliczek U, Grimm M, Rahbari NN, Koch M, Saadati M, et al. Tumour-specific cytotoxic T lymphocyte activity determines colorectal cancer patient prognosis. J Clin Invest. 2015;125(2):739-51.
7. Abiko K, Matsumura N, Hamanishi J, Horikawa N, Murakami R, Yamaguchi K, Yoshioka Y, Baba T, Konishi I, and Mandai M. IFN-gamma from lymphocytes induces PD-L1 expression and promotes progression of ovarian cancer. British journal of cancer. 2015;112(9):1501-9.
8. Alsaab HO, Sau S, Alzhrani R, Tatiparti K, Bhise K, Kashaw SK, and Iyer AK. PD-1 and PD-L1 Checkpoint Signalling Inhibition for Cancer Immunotherapy: Mechanism, Combinations, and Clinical Outcome. Frontiers in pharmacology. 2017;8(561.
9. Zou W, Wolchok JD, and Chen L. PD-L1 (B7-H1) and PD-1 pathway blockade for cancer therapy: Mechanisms, response biomarkers, and combinations. Science translational medicine. 2016;8(328):328rv4.
10. Hodi FS, O'Day SJ, McDermott DF, Weber RW, Sosman JA, Haanen JB, Gonzalez R, Robert C, Schadendorf D, Hassel JC, et al. Improved survival with ipilimumab in patients with metastatic melanoma. The New England journal of medicine. 2010;363(8):711-23.
11. Slovin SF, Higano CS, Hamid O, Tejwani S, Harzstark A, Alumkal JJ, Scher HI, Chin K, Gagnier P, McHenry MB, et al. Ipilimumab alone or in combination with radiotherapy in metastatic castration-resistant prostate cancer: results from an open-label, multicenter phase I/II study. Annals of oncology : official journal of the European Society for Medical Oncology. 2013;24(7):1813-21.
12. Yang JC, Hughes M, Kammula U, Royal R, Sherry RM, Topalian SL, Suri KB, Levy C, Allen T, Mavroukakis S, et al. Ipilimumab (anti-CTLA4 antibody) causes regression of metastatic renal cell cancer associated with enteritis and hypophysitis. Journal of immunotherapy. 2007;30(8):825-30.
13. Brahmer JR, Tykodi SS, Chow LQ, Hwu WJ, Topalian SL, Hwu P, Drake CG, Camacho LH, Kauh J, Odunsi K, et al. Safety and activity of anti-PD-Li antibody in patients with advanced cancer. The New England journal of medicine. 2012;366(26):2455-65.
14. Topalian SL, Hodi FS, Brahmer JR, Gettinger SN, Smith DC, McDermott DF, Powderly JD, Carvajal RD, Sosman JA, Atkins MB, et al. Safety, activity, and immune correlates of anti-PD-i antibody in cancer. The New England journal of medicine. 2012;366(26):2443-54.
15. Page DB, Postow MA, Callahan MK, Allison JP, and Wolchok JD. Immune modulation in cancer with antibodies. Annu Rev Med. 2014;65(185-202.
16. Bu X, Mahoney KM, and Freeman GJ. Learning from PD-1 Resistance: New Combination Strategies. Trends Mol Med. 2016;22(6):448-51.
17. Carbognin L, Pilotto S, Milella M, Vaccaro V, Brunelli M, Calio A, Cuppone F, Sperduti I, Giannarelli D, Chilosi M, et al. Differential Activity of Nivolumab, Pembrolizumab and MPDL3280A according to the Tumour Expression of Programmed Death-Ligand-1 (PD-L1): Sensitivity Analysis of Trials in Melanoma, Lung and Genitourinary Cancers. PLoS One. 2015;10(6):e0130142.
18. Hugo W, Zaretsky JM, Sun L, Song C, Moreno BH, Hu-Lieskovan S, Berent-Maoz B, Pang J, Chmielowski B, Cherry G, et al. Genomic and Transcriptomic Features of Response to Anti-PD-1 Therapy in Metastatic Melanoma. Cell. 2016;165(1):35-44.
19. Royal RE, Levy C, Turner K, Mathur A, Hughes M, Kammula US, Sherry RM, Topalian SL, Yang JC, Lowy I, et al. Phase 2 trial of single agent Ipilimumab (anti-CTLA-4) for locally advanced or metastatic pancreatic adenocarcinoma. Journal of immunotherapy. 2010;33(8):828-33.
20. Nowicki TS, Hu-Lieskovan S, and Ribas A. Mechanisms of Resistance to PD-1 and PD-L1 Blockade. Cancer journal. 2018;24(1):47-53.
21. Fourcade J, Sun Z, Benallaoua M, Guillaume P, Luescher IF, Sander C, Kirkwood JM, Kuchroo V, and Zarour HM. Upregulation of Tim-3 and PD-1 expression is associated with tumour antigen-specific CD8+ T cell dysfunction in melanoma patients. The Journal of experimental medicine. 2010;207(10):2175-86.
22. Wang L, Rubinstein R, Lines JL, Wasiuk A, Ahonen C, Guo Y, Lu LF, Gondek D, Wang Y, Fava RA, et al. VISTA, a novel mouse Ig superfamily ligand that negatively regulates T cell responses. The Journal of experimental medicine. 2011;208(3):577-92.
23. Jing W, Gershan JA, Weber J, Tlomak D, McOlash L, Sabatos-Peyton C, and Johnson BD. Combined immune checkpoint protein blockade and low dose whole body irradiation as immunotherapy for myeloma. Journal for immunotherapy of cancer. 2015;3(1):2.
24. Hallett WH, Jing W, Drobyski WR, and Johnson BD. Immunosuppressive effects of multiple myeloma are overcome by PD-L1 blockade. Biology of blood and marrow transplantation : journal of the American Society for Blood and Marrow Transplantation. 2011;17(8):1133-45.
25. Benson DM, Jr., Bakan CE, Mishra A, Hofmeister CC, Efebera Y, Becknell B, Baiocchi RA, Zhang J, Yu J, Smith MK, et al. The PD-1/PD-L1 axis modulates the natural killer cell versus multiple myeloma effect: a therapeutic target for CT-011, a novel monoclonal anti-PD-i antibody. Blood. 2010;116(13):2286-94.
26. Tamura H, Ishibashi M, Yamashita T, Tanosaki S, Okuyama N, Kondo A, Hyodo H, Shinya E, Takahashi H, Dong H, et al. Marrow stromal cells induce B7-H1 expression on myeloma cells, generating aggressive characteristics in multiple myeloma. Leukemia. 2013;27(2):464-72.
27. Lesokhin AM, Ansell SM, Armand P, Scott EC, Halwani A, Gutierrez M, Millenson MM, Cohen AD, Schuster SJ, Lebovic D, et al. Nivolumab in Patients With Relapsed or Refractory Hematologic Malignancy: Preliminary Results of a Phase Ib Study. Journal of clinical oncology : official journal of the American Society of Clinical Oncology. 2016;34(23):2698-704.
28. Sonneveld P, Schmidt-Wolf IG, van der Holt B, El Jarari L, Bertsch U, Salwender H, Zweegman S, Vellenga E, Broyl A, Blau IW, et al. Bortezomib induction and maintenance treatment in patients with newly diagnosed multiple myeloma: results of the randomized phase III HOVON-65/ GMMG-HD4 trial. J Clin Oncol. 2012;30(24):2946-55.
29. Hulin C, Belch A, Shustik C, Petrucci MT, Duhrsen U, Lu J, Song K, Rodon P, Pegourie B, Garderet L, et al. Updated Outcomes and Impact of Age With Lenalidomide and Low-Dose Dexamethasone or Melphalan, Prednisone, and Thalidomide in the Randomized, Phase III FIRST Trial. J Clin Oncol. 2016.
30. Lokhorst HM, Plesner T, Laubach JP, Nahi H, Gimsing P, Hansson M, Minnema MC, Lassen U, Krejcik J, Palumbo A, et al. Targeting CD38 with Daratumumab Monotherapy in Multiple Myeloma. N Engl JMed. 2015;373(13):1207-19.
31. Child JA, Morgan GJ, Davies FE, Owen RG, Bell SE, Hawkins K, Brown J, Drayson MT, and Selby PJ. High-Dose Chemotherapy with Hematopoietic Stem-Cell Rescue for Multiple Myeloma. N Engl J Med. 2003;348(19):1875-83.
32. Seckinger A, Delgado JA, Moser S, Moreno L, Neuber B, Grab A, Lipp S, Merino J, Prosper F, Emde M, et al. Target Expression, Generation, Preclinical Activity, and Pharmacokinetics of the BCMA-T Cell Bispecific Antibody EM801 for Multiple Myeloma Treatment. Cancer cell. 2017;31(3):396-410.
33. Greipp PR, Miguel JS, Durie BGM, Crowley JJ, Barlogie B, Bladé J, Boccadoro M, Child JA, Avet-Loiseau H, Harousseau J-L, et al. International staging system for multiple myeloma. J Clin Oncol. 2005;23(15):3412-20.
34. Durie BG. Staging and kinetics of multiple myeloma. Semin Oncol. 1986;13(3):300-9.
35. Blade J, Samson D, Reece D, Apperley J, Bjorkstrand B, Gahrton G, Gertz M, Giralt S, Jagannath S, and Vesole D. Criteria for evaluating disease response and progression in patients with multiple myeloma treated by high-dose therapy and haemopoietic stem cell transplantation. Myeloma Subcommittee of the EBMT. European Group for Blood and Marrow Transplant. Br J Haematol. 1998;102(5):1115-23.
36. Seckinger A, Delgado JA, Moser S, Moreno L, Neuber B, Grab A, Lipp S, Merino J, Prosper F, Emde M, et al. Target Expression, Generation, Preclinical Activity, and Pharmacokinetics of the BCMA-T Cell Bispecific Antibody EM801 for Multiple Myeloma Treatment. Cancer Cell. 2017;31(3):396-410.
37. Hose D, Reme T, Hielscher T, Moreaux J, Meissner T, Seckinger A, Benner A, Shaughnessy JD, Barlogie B, Zhou Y, et al. Proliferation is a central independent prognostic factor and target for personalized and risk adapted treatment in multiple myeloma. Haematologica. 2011;96(87-95.
38. Hose D, Moreaux J, Meissner T, Seckinger A, Goldschmidt H, Benner A, Mahtouk K, Hillengass J, Reme T, Vos JD, et al. Induction of angiogenesis by normal and malignant plasma cells. Blood. 2009;114(1):128-43.
39. Hose D, Reme T, Meissner T, Moreaux J, Seckinger A, Lewis J, Benes V, Benner A, Hundemer M, Hielscher T, et al. Inhibition of aurora kinases for tailored risk-adapted treatment of multiple myeloma. Blood. 2009;113(18):4331-40.
40. Seckinger A, Meissner T, Moreaux J, Goldschmidt H, Fuhler GM, Benner A, Hundemer M, Reme T, Shaughnessy JD, Barlogie B, et al. Bone morphogenic protein 6: a member of a novel class of prognostic factors expressed by normal and malignant plasma cells inhibiting proliferation and angiogenesis. Oncogene. 2009;28(44):3866-79.
41. Moreaux J, Cremer FW, Reme T, Raab M, Mahtouk K, Kaukel P, Pantesco V, Vos JD, Jourdan E, Jauch A, et al. The level of TACI gene expression in myeloma cells is associated with a signature of microenvironment dependence versus a plasmablastic signature. Blood. 2005;106(3):1021-30.
42. Zhang XG, Gaillard JP, Robillard N, Lu ZY, Gu ZJ, Jourdan M, Boiron JM, Bataille R, and Klein B. Reproducible obtaining of human myeloma cell lines as a model for tumour stem cell study in human multiple myeloma. Blood. 1994;83(12):3654-63.
43. Corre J, Mahtouk K, Attal M, Gadelorge M, Huynh A, Fleury-Cappellesso S, Danho C, Laharrague P, Klein B, Reme T, et al. Bone marrow mesenchymal stem cells are abnormal in multiple myeloma. Leukemia. 2007;21(5):1079-88.
44. Fuhler GM, Baanstra M, Chesik D, Somasundaram R, Seckinger A, Hose D, Peppelenbosch MP, and Bos NA. Bone marrow stromal cell interaction reduces syndecan-i expression and induces kinomic changes in myeloma cells. Exp Cell Res. 2010;316(11):1816-28.
45. Griewank KG, Yu X, Khalili J, Sozen MM, Stempke-Hale K, Bernatchez C, Wardell S, Bastian BC, and Woodman SE. Genetic and molecular characterization of uveal melanoma cell lines. Pigment cell & melanoma research. 2012;25(2):182-7.
46. Dudley ME, Wunderlich JR, Shelton TE, Even J, and Rosenberg SA. Generation of tumour-infiltrating lymphocyte cultures for use in adoptive transfer therapy for melanoma patients. Journal of Immunotherapy. 2003;26(4):332-42.
47. Jin J, Sabatino M, Somerville R, Wilson JR, Dudley ME, Stroncek DF, and Rosenberg SA. Simplified method of the growth of human tumour infiltrating lymphocytes in gas-permeable flasks to numbers needed for patient treatment. JImmunother. 2012;35(3):283-92.
48. Seckinger A, Meißner T, Moreaux J, Depeweg D, Hillengass J, Hose K, Reme T, Rosen-Wolff A, Jauch A, Schnettler R, et al. Clinical and prognostic role of annexin A2 in multiple myeloma. Blood. 2012;120(5):1087-94.
49. Wan YW, Allen GI, and Liu Z. TCGA2STAT: simple TCGA data access for integrated statistical analysis in R. Bioinformatics. 2016;32(6):952-4.
50. Gilbert DF, Erdmann G, Zhang X, Fritzsche A, Demir K, Jaedicke A, Muehlenberg K, Wanker EE, and Boutros M. A novel multiplex cell viability assay for high-throughput RNAi screening. PLoS One. 2011;6(12):e28338.
51. Khandelwal N, Breinig M, Speck T, Michels T, Kreutzer C, Sorrentino A, Sharma AK, Umansky L, Conrad H, Poschke I, et al. A high-throughput RNAi screen for detection of immune-checkpoint molecules that mediate tumour resistance to cytotoxic T lymphocytes. EMBO Mol Med. 2015;7(4):450-63.
52. Boutros M, Bras LP, and Huber W. Analysis of cell-based RNAi screens. Genome Biol. 2006;7(7):R66.
53. Brackertz B, Conrad H, Daniel J, Kast B, Kronig H, Busch DH, Adamski J, Peschel C, and Bernhard H. FLT3-regulated antigens as targets for leukemia-reactive cytotoxic T lymphocytes. Blood Cancer J. 2011;1(3):e11.
54. Zhang C, Xin H, Zhang W, Yazaki PJ, Zhang Z, Le K, Li W, Lee H, Kwak L, Forman S, et al. CD5 Binds to Interleukin-6 and Induces a Feed-Forward Loop with the Transcription Factor STAT3 in B Cells to Promote Cancer. Immunity. 2016;44(4):913-23.
55. Tiedemann RE, Zhu YX, Schmidt J, Yin H, Shi CX, Que Q, Basu G, Azorsa D, Perkins LM, Braggio E, et al. Kinome-wide RNAi studies in human multiple myeloma identify vulnerable kinase targets, including a lymphoid-restricted kinase, GRK6. Blood. 2010;115(8):1594-604.
56. Jaffer ZM, and Chernoff J. p21-activated kinases: three more join the Pak. Int J Biochem Cell Biol. 2002;34(7):713-7.
57. King AJ, Sun H, Diaz B, Barnard D, Miao W, Bagrodia S, and Marshall MS. The protein kinase Pak3 positively regulates Raf-1 activity through phosphorylation of serine 338. Nature. 1998;396(6707):180-3.
58. Ye DZ, and Field J. PAK signalling in cancer. Cell Logist. 2012;2(2):105-16.
59. Algazi AP, Tsai KK, Shoushtari AN, Munhoz RR, Eroglu Z, Piulats JM, Ott PA, Johnson DB, Hwang J, Daud AI, et al. Clinical outcomes in metastatic uveal melanoma treated with PD-1 and PD-L1 antibodies. Cancer. 2016;122(21):3344-53.
60. Elnemr A, Ohta T, Yachie A, Kayahara M, Kitagawa H, Fujimura T, Ninomiya I, Fushida S, Nishimura GI, Shimizu K, et al. Human pancreatic cancer cells disable function of Fas receptors at several levels in Fas signal transduction pathway. International journal of oncology. 2001;18(2):311-6.
61. Elnemr A, Ohta T, Yachie A, Kayahara M, Kitagawa H, Ninomiya I, Fushida S, Fujimura T, Nishimura G, Shimizu K, et al. Human pancreatic cancer cells express non-functional Fas receptors and counterattack lymphocytes by expressing Fas ligand; a potential mechanism for immune escape. International journal of oncology. 2001;18(1):33-9.
62. Sakagami H, Kamata A, Nishimura H, Kasahara J, Owada Y, Takeuchi Y, Watanabe M, Fukunaga K, and Kondo H. Prominent expression and activity-dependent nuclear translocation of Ca2+/calmodulin-dependent protein kinase Idelta in hippocampal neurons. Eur J Neurosci. 2005;22(11):2697-707.
63. Wozniak AL, Wang X, Stieren ES, Scarbrough SG, Elferink CJ, and Boehning D. Requirement of biphasic calcium release from the endoplasmic reticulum for Fas-mediated apoptosis. J Cell Biol. 2006;175(5):709-14.
64. Swulius MT, and Waxham MN. Ca(2+)/calmodulin-dependent protein kinases. Cell Mol Life Sci. 2008;65(17):2637-57.
65. McIlwain DR, Berger T, and Mak TW. Caspase functions in cell death and disease. Cold Spring Harb Perspect Biol. 2013;5(4):a008656.
66. Parrish AB, Freel CD, and Kornbluth S. Cellular mechanisms controlling caspase activation and function. Cold Spring Harb Perspect Biol. 2013;5(6).
67. Alvarado-Kristensson M, Melander F, Leandersson K, Ronnstrand L, Wernstedt C, and Andersson T. P38-MAPK signals survival by phosphorylation of caspase-8 and caspase-3 in human neutrophils. The Journal of experimental medicine. 2004;199(4):449-58.
68. Suzuki A, Kusakai G, Kishimoto A, Shimojo Y, Miyamoto S, Ogura T, Ochiai A, and Esumi H. Regulation of caspase-6 and FLIP by the AMPK family member ARK5. Oncogene. 2004;23(42) :7067-75.
69. Alvarado-Kristensson M, and Andersson T. Protein phosphatase 2A regulates apoptosis in neutrophils by dephosphorylating both p38 MAPK and its substrate caspase 3. J Biol Chem. 2005;280(7):6238-44.
70. Bergamaschi A, Kim YH, Kwei KA, La Choi Y, Bocanegra M, Langerod A, Han W, Noh DY, Huntsman DG, Jeffrey SS, et al. CAMK1D amplification implicated in epithelial-mesenchymal transition in basal-like breast cancer. Mol Oncol. 2008:2(4):327-39.
71. Ashkenazi A. Targeting the extrinsic apoptosis pathway in cancer. Cytokine Growth Factor Rev. 2008;19(3-4):325-31.
72. Green DR, and Ferguson TA. The role of Fas ligand in immune privilege. Nat Rev Mol Cell Biol. 2001;2(12):917-24.
73. Kischkel FC, Hellbardt S, Behrmann I, Germer M, Pawlita M, Krammer PH, and Peter ME. Cytotoxicity-dependent APO-1 (Fas/CD95)-associated proteins form a death-inducing signalling complex (DISC) with the receptor. The EMBO journal. 1995;14(22):5579-88.
74. Mellier G, Huang S, Shenoy K, and Pervaiz S. TRAILing death in cancer. Mol Aspects Med. 2010;31(1):93-112.
75. Cerretti DP, Kozlosky CJ, Mosley B, Nelson N, Van Ness K, Greenstreet TA, March CJ, Kronheim SR, Druck T, Cannizzaro LA, et al. Molecular cloning of the interleukin-1 beta converting enzyme. Science. 1992;256(5053):97-100.
76. Lavrik I, Krueger A, Schmitz I, Baumann S, Weyd H, Krammer PH, and Kirchhoff S. The active caspase-8 heterotetramer is formed at the CD95 DISC. Cell Death Differ. 2003;10(1):144-5.
77. Nicholson DW, Ali A, Thornberry NA, Vaillancourt JP, Ding CK, Gallant M, Gareau Y, Griffin PR, Labelle M, Lazebnik YA, et al. Identification and inhibition of the ICE/CED-3 protease necessary for mammalian apoptosis. Nature. 1995;376(6535):37-43.
78. Irmler M, Thome M, Hahne M, Schneider P, Hofmann K, Steiner V, Bodmer JL, Schroter M, Burns K, Mattmann C, et al. Inhibition of death receptor signals by cellular FLIP. Nature. 1997;388(6638):190-5.
79. Pistritto G, Trisciuoglio D, Ceci C, Garufi A, and D'Orazi G. Apoptosis as anticancer mechanism: function and dysfunction of its modulators and targeted therapeutic strategies. Aging. 2016;8(4):603-19.
80. Pepper C, Hoy T, and Bentley DP. Bcl-2/Bax ratios in chronic lymphocytic leukaemia and their correlation with in vitro apoptosis and clinical resistance. British journal of cancer. 1997;76(7):935-8.
81. Soengas MS, Capodieci P, Polsky D, Mora J, Esteller M, Opitz-Araya X, McCombie R, Herman JG, Gerald WL, Lazebnik YA, et al. Inactivation of the apoptosis effector Apaf-i in malignant melanoma. Nature. 2001;409(6817):207-11.
82. Berthelet J, and Dubrez L. Regulation of Apoptosis by Inhibitors of Apoptosis (IAPs). Cells. 2013;2(1):163-87.
83. Marivin A, Berthelet J, Plenchette S, and Dubrez L. The Inhibitor of Apoptosis (IAPs) in Adaptive Response to Cellular Stress. Cells. 2012;1(4):711-37.
84. Chen X, Duan N, Zhang C, and Zhang W. Survivin and Tumourigenesis: Molecular Mechanisms and Therapeutic Strategies. J Cancer. 2016;7(3):314-23.
85. LaCasse EC, Mahoney DJ, Cheung HH, Plenchette S, Baird S, and Korneluk RG. IAP-targeted therapies for cancer. Oncogene. 2008;27(48):6252-75.
86. Cuadrado A, and Nebreda AR. Mechanisms and functions of p38 MAPK signalling. The Biochemical journal. 2010;429(3):403-17.
87. Sun X, Gao L, Chien HY, Li WC, and Zhao J. The regulation and function of the NUAK family. Journal of molecular endocrinology. 2013;51(2):R15-22.
88. Suzuki A, Kusakai G, Kishimoto A, Lu J, Ogura T, and Esumi H. ARK5 suppresses the cell death induced by nutrient starvation and death receptors via inhibition of caspase 8 activation, but not by chemotherapeutic agents or UV irradiation. Oncogene. 2003;22(40):6177-82.
89. Bellucci R, Nguyen HN, Martin A, Heinrichs S, Schinzel AC, Hahn WC, and Ritz J. Tyrosine kinase pathways modulate tumour susceptibility to natural killer cells. J Clin Invest. 2012;122(7):2369-83.
90. Dunn GP, Sheehan KC, Old LJ, and Schreiber RD. IFN unresponsiveness in LNCaP cells due to the lack of JAKi gene expression. Cancer research. 2005;65(8):3447-53.
91. Parampalli Yajnanarayana S, Stubig T, Cornez I, Alchalby H, Schonberg K, Rudolph J, Triviai I, Wolschke C, Heine A, Brossart P, et al. JAK1/2 inhibition impairs T cell function in vitro and in patients with myeloproliferative neoplasms. Br J Haematol. 2015;169(6):824-33.
92. Schonberg K, Rudolph J, Vonnahme M, Parampalli Yajnanarayana S, Cornez I, Hejazi M, Manser AR, Uhrberg M, Verbeek W, Koschmieder S, et al. JAK Inhibition Impairs NK Cell Function in Myeloproliferative Neoplasms. Cancer research. 2015;75(11):2187-99.

## Claims

1. **A Calcium/calmodulin-dependent protein kinase type 1D (CAMK1D) inhibitor for use in a treatment of a proliferative disorder** in a subject, wherein the treatment involves inhibiting an activity, function, expression and/or stability of CAMK1D, and thereby sensitising cells involved with the proliferative disorder to a cell-dependent or cell-independent cytotoxic stimulus; wherein the treatment comprises administering the CAMK1D inhibitor to the subject.

2. **A CAMK1D inhibitor for use in a treatment of a proliferative disorder in a subject, the treatment comprising exposing cells involved with the proliferative disorder** in the subject to: (i) a cell-dependent or cell-independent cytotoxic stimulus; and (ii) the CAMK1D inhibitor.

3. The CAMK1D inhibitor for use of claim 2, wherein in (i) the cells involved with the proliferative disorder are exposed to the cell-dependent or cell-independent cytotoxic stimulus by
(a) a cell-mediated immune response, such as CTL response, wherein the immune cells express and/or secrete a cell-dependent or cell-independent cytotoxic stimulus, in particular wherein the cells involved with the proliferative disorder are exposed to the immune cells;
(b) an administration of immune cells which express and/or secrete a cell-dependent or cell-independent cytotoxic stimulus; and/or
(c) an administration of a substance or composition eliciting the cell-dependent or cell-independent cytotoxic stimulus to the subject.

4. The CAMK1D inhibitor for use of claim 2 or 3, wherein in (ii), exposing a cell involved with the proliferative disorder in the subject to the CAMK1D inhibitor is sensitising cells involved with the proliferative disorder to a pro apoptotic stimulus, and wherein the treatment comprises administering the CAMK1D inhibitor to the subject

5. **A CAMK1D inhibitor for use in a treatment of a proliferative disorder** in a subject, wherein the treatment is for sensitizing a cell involved with the proliferative disorder to a cell-dependent or cell-independent cytotoxic stimulus, the treatment comprising administering the CAMK1D inhibitor to the subject.

6. **A CAMK1D inhibitor for use in a treatment for the sensitisation of a subject suffering from a proliferative disorder to a therapy involving the administration of a cell-dependent or cell-independent cytotoxic stimulus to the subject,** the treatment comprising administering the CAMK1D inhibitor to the subject

7. The CAMK1D inhibitor for use of any one of claims 1 to 6, wherein the cell-dependent or cell-independent cytotoxic stimulus is selected from a substance or composition capable of binding to, and activating or increasing an activity of, a death receptor signalling pathway in the cells involved with the proliferative disorder, for example selected from (i) an agonist of TNR6 signalling (such as an agonistic anti-TNR6 antibody, a membrane bound or soluble TNR6 ligand (FAS ligand), or (ii) an agonist of TRAIL receptor signalling, such as a TRAIL or an agonistic anti-TRAIL receptor (anti-"DR4" or anti-"DR5") antibody.

8. The CAMK1D inhibitor for use of any one of item 1 to 7, wherein the cell-dependent or cell-independent cytotoxic stimulus is capable of inducing apoptosis in the cells involved with the proliferative disorder via activation of one or more caspases.

9. The CAMK1D inhibitor for use of any one of claims 1 to 8, wherein the proliferative disorder is a tumour, such as a solid or a liquid tumour.

10. The CAMK1D inhibitor for use of any one of claims 1 to 9, wherein the proliferative disorder is **characterized by** expression of (i) mRNA and/or protein of CAMK1D, or (ii) expression of mRNA and/or protein CAMK1D and expression of a death receptor, in particular such as TNR6 (Fas) or a TRAIL receptor (DR4/DR5); in the cells involved with the proliferative disorder, and thus preferably tumour cells.

11. The CAMK1D inhibitor for use of any one of claims 1 to 10, wherein the CAMK1D inhibitor is (i) a small molecule, in particular, a small molecule ligand or a small cell-permeable molecule; or is (ii) selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a hetero-bi-functional compound (such as a PROTAC or a HyT molecule); a carbohydrate such as a polysaccharide or oligosaccharide and the like, including variants or derivatives thereof; a lipid such as a fatty acid and the like, including variants or derivatives thereof.

12. **An in vitro method for determining whether a subject has, or is at risk of, developing a proliferative disorder,** such as a tumour, that is associated with cellular resistance against a cell-dependent or cell-independent cytotoxic stimulus, such as of a cell-mediated immune response, the method comprising the step of:
(a) detecting an applicable biomarker in a biological sample from said subject;
wherein the detection of the applicable biomarker in the sample indicates the proliferative disorder, or a risk of developing the proliferative disorder, in the subject; and
wherein the applicable biomarker is one or more selected from the group consisting of:
(i) CAMK1D, in particular the presence (or an amount) of or expression and/or activity of CAMK1D, preferably of phosphorylated CAMK1D;
(ii) death receptor, in particular the presence (or an amount) of or expression and/or activity of a death receptor;
(iii) A death receptor ligand or a cell expressing a death receptor ligand, such as a FAS ligand, in particular the presence (or an amount) of or expression and/or activity of a death receptor ligand.

13. **An in vitro method for determining** whether a subject has, or has a risk of developing, a disease, disorder or condition that is associated with resistance against pro apoptotic stimuli, such as pro apoptotic stimuli elicited by cell-mediated immune responses, and wherein the proliferative disorder is associated with expression or activity of CAMK1D, the method comprising the steps of:
(a) contacting cells of the subject suspected to be involved with the disease, disorder or condition with a CAMK1D inhibitor in the presence of a pro apoptotic signal: (i) immune cells capable of eliciting or eliciting pro apoptotic stimuli towards cells involved with the proliferative disease, disorder or condition, such as lymphocytes, T-cells, CTLs and TILs; or (ii) a pro apoptotic stimulus such as a soluble or substrate bound death receptor agonist or ligand; and
(b) determining initiation of apoptosis in the cells involved with the proliferative disorder of the subject,
wherein an enhancement of the initiation of apoptosis in the cells of the subject indicates that the subject has or has a risk of developing such disease, disorder or condition.

14. **An in vitro method for stratifying a subject** that suffers from a proliferative disorder into a patient group that is distinguished by having a poor prognosis or into a patient group that does not have a poor prognosis, the method comprising the steps of:
(a) detecting an applicable biomarker in a biological sample from said subject, in particular wherein the biological sample comprises cells involved with the proliferative disorder;
wherein the detection of the applicable biomarker in the sample indicates that the subject is stratified into the group of patients having a poor prognosis, and wherein no detection of the applicable biomarker in the sample indicates that the subject is stratified into the group of patients not having a poor prognosis; and
wherein the applicable biomarker is one, preferably both, selected from the group consisting of:
(i) CAMK1D, in particular the presence (or an amount) of or expression and/or activity of CAMK1D, preferably of phosphorylated CAMK1D; and
(ii) a death receptor, such as a member of tumour necrosis factor (TNF) receptor superfamily (e.g., TNFR1, TNR6 (Fas), DR3, DR4, DR5, DR6, and LTpR) in particular the presence (or an amount) of or expression and/or activity of a death receptor;

15. The in vitro method of claim 14, wherein the applicable biomarker is detected in cells involved with the proliferative disorder contained in the biological sample.

16. **A use of an antigen binding protein (ABP)** capable of binding specifically to CAMK1D or phosphorylated CAMK1D in an in-vitro diagnosis of a proliferative disease, disorder or condition in a subject; wherein the proliferative disease, disorder or condition is associated with resistance against a pro apoptotic signal, such as pro apoptotic stimuli elicited by cell-mediated immune responses, and wherein the proliferative disease, disorder or condition is associated with expression or activity of CAMK1D.

17. **A kit for use in a diagnostic method** for determining whether a subject has, or has a risk of developing, a disease, disorder or condition that is associated with resistance against a pro apoptotic stimulus, such as a cell-mediated response mediated by a TNR6 ligand positive immune cell, and that is associated with expression or activity of CAMK1D; wherein:
the diagnostic method comprises a step of surgically obtaining a sample from the subject; and
the kit comprises: (a) either (x) a nucleic acid capable of binding specifically to CAMK1D, or (y) an ABP binding specifically to CAMK1D; and (b) optionally (i) instructions describing how to use the ABP or a nucleic acid or kit for detecting CAMK1D activity in the sample; and/or (ii) one or more other item, component, reagent or other means useful for the use of the kit or the detection of CAMK1D activity in the sample.

18. **A method for identifying and/or characterising** a compound suitable for a treatment of a disease, disorder or condition that is **characterised by** a resistance against death receptor signalling, in particular a resistance against a cell-mediated immune response, and that is **characterised by** expression or activity of CAMK1D, the method comprising the steps of:
(a) bringing into contact a first cell or cell-free system which comprises and/or expresses CAMK1D mRNA or protein and (i) a candidate compound, or (ii) a candidate compound and a cell-dependent or cell-independent cytotoxic stimulus; and
(b) determining (i) the expression, activity, function and/or stability of the (eg protein or mRNA of) CAMK1D, in the first cell or cell-free system; and/or (ii) the cytotoxicity of the cell-dependent or cell-independent cytotoxic stimulus against the first cell or cell-free system;
wherein: (i) a reduced expression, activity function and/or stability of the (eg protein or mRNA of) CAMK1D, in said first cell or cell-free system contacted with the candidate compound compared to said first cell or cell-free system not contacted with said candidate compound; and/or (ii) an enhanced cytotoxicity of the cell-dependent or cell-independent cytotoxic stimulus against the first cell or cell-free system contacted with the candidate compound compared to the cytotoxicity of the cell-dependent or cell-independent cytotoxic stimulus against the first cell or cell-free system not contacted with the candidate compound; indicates that the candidate compound is a compound suitable for the treatment of the disease, disorder or condition that is **characterised by** resistance against death receptor signalling, in particular resistance against a cell-mediated immune response, and that is **characterised by** expression or activity of CAMK1D.

19. **A method for identifying and/or characterising a compound** suitable for a treatment of a disease, disorder or condition that is **characterised by** resistance against death receptor signalling, in particular resistant against a cell-mediated immune response, and that is **characterised by** expression or activity of CAMK1D, the method comprising the steps of:
(a) bringing into contact a first cell or cell free system which comprises and/or expresses CAMK1D mRNA or protein and one or more effector caspase(s) - in particular caspase-3, -6 and/or -7 - mRNA or protein, and (i) a candidate compound, or (ii) a candidate compound, and a cell-dependent or cell-independent cytotoxic stimulus; and
(b) determining (i) the expression, activity, function and/or stability of the (eg protein or mRNA of) of the one or more effector caspase(s) or of one or more phosphorylated effector caspase(s) - in particular caspase-3, -6 and/or -7 - in the first cell or cell-free system; and/or (ii) the cytotoxicity of the cell-dependent or cell-independent cytotoxic stimulus against the first cell or cell-free system;
wherein: (i) an increased expression, activity, function and/or stability of the (eg protein or mRNA of) one or more effector caspase(s) or of one or more phosphorylated effector caspase(s) - in particular caspase-3, -6 and/or -7 - in the first cell or cell-free system, in said first cell or cell-free system contacted with the candidate compound compared to said first cell or cell-free system not contacted with said candidate compound; and/or (ii) an enhanced cytotoxicity of the cell-dependent or cell-independent cytotoxic stimulus against the first cell or cell-free system contacted with the candidate compound compared to the cytotoxicity of the cell-dependent or cell-independent cytotoxic stimulus against the first cell or cell-free system not contacted with the candidate compound; indicates that the candidate compound is a compound suitable for the treatment of the disease, disorder or condition that is **characterised by** resistance against death receptor signalling, in particular resistance against a cell-mediated immune response, and that is **characterised by** expression or activity of CAMK1D.

20. **A method for identifying and/or characterising a compound** suitable for a treatment of a disease, disorder or condition that is **characterised by** resistance against death receptor signalling, in particular resistant against a cell-mediated immune response, and that is **characterised by** expression or activity of CAMK1D, the method comprising the steps of:
(a) bringing into contact a first cell or cell free system which comprises and/or expresses CAMK1D mRNA or protein and calmodulin mRNA or protein, and (i) a candidate compound, or (ii) a candidate compound, and a cell-dependent or cell-independent cytotoxic stimulus; and
(b) determining or detecting (i) the expression, activity, function and/or stability of the (eg protein or mRNA of) of calmodulin in the first cell or cell-free system; and/or (ii) the specific binding of a Ca2+/calmodulin protein complex to CAMK1D protein;
wherein: (i) an increased expression, activity function and/or stability of the (eg protein or mRNA of) calmodulin in the first cell or cell-free system, in said first cell or cell-free system contacted with the candidate compound compared to said first cell or cell-free system not contacted with said candidate compound; and/or (ii) a reduced specific binding of a Ca2+/calmodulin protein complex to CAMK1D protein in the presence of the candidate compound compared to the absence of the candidate; indicates that the candidate compound is a compound suitable for the treatment of the disease, disorder or condition that is **characterised by** resistance against death receptor signalling, in particular resistance against a cell-mediated immune response, and that is **characterised by** expression or activity of CAMK1D.

21. The method of any one of claim 18 to 20, wherein the cell-dependent or cell-independent cytotoxic stimulus is a substance or composition capable of binding to, and activating or increasing activity of, a death receptor signalling pathway, or a downstream component of death receptor signalling, in the cell or cell-free, and preferably is an agonist of TNR6 signalling, such as a membrane bound or soluble TNR6 ligand (FAS ligand), or is an agonist of TRAIL receptor signalling, such as TRAIL.
